# EUROPEAN PATENT SPECIFICATION

(11) **EP 1 248 849 B1**
(45) Date of publication and mention of the grant of the patent: **20.08.2008**
(21) Application number: 01942383.9
(22) Date of filing: 10.01.2001
(51) Int. Cl.: C12N 15/63, C12N 5/10, C07K 14/47, A61P 3/00, A61K 38/17

(54) **OBG3 GLOBULAR HEAD AND USES THEREOF FOR DECREASING BODY MASS**
OBG3 GLOBULAREKOPF UND SEINE VERWENDUNGEN ZUR REDUZIERUNG DES KÖRPERSGEWICHTES
TETE GLOBULAIRE OBG3 ET SES UTILISATIONS POUR REDUIRE LA MASSE CORPORELLE

(30) Priority: 14.01.2000 US 176228 P; 13.04.2000 US 198087 P; 01.09.2000 US 229881 P
(43) Date of publication of application: 16.10.2002
(73) Proprietor: Serono Genetics Institute S.A., 91000 Evry (FR)
(72) Inventor: BIHAIN, Bernard, 35260 Cancale (FR); ERICKSON, Mary, Ruth, San Diego, CA 92131 (US); FRUEBIS, Joachim, Redmond, WA, 98053-7975 (US); YEN-POTIN, Frances, San Diego, CA 92131 (US)
(74) Representative: Brasnett, Adrian Hugh
(86) International application number: PCT/IB2001/000084
(87) International publication number: WO 2001/051645

(56) References cited:
- WO-A-99/07736
- WO-A-99/21577
- US-A- 5 869 330

## Description

### FIELD OF THE INVENTION

The present invention relates to the field of metabolic research, in particular the discovery of compounds effective for reducing body mass and useful for treating obesity-related diseases and disorders. The obesity-related diseases or disorders envisioned to be treated by the methods of the invention include, but are not limited to, hyperlipidemia, atherosclerosis, diabetes, and hypertension.

### BACKGROUND OF THE INVENTION

The following discussion is intended to facilitate the understanding of the invention, but is not intended nor admitted to be prior art to the invention.

Obesity is a public health problem that is serious, widespread, and increasing. In the United States, 20 percent of the population is obese; in Europe, a slightly lower percentage is obese (Friedman (2000) Nature 404:632-634). Obesity is associated with increased risk of hypertension, cardiovascular disease, diabetes, and cancer as well as respiratory complications and osteoarthritis (Kopelman (2000) Nature 404:635-643). Even modest weight loss ameliorates these associated conditions.

While still acknowledging that lifestyle factors including environment, diet, age and exercise play a role in obesity, twin studies, analyses of familial aggregation, and adoption studies all indicate that obesity is largely the result of genetic factors (Barsh et al (2000) Nature 404:644-651). In agreement with these studies, is the fact that an increasing number of obesity-related genes are being identified. Some of the more extensively studied genes include those encoding leptin *(ob)* and its receptor (db), pro-opiomelanocortin *(Pomc),* melanocortin-4-receptor *(Mc4r),* agouti protein *(A^{y}),* carboxypeptidase E *(fat),* 5-hydroxytryptamine receptor 2C *(Htr2c),* nescient basic helix-loop-helix 2 *(Nhlh2),* prohormone convertase 1 IPCSK1), and tubby protein (tubby) (rev'd in Barsh et al (2000) Nature 404:644-651).

### SUMMARY OF THE INVENTION

The instant invention is based on the discovery that portions of the full length OBG3 polypeptide, termed OBG3 polypeptide fragments or gOBG3 polypeptide fragments, have unexpected effects *in vitro* and *in vivo,* including utility for weight reduction and prevention of weight gain in humans and other mammals. These unexpected effects of OBG3 or gOBG3 polypeptide fragment administration in mammals also include reduction of elevated free fatty acid levels caused by administration of epinephrine, i.v. injection of "intralipid", or administration of a high fat test meal, as well as increased fatty acid oxidation in muscle cells, and weight reduction in mammals consuming a high fat/high sucrose diet. These effects are unexpected and surprising given that administration of full-length OBG3 polypeptide typically has no effect *in vivo* or *in vitro.* To the extent that any effect is observed following administration of full-length OBG3 polypeptide, the levels of full-length OBG3 polypeptide required for an effect render it unfeasible as a potential treatment for humans at this time. In contrast, the gOBG3 polypeptide fragments described herein are radically more effective and thus can be provided at levels that are feasible for treatments in humans.

Thus, a first aspect of the invention is use of a globular fragment of OBG3, wherein the globular OBG3 fragment is selected from the group consisting of
(i) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6; or
(ii) a variant of any of the globular OBG3 fragments having one or more permissive amino acid substitutions, wherein each of said permissive amino acid substitutions is a conservative amino acid substitution wherein the variant has at least one but not more than 10 conservative amino acid substitutions; or
(iii) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6; linked to polyethylene glycol or in the form of an IgG Fc fusion peptide;
the globular OBG3 fragment of (ii) or (iii) causing a change in at least one of the parameters selected from post-prandial lipemia, free fatty acid level, triglyceride level, glucose level, free fatty acid oxidation, or weight, the change caused by the globular OBG3 fragment of (ii) or (iii) being comparable to a change caused by the globular OBG3 fragment of (i), for the preparation of a medicament for the prevention or treatment of an obesity related disorder, or for the preparation of a medicament for the reduction of body mass or prevention of weight gain.

In a preferred embodiment a gOBG3 polypeptide fragments described herein is able to lower circulating (either blood, serum or plasma) levels (concentration) of :(i) free fatty acids, (ii) glucose, and/or (iii) triglycerides. Further preferred polypeptide fragments demonstrating free fatty acid level lowering activity, glucose level lowering activity, and/or triglyceride level lowing activity, have an activity that is significantly greater than full length OBG3 at the same molar concentration, have a greater than transient activity and/or have a sustained activity.
Further preferred gOBG3 polypeptide fragments are those that significantly stimulate muscle lipid or free fatty acid oxidation as compared to full length OBG3 polypeptides at the same molar concentration. Further preferred gOBG3 fragments are those that cause C2C12 cells differentiated in the presence of said fragments to undergo at least 10%, 20%, 30%, 35%, or 40% more oleate oxidation as compared to untreated cells or cells with full length OBG3.

Further preferred gOBG3 polypeptide fragments are those that are at least 30% more efficient than full length OBG3 at increasing leptin uptake in a liver cell line (preferably BPRCL mouse liver cells (ATCC CRL-2217)).

Further preferred gOBG3 polypeptide fragments are those that significantly reduce the postprandial increase in plasma free fatty acids due to a high fat meal.

Further preferred gOBG3 polypeptide fragments are those that significantly reduce or eliminate ketone body production as the result of a high fat meal.

Further preferred gOBG3 polypeptide fragments are those that form multimers (e.g., heteromultimers or homomultimers) in vitro and/or in vivo. Preferred multimers are homodimers or homotrimers. Other preferred multimers are homomultimers comprising at least 4, 6, 8, 9, 10 or 12 OBG3 or gOBG3 polypeptide fragment subunits. Other preferred multimers are hetero multimers comprising a gOBG3 polypeptide fragment described herein
A recombinant vector may comprise a promoter operably linked to a coding region capable of expressing:
(a) a globular OBG3 fragment or variant as described herein; or
(b) a fusion of said globular OBG3 fragment wherein the fragment is in the form of an IgG Fc fusion peptide.

A recombinant cell may comprise said recombinant vector A host cell may be recombinant for a polynucleotide described herein

A pharmaceutical or physiologically acceptable composition may comprise, consist essentially of, or consist of, a gOBG3 polypeptidefragment described herein and, alternatively, a pharmaceutical or physiologically acceptable diluent.

A method of reducing body mass may comprise 6 providing or administering to individuals in need of reducing body mass said pharmaceutical or physiologically acceptable composition

The identification of said individuals in need of reducing body mass to be treated with said pharmaceutical or physiologically acceptable composition may comprise genotyping OBG3 single nucleotide polymorphisms (SNPs) or measuring OBG3 or gOBG3 polypeptide or mRNA levels in clinical samples from said individuals. Preferably, said clinical samples are selected from the group consisting of plasma, urine, and saliva. Preferably, gOBG3 polypeptide fragment is administered to an individual with at least a 10%, 20%, 30%, 40%, 50%, 60%, 70%, 80%, 90%, or 100% reduction in blood, serum or plasma levels of full length OBG3 or the naturally proteolytically cleaved OBG3 fragment as compared to healthy, non-obese patients.

A method of preventing or treating an obesity-related disease or disorder may comprise providing or administering to an individual in need of such treatment a pharmaceutical or physiologically acceptable composition as described herein. In preferred embodiments, the identification of said individuals m need of such treatment to be treated with said pharmaceutical or physiologically acceptable composition comprises genotyping OBG3 single nucleotide polymorphisms (SNPs) or measuring OBG3 or gOBG3 polypeptide or mRNA levels in clinical samples from said individuals. Preferably, said clinical samples are selected from the group consisting of blood, serum, plasma, urine, and saliva. Preferably, said obesity-related disease or disorder is selected from the group consisting of obesity, insulin resistance, atherosclerosis, atheromatous disease, heart disease, hypertension, stroke, Syndrome X, non-insulin-dependent diabetes and Type II diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders to be treated by compounds of the invention include hyperlipidemia and hyperuricemia. Yet other obesity-related diseases or disorders of the invention include cachexia, wasting, AIDS-related weight loss, anorexia, and bulimia. In preferred embodiments, said individual is a mammal, preferably a human.

A method of causing or inducing a desired biological response in an individual may comprise the steps of: providing or administering to an individual a composition comprising a gOBG3 polypeptide fragment, wherein said biological response is selected from the group consisting of:
(a) lowering circulating (either blood, serum, or plasma) levels (concentration) of free fatty acids;
(b) lowering circulating (either blood, serum or plasma) levels (concentration) of glucose;
(c) lowering circulating (either blood, serum or plasma) levels (concentration) of triglycerides;
(d) stimulating muscle lipid or free fatty acid oxidation;
(c) increasing leptin uptake in the liver or liver cells;
(e) reducing the postprandial increase in plasma free fatty acids due to a high fat meal; and,
(f) reducing or eliminating ketone body production as the result of a high fat meal; and further wherein said biological response is significantly greater than, or at least 10%, 20%, 30%, 35%, or 40% greater than, the biological response caused or induced by a full length OBG3 polypeptide at the same molar concentration; or alternatively wherein said biological response is greater than a transient response; or alternatively wherein said biological response is sustained.
A method of making the globular OBG3 polypeptide fragment for use in the first aspect may comprise the steps of (a) culturing a host cell under conditions suitable for expressing the fragment, and (b) isolating the fragment.

A method of making a recombinant gOBG3 polypeptide fragment or a full-length OBG3 polypeptide may comprise providing a transgenic, non-human mammal whose milk contains said recombinant gOBG3 polypeptide fragment or full-length protein, and purifying said recombinant gOBG3 polypeptide fragment or said full-length OBG3 polypeptide from the milk of said non-human mammal. In one embodiment, said non-human mammal is a cow, goat, sheep, rabbit, or mouse. In another embodiment, the method may comprise purifying a recombinant full-length OBG3 polypeptide from said milk, and cleaving said protein in vitro to obtain a desired gOBG3 polypeptide fragment.

Preferably, obesity-related diseases and disorders are selected from the group consisting of obesity, insulin resistance, atherosclerosis, atheromatous disease, heart disease, hypertension, stroke, Syndrome X, non-insulin-dependent diabetes and Type II diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders include hyperlipidemia and hyperuricemia. Yet other obesity-related diseases or disorders include cachexia, wasting, AIDS-related weight loss, anorexia, and bulimia.

Methods of reducing body weight for cosmetic purposes may comprise providing to an individual a pharmaceutical or physiologically acceptable composition described herein, or the gOBG3 polypeptide fragment described herein. Preferably, for reducing body weight said individual has a BMI of at least 20 and no more than 25. Alternatively, for said increasing body weight said individual preferably has a BMI of at least 15 and no more than 20.

A pharmaceutical or physiologically acceptable composition described herein may be used for reducing body weight for cosmetic reasons.

Methods of treating insulin resistance may comprise providing to an individual said pharmaceutical or physiologically acceptable composition or the gOBG3 polypeptide fragment described herein.

In a preferred aspect of the methods above and disclosed herein, the amount of gOBG3 polypeptide fragment administered to an individual is sufficient to bring circulating (blood, serum, or plasma) levels (concentration) of OBG3 polypeptides to their normal levels (levels in non-obese individuals). "Normal levels" may be specified as the total concentration of all circulating OBG3 polypeptides (full length OBG3 and fragments thereof) or the concentration of all circulating proteolytically cleaved OBG3 polypeptides only.

### BRIEF DESCRIPTION OF THE FIGURES

Figure 1 shows an alignment of the sequences of the human (APM1), and mouse (AdipoQ and ACRP30) OBG3 polypeptides.
Figure 2 shows the nucleic acid sequence of AdipoQ cloned into the BamHI and XhoI sites of pTrcHisB. AdipoQ begins at 510 and ends at 1214 (insert in bold). This construct does not contain the N-term signal sequence (MLLLQALLFLLILP).
Figure 3 shows a schematic drawing of the protein structure of APM1. The putative signal sequence at the N-terminus (AA 1-17), the unique region (AA 18-41), the collagen region (AA 42-107), and the globular region (AA 108-244) at the carboxy terminus are shown. Two protease cleavage sites after AA 100 and AA 131 are also shown.
Figure 4 shows the nucleic acid sequence of the globular region of AdipoQ cloned into pTrcHisB. AdipoQ globular region begins at 510 and ends at 927 bp. The insert is in bold.
Figure 5 is a graph showing a comparison of the effect of AdipoQ (AQ) and AdipoQ globular head (AQ-GH) on cell-associated ¹²⁵I-leptin in the mouse liver cell line BPRCL. Results are shown as percent of control values in the presence of increasing amounts of compound (AQ or AQ-GH), and are the mean of triplicate determinations.
Figures 6A, 6B, and 6C show graphs of ¹²⁵I-LDL binding, uptake, and degradation, respectively, in the mouse liver cell line BPRCL in the presence of increasing amounts of gOBG3.
Figure 7 shows a protein sequence alignment of the obg3 clone (obg3 clone; the insert in Fig. 2) with the published sequences of human (apml) and mouse (AdipoQ and acrp30) obg3. In the alignment, amino acids (AAs) 45 to 110 contain the collagen-like region; AAs 111-247 contain the globular region. The cut sites from lysine-blocked trypsin fall after AAs 58, 61, 95,103, 115,125, and 134. As determined by amino-terminal sequencing of the gOBG3 product, the gOBG3 start site is at AA 104 (101 for human gOBG3 or APM1).
Figure 8 shows a graphical representation of the effect of gOBG3 (3 x 25 µg ip) on plasma FFA in C57BL6/J mice following a high fat meal (* p < 0.02).
Figures 9A and 9B show graphical representations of the effect of gOBG3 (3 x 25 µg ip) on plasma TG in C57BL6/J mice following a high fat meal (p < 0.05 at 2, 3 and 4 hours). Figure 9A shows TG in mg/dl; Figure 9B shows TG as a percent of the starting value.
Figure 10 shows a graphical representation of the effect of gOBG3 (3 x 25µg ip) on plasma glucose in C57BL6/J mice following a high fat meal.
Figures 11A and 11B show graphical representations of the effect of gOBG3 (3x25 µg ip) on plasma FFA in C57BL6/J mice following a high fat meal. Figure 11A shows FFA as mM; Figure 11B shows FFA as a percent of the starting value.
Figures 12A and 12B show graphical representations of the effect of gOBG3 (3x25 µg) on plasma leptin in C57BL6/J mice following a high fat meal. Figure 12A shows leptin as ng/mL; Figure 12B shows leptin as a percent of the starting value.
Figures 13A and 13B show graphical representations of the effect of gOBG3 (3x25 µg) on plasma Insulin in C57BL6/J mice following a high fat meal. Figure 13A shows insulin levels in ng/mL; Figure 13B shows insulin as a percent of the starting value.
Figures 14A and 14B show graphical representations of the effect of OBG3 on plasma FFA in C57BL6/J mice following a high fat meal. At t = 2 hours a significant reduction in FFA was seen for both treatment groups (p<0.05). Figure 14A shows FFA levels in mM; Figure 14B shows FFA as a percent of the starting value.
Figures 15A and 15B show graphical representations of the effect of OBG3 on plasma TG in C57BL6/J mice following a high fat meal. Figure 15A shows TG levels in mg/dl; Figure 15B shows TG as a percent of the starting value.
Figures 16A and 16B show graphical representations of the effect of OBG3 on plasma glucose in C57BL6/J mice following a high fat meal. Figure 16A shows glucose levels as mg/dl; Figure 16B shows glucose levels as a percent of the starting value.

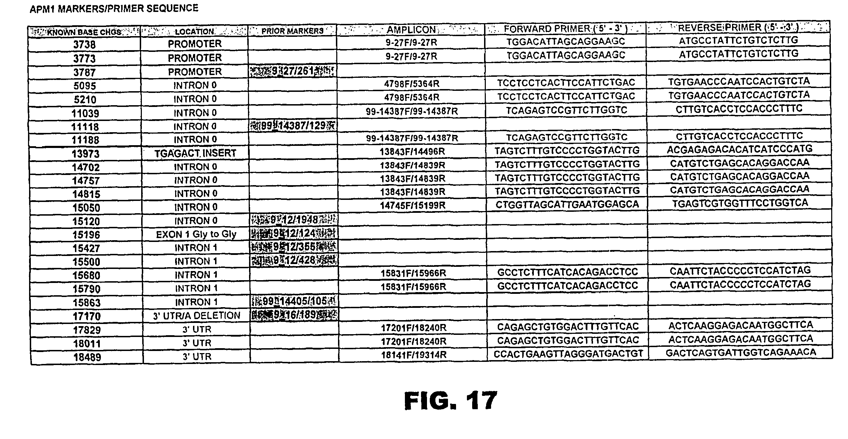

Figure 17 shows a table identifying additional APM1 SNPs. Information concerning Known Base Changes, Location, Prior Markers, Amplicon, and Forward and Reverse primers for microsequencing are shown.
Figures 18A and 18B show graphical representations of the effect of gACRP30 injection in mice on the FFA (Fig. 18A) and glucose (Fig. 18B) increases resulting from epinephrine injection.
Figure 19 shows a graphical representation of the effect of gACRP30 treatment on fatty acid metabolism in muscle isolated from mice.
Figures 20A and 20B show a graphical representation of the effect of gACRP30 treatment on triglyceride content of muscle and liver isolated from mice.
Figures 21A, 21B, 21C, & 21D show graphical representations of the effect of gACRP30 treatment on weight gain & loss in mice. Treatments shown are saline (diamond), ACRP30 (Box), and gACRP30 (triangle). Fig. 21A shows results of treatment of mice after 19 days on a high fat diet. Fig, 21B shows results of treatment of mice after 6 months on a high fat diet.
Figure 22 shows a table of the tested blood chemistry values with saline injections, ACRP30 injections, or gACRP30 injections.

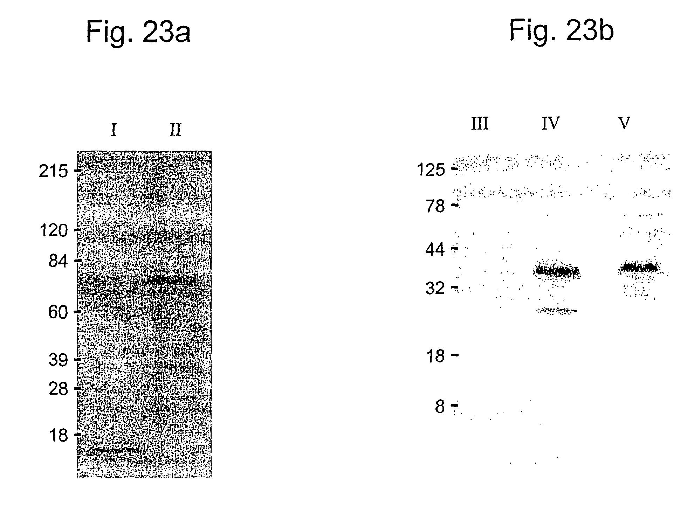

Figures 23A and 23B show a SDS-PAGE separation of the purification of ACRP30 and gACRP30 (23A) and a cleavage product of apml (23B). Fig23A, Lane II shows the complete form of ACRP30 purified by FPLC. Lane I shows the proteolytic cleavage product gACRP30. Fig. 23B shows a cleavage product of apm-1 after immunoprecipitation followed by Western blotting. The apparent molecular weight of this truncated form is 27kDa, corresponding to about 70% of the complete form of apm-1 (Lane IV). This truncated form was not detectable when a second anti-serum, specific for the human non-homologous region (HDQETTTQGPGVLLPKGA) of the protein was used for immunoprecipitation (Lane V) and the same and-globuiar head antiserum for detection. A preimmune serum of the same animal did not detect any protein; a dimer of apm-1 was seen with both specific antibodies (apparent MW 74kDa).
Figure 24 shows a graph depicting the removal of plasma FFAs after Intralipid injection following treatment with gACRP30 (diamonds) or a saline control (squares).

### DETAILED DESCRIPTION OF THE INVENTION

Before describing the invention in greater detail, the following definitions are set forth to illustrate and define the meaning and scope of the terms used to describe the invention herein.

As used interchangeably herein, the terms "oligonucleotides", and "polynucleotides" and nucleic acid include RNA, DNA, or RNA/DNA hybrid sequences of more than one nucleotide in either single chain or duplex form. The terms encompass "modified nucleotides" which comprise at least one modification, including by way of example and not limitation: (a) an alternative linking group, (b) an analogous form of purine, (c) an analogous form of pyrimidine, or (d) an analogous sugar. For examples of analogous linking groups, purines, pyrimidines, and sugars see for example PCT publication No. WO 95/04064. The polynucleotide sequences described herein may be prepared by any known method, including synthetic, recombinant, *ex vivo* generation, or a combination thereof, as well as utilizing any purification methods known in the art.

The terms polynucleotide construct, recombinant polynucleotide and recombinant polypeptide are used herein consistently with their use in the art The terms "upstream" and "downstream" are also used herein consistently with their use in the art. The terms "base paired" and "Watson & Crick base paired" are used interchangeably herein and consistently with their use in the art Similarly, the terms "complementary", "complement thereof", "complement", "complementary polynucleotide", "complementary nucleic acid" and "complementary micleotide sequence" are used interchangeably herein and consistently with their use in the art.

The term "purified" is used herein to describe a polynucleotide or polynucleotide vector described herein that has been separated from other compounds including, but not limited to, other nucleic acids, carbohydrates, lipids and proteins (such as the enzymes used in the synthesis of the polynucleotide). Purified can also refer to the separation of covalently closed polynucleotides from linear polynucleotides, or vice versa, for example. A polynucleotide is substantially pure when at least about 50%, 60%, 75%, or 90% of a sample contains a single polynucleotide sequence. In some cases this involves a determination between conformations (linear versus covalently closed). A substantially pure polynucleotide typically comprises about 50. 60, 70, 80, 90, 95, 99% weight/weight of a nucleic acid sample. Polyrnucleotide purity or homogeneity may be indicated by a number of means well known in the art, such as agarose or polyacrylamide gel electrophoresis of a sample, followed by visualizing a single polynucleotide band upon staining the gel. For certain purposes higher resolution can be provided by using HPLC or other means well known in the art.

Similarly, the term "purified" is used herein to describe a polypeptide that has been separated from other compounds including, but not limited to, nucleic acids, lipids, carbohydrates and other proteins. In some preferred embodiments, a polypeptide is substantially pure when at least about 50%, 60%, 75%, 85%, 90%, 95%, 96%, 97%, 98%, 99%, or 99.5% of the polypeptide molecules of a sample have a single amino acid sequence. In some preferred embodiments, a substantially pure polypeptide typically comprises about 50%, 60%, 70%, 80%, 90% 95%, 96%, 97%, 98%, 99% or 99.5% weight/weight of a protein sample. Polypeptide purity or homogeneity is indicated by a number of methods well known in the art, such as agarose or polyacrylamide gel electrophoresis of a sample, followed by visualizing a single polypeptide band upon staining the gel. For certain purposes higher resolution can be provided by using HPLC or other methods well known in the art.

Further, as used herein, the term "purified" does not require absolute purity; rather, it is intended as a relative definition. Purification of starting material or natural material to at least one order of magnitude, preferably two or three orders, and more preferably four or five orders of magnitude is expressly contemplated. Alternatively, purification may be expressed as "at least" a percent purity relative to heterologous polynucleotides (DNA, RNA or both) or polypeptides. Preferably, the polynucleotides or polypeptides described herein are at least; 10%, 20%, 30% 40%, 50%, 60%, 70%, 80%, 90%, 95%, 96%, 96%, 98%, 99%, 99.5% or 100% pure relative to heterologous polynucleotides or polypeptides. Further preferred are polynucleotides or polypeptides having an "at least" purity ranging from any number, to the thousandth position, between 90% and 100% (e.g., at least 99.995% pure) relative to heterologous polynucleotides or polypeptides. Additionally, purity of the polynucleotides or polypepitides may be expressed as a percentage (as described above) relative to all materials and compounds other than the carrier solution. Each number, to the thousandth position, may be claimed as individual species of purity.

The term "isolated" requires that the material be removed from its original environment (*e.g.,* the natural environment if it is naturally occurring). For example, a naturally-occurring polynucleotide or polypeptide present in a living animal is not isolated, but the same polynucleotide or DNA or polypeptide, separated from some or all of the coexisting materials in the natural system, is isolated. Such polynucleotide could be part of a vector and/or such polynucleotide or polypeptide could be part of a composition, and still be isolated in that the vector or composition is not part of its natural environment.

Specifically excluded from the definition of "isolated" are naturally occurring chromosomes (*e.g.,* chromosome spreads), artificial chromosome libraries, genomic libraries, and cDNA libraries that exist either as an *in vitro* nucleic acid preparation or as a transfected/transformed host cell preparation, wherein the host cells are either an *in vitro* heterogeneous preparation or plated as a heterogeneous population of single colonies. Also specifically excluded are the above libraries wherein a 5' EST makes up less than 5% (or alternatively 1%, 2%, 3%, 4%, 10%, 25%, 50%, 75%, or 90%, 95%, or 99%) of the number of nucleic acid inserts in the vector molecules. Further specifically excluded are whole cell genomic DNA or whole cell RNA preparations (including said whole cell preparations which are mechanically sheared or enzymatically digested). Further specifically excluded are the above whole cell preparations as either an *in vitro* preparation or as a heterogeneous mixture separated by electrophoresis (including blot transfers of the same) wherein the polynucleotides described herein have not been further separated from the heterologous polynucleotides in the electrophoresis medium (*e.g.,* further separating by excising a single band from a heterogeneous band population in an agarose gel or nylon blot).

The term "primer" denotes a specific oligonucleotide sequence which is complementary to a target nucleotide sequence and used to hybridize to the target nucleotide sequence. A primer serves as an initiation point for nucleotide polymerization catalyzed by DNA polymerase, RNA polymerase, or reverse transcriptase.

The term "probe" denotes a defined nucleic acid segment (or nucleotide analog segment, e.g., PNA as defined hereinbelow) which can be used to identify a specific polynucleotide sequence present in a sample, said nucleic acid segment comprising a nucleotide sequence complementary to the specific polynucleotide sequence to be identified.

The term "polypeptide" refers to a polymer of amino acids without regard to the length of the polymer. Thus, peptides, oligopeptides, and proteins are included within the definition of polypeptide. This term also does not specify or exclude post-expression modifications of polypeptides. For example, polypeptides that include the covalent attachment of glycosyl groups, acetyl groups, phosphate groups, lipid groups and the like are expressly encompassed by the term polypeptide. Also included within the definition are polypeptides which contain one or more analogs of an amino acid (including, for example, non-naturally occurring amino acids, amino acids which only occur naturally in an unrelated biological system, modified amino acids from mammalian systems etc.), polypeptides with substituted linkages, as well as other modifications known in the art, both naturally occurring and non-naturally occurring. As used herein, the term "OBG3" refers generically to the murine or human OBG3, unless otherwise specified. The terms "ACRP30" and "AdipoQ" refer specifically to the murine form ofOBG3 and the term "APM-1" refers specifically to the human form of the gene.

Without being limited by theory, the compounds/polypeptides described herein are capable of modulating the partitioning of dietary lipids between the liver and peripheral tissues, and are thus believed to treat "diseases involving the partitioning of dietary lipids between the liver and peripheral tissues." The term "peripheral tissues" is meant to include muscle and adipose tissue. In preferred embodiments, the compounds/polypeptidesdescribed herein partition the dietary lipids toward the muscle. In alternative preferred embodiments, the dietary lipids are partitioned toward the adipose tissue. In other preferred embodiments, the dietary lipids are partitioned toward the liver. In yet other preferred embodiments, the compounds/polypeptides described herein increase or decrease the oxidation of dietary lipids, preferably free fatty acids (FFA) by the muscle. Dietary lipids include, but are not limited to triglycerides and free fatty acids.

Preferred diseases believed to involve the partitioning of dietary lipids include obesity and obesity-related diseases and disorders such as obesity, insulin resistance, atherosclerosis, atheromatous disease, heart disease, hypertension, stroke, Syndrome X, non-insulin-dependent diabetes and Type II diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders to be treated by compound described herein include hyperlipidemia and hyperuricemia. Yet other obesity-related diseases or disorders include cachexia, wasting, AIDS related weight loss, anorexia, and bulimia.

The term "heterologous", when used herein, is intended to designate any polypeptide or polynucleotide other than an OBG3 or gOBG3 polypeptide or a polynucleotide encoding an OBG3 or gOBG3 polypeptide of the present disclosure.

The terms "comprising", "consisting of" and "consisting essentially of are defined according to their standard meaning. With this in mind, the terms may be substituted for one another throughout the instant application in order to attach the specific meaning associated with each term.

The term "host cell recombinant for" a particular polynucleotide described herein means a host cell that has been altered by the hands of man to contain said polynucleotide in a way not naturally found in said cell. For example, said host cell may be transiently or stably transfected or transduced with said polynucleotide.

The term "obesity" as used herein is defined in the WHO classifications of weight (Kopelman (2000) Nature 404:635643). Underweight is less than 18.5 (thin); Healthy is 18.5-24.9 (normal); grade 1 overweight is 25.0-29.9 (overweight); grade 2 overweight is 30.0-39.0 (obesity); grade 3 overweight is greater than or equal to 40.0 BMI. BMI is body mass index (morbid obesity) and is kg/m². Waist circumference can also be used to indicate a risk of metabolic complications where in men a circumference of greater than or equal to 94 em indicates an increased risk, and greater than or equal to 102 cm indicates a substantially increased risk. Similarly for women, greater than or equal to 88 cm indicates an increased risk, and greater than or equal to 88 cm indicates a substantially increased risk. The waist circumference is measured in cm at midpoint between lower border of ribs and upper border of the pelvis. Other measures of obesity include, but are not limited to, skinfold thickness which is a measurement in cm of skinfold thickness using calipers, and bioimpedance, which is based on the principle that lean mass conducts current better than fat mass because it is primarily an electrolyte solution; measurement of resistance to a weak current (impedance) applied across extremities provides an estimate of body fat using an empirically derived equation.

The term "agent acting on the partitioning of dietary lipids between the liver and peripheral tissues" refers to a compound or polypeptide described herein that modulates the partitioning of dietary lipids between the liver and the peripheral tissues as previously described. Preferably, the agent increases or decreases the oxidation of dietary lipids, preferably free fatty acids (FFA) by the muscle. Preferably the agent decreases or increases the body weight of individuals or is used to treat or prevent an obesity-related disease or disorder such as obesity, insulin resistance, atherosclerosis, atheromatous disease, heart disease, hypertension, stroke, Syndrome X, non-insulin-dependent diabetes and Type II diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders to be treated by compounds described herein include hyperlipidemia and hyperuricemia. Yet other obesity-related diseases or disorders of the invention include cachexia, wasting, AIDS-related weight loss, anorexia, and bulimia.

The terms "response to an agent acting on the partitioning of dietary lipids between the liver and peripheral tissues" refer to drug efficacy, including but not limited to, ability to metabolize a compound, ability to convert a pro-drug to an active drug, and the pharmacokinetics (absorption, distribution, elimination) and the pharmacodynamics (receptor-related) of a drug in an individual.

The terms "side effects to an agent acting on the partitioning of dietary lipids between the liver and peripheral tissues " refer to adverse effects of therapy resulting from extensions of the principal pharmacological action of the drug or to idiosyncratic adverse reactions resulting from an interaction of the drug with unique host factors. "Side effects to an agent acting on the partitioning of dietary lipids between the liver and peripheral tissues " can include, but are not limited to, adverse reactions such as dermatologic, hematologic or hepatologic toxicities and further includes gastric and intestinal ulceration, disturbance in platelet function, renal injury, nephritis, vasomotor rhinitis with profuse watery secretions, angioneurotic edema, generalized urticaria, and bronchial asthma to laryngeal edema and bronchoconstriction, hypotension, and shock.

The term "OBG3-related diseases and disorders" as used herein refers to any disease or disorder comprising an aberrant functioning of OBG3, or which could be treated or prevented by modulating OBG3 levels or activity. "Aberrant functioning of OBG3" includes, but is not limited to, aberrant levels of expression of OBG3 (either increased or decreased, but preferably decreased), aberrant activity of OBG3 (either increased or decreased), and aberrant interactions with ligands or binding partners (either increased or decreased). By "aberrant" is meant a change from the type, or level of activity seen in normal cells, tissues, or patients, or seen previously in the cell, tissue, or patient prior to the onset of the illness. In preferred embodiments, these OBG3-related diseases and disorders include obesity and the obesity-related diseases and disorders described previously.

The term "cosmetic treatments" is meant to include treatments with compounds or polypeptides described herein that increase or decrease the body mass of an individual where the individual is not clinically obese or clinically thin. Thus, these individuals have a body mass index (BMI) below the cut-off for clinical obesity (*e.g*. below 25 kg/m²) and above the cut-off for clinical thinness (*e.g.* above 18.5 kg/m²). In addition, these individuals are preferably healthy (*e.g*. do not have an obesity-related disease or disorder of the invention). "Cosmetic treatments" are also meant to encompass, in some circumstances, more localized increases in adipose tissue, for example, gains or losses specifically around the waist or hips, or around the hips and thighs, for example. These localized gains or losses of adipose tissue can be identified by increases or decreases in waist or hip size, for example.

The term "preventing" as used herein refers to administering a compound prior to the onset of clinical symptoms of a disease or condition so as to prevent a physical manifestation of aberrations associated with obesity or OBG3.

The term "treating" as used herein refers to administering a compound after the onset of clinical symptoms.

The term "in need of treatment" as used herein refer to a judgment made by a caregiver *(e.g.* physician, muse, nurse practitioner, etc in the case of humans; veterinarian in the case of animals, including non-human mammals) that an individual or animal requires or will benefit from treatment. This judgment is made based on a variety of factors that are in the realm of a caregiver's expertise, but that include the knowledge that the individual or animal is ill, or will be ill, as the result of a condition that is treatable by the compounds described herein.

The term "perceives a need for treatment" refers to a sub-clinical determination that an individual desires to reduce weight for cosmetic reasons as discussed under "cosmetic treatment" above. The term "perceives a need for treatment" in other embodiments can refer to the decision that an owner of an animal makes for cosmetic treatment of the animal.

The term "individual" as used herein refers to any animal, including mammals, preferably mice, rats, other rodents, rabbits, dogs, cats, swine, cattle, sheep, horses, or primates, and most preferably humans.

The term "non-human animal" refers to any non-human vertebrate, including birds and more usually mammals, preferably primates, animals such as swine, goats, sheep, donkeys, horses, cats, dogs, rabbits or rodents, more preferably rats or mice. Both the terms "animal" and "mammal" expressly embrace humman subjects unless preceded with the term "non-human".

The inventors have found that a fragment of OBG3, called gOBG3, is able to significantly reduce the postprandial response of plasma free fatty acids, glucose, and triglycerides in mice fed a high fat/sucrose meal. There was no significant effect on leptin, insulin or glucagon levels. In addition, gOBG3 was found to increase muscle free fatty acid oxidation *in vitro* and *ex vivo.* Further, gOBG3 was shown to decrease and then to prevent an increase in weight gain in mice that had been fed a high fat/sucrose diet for 19 days. In mice that had been maintained on the same high fat/sucrose diet for 6 mouths, gOBG3 treatment resulted in a sustained weight loss over 16 days that was significant, despite being maintained on the high fat/sucrose diet.

The instant invention encompasses the use of gOBG3 polypeptide fragments in the partitioning of free fatty acid (FFA) and as an important new tool to control energy homeostasis. Of the tissues that can significantly remove lipids from circulation and cause FFA oxidation, muscle is quantitatively the most important. Globular OBG3 is a unique and novel pharmacological tool that controls body weight without interfering with food intake.

### I. Globular OBG3 Polypeptide Fragments for use in theInvention

OBG3 polypeptide fragments that have measurable activity *in vitro* and *in vivo* have been identified. These activities include, but are not limited to, reduction of the postprandial response of plasma free fatty acids, glucose, and triglycerides in mice fed a high fat/sucrose meal (Example 8). increase in muscle free fatty acid oxidation *in vitro* and *ex vivo* (Example 12), and sustained weight loss in mice on a high fat/sucrose diet (Example 14). Other assays for OBG3 polypeptide fragment activity *in vitro* and *in vivo* are also provided (Examples 4, 7, 9, 11, 13, for example), and equivalent assays can be designed by those with skill in the art.

In contrast, the "intact" or "full-length" OBG3 polypeptide does not have either the *in vivo* or the *in vitro* activities that have been identified for gOBG3 polypeptide fragments described herein. In most cases, the activities are either not present or at a minimum are undetectable over control values in the assays used. In other cases, the activities can be measured, but are present either at extremely reduced levels and/or require significantly more protein on a molar basis compared with the gOBG3 polypeptide fragments (*see, e.g.* Example 10). By "intact" or "full-length" OBG3 polypeptide as used herein is meant the full length polypeptide sequence of any OBG3 polypeptide, form the N-terminal methionine to the C-terminal stop codon. Examples of intact or full length OBG3 polypeptides are found in SEQ ID NO:2 (mouse), SEQ ID NO.4 (mouse), and SBQ ID NO:6 (humau). The term "OBG3 polypeptide fragments" as used herein refers to fragments of the "intact" or "full-length" OBG3 polypeptide that have "obesity-related activity". The term "gOBG3 polypeptide fragments" refers to polypeptide fragments of the globular region only and is thus a narrower term than "OBG3 polypeptide fragments". The term "fragment" means a polypeptide having a sequence that is entirely the same as part, but not all, of an intact of full-length OBG3 polypeptide. Such fragments may be "freestanding" (*i.e*. not part of or fused to other polypeptides), or one or more fragments may be present in a single polypeptide. OBG3 or gOBG3 fragments are contiguous fragments of the full length OBG3 polypeptide unless otherwise specified.

The term "obesity-related activity" as used herein refers to at least one, and preferably all, of the activities described herein for OBG3 polypeptide fragments. Assays for the determination of these activities are provided herein (*e.g.* Examples 4,7-9,11-14), and equivalent assays can be designed by those with ordinary skill in the art. Optionally, "obesity-related activity" can be selected from the group consisting of lipid partitioning, lipid metabolism, and insulin-like activity, or an activity within one of these categories. By "lipid partitioning" activity is meant the ability the ability to effect the location of dietary lipids among the major tissue groups including, adipose tissue, liver, and muscle. The inventors have shown that gOBG3 polypeptide fragments play a role in the partitioning of lipids to the muscle, liver or adipose tissue. By "lipid metabolism" activity is meant the ability to influence the metabolism of lipids. The inventors have shown that gOBG3 polypeptide fragments have the ability to affect the level of free fatty acids in the plasma as well as to increase the metabolism of lipids in the muscle through free fatty acid oxidation experiments (Examples 4, 8, 10, 11, 12) and to transiently affect the levels of triglycerides in the plasma and the muscle (Examples 8, 10 13). By "insulin-like" activity is meant the ability of OBG3 polypeptide fragments to modulate the levels of glucose in the plasma. The inventors have found that 9 OBG3 polypeptide fragments do not significantly impact insulin levels but do impact glucose levels similarly to the effects of insulin (Examples 9 & 10). These effects are not seen in the presence of the intact (full-length) OBG3 polypeptide or are significantly greater in the presence of the OBG3 polypeptide fragments compared with the full-length OBG3 polypeptide.

The term "significantly greater "as used herein refers to a comparison of the activity of an OBG3 polypeptide fragment in an obesity-related assay compared with the activity of a full-length OBG3 polypeptide in the same assay. By "significantly" as used herein is meant statistically significant as it is typically determined by those with ordinary skill in the art. For example, data are typically calculated as a mean ± SEM, and a p-value ≤ 0.05 is considered statistically significant. Statistical analysis is typically done using either the unpaired Student's t test or the paired Student's t test, as appropriate in each study. Examples of a significant change in activity as a result of the presence of an OBG3 polypeptide fragment compared to the presence of a full-length OBG3 polypeptide include an increase or decrease in a given parameter of at least 5%, 10%, 15%, 20%, 25%, 30%, 35%, 40%, 45%, 50%, 55%, 60%, 65%, 70%, or 75%. One or more, but not necessarily all, of the measurable parameters will change significantly in the presence of gOBG3 polypeptide fragments as compared to in the presence of an intact OBG3 polypeptide.

Representative "obesity-related assays" are provided in Examples 4, 7, 9, and 11-14. These assays include, but are not limited to, methods of measuring the postprandial response, methods of measuring free fatty acid oxidation, and methods of measuring weight modulation. In preferred embodiments, the post-prandial response is measured in non-human animals, preferably mice. In preferred embodiments changes in dietary lipids are measured, preferably free fatty acids and/or triglycerides. In other embodiments, other physiologic parameters are measured including, but not limited to, levels of glucose, insulin, and leptin. In other preferred embodiments, free fatty acid oxidation is measured in cells *in vitro* or *in vivo*, preferably in muscle cells or tissue of non-human animals, preferably mice. In yet other preferred embodiments weight modulation is measured in human or non-human animals, preferably rodents (rats or mice), primates, canines, felines or procines, on a high fat/sucrose diet. Optionally, "obesity-related activity" includes other activities not specifically identified herein. In general, "measurable parameters" relating to obesity and the field of metabolic research can be selected from the group consisting of free fatty acid levels, free fatty acid oxidation, triglyceride levels, glucose levels, insulin levels, leptin levels, food intake, weight, leptin and lipoprotein binding, uptake and degradation and LSR expression.

In these obesity-related assays, preferred gOBG3 polypeptide fragments described herein but not full-length OBG3 polypeptides, would cause a significant change in at least one of the measurable parameters selected from the group consisting of post-prandial lipemia, free fatty acid levels, triglyceride levels, glucose levels, free fatty acid oxidation, and weight. Alternatively, preferred gOBG3 polypeptide fragments, but not full-length OBG3 polypeptides, would have a significant change in at least one of the measurable parameters selected from the group consisting of an increase in LSR activity, an increase in leptin activity and an increase in lipoprotein activity, By "LSR" active is meant expression of LSR on the surface of the cell, or in a particular conformation, as well as ability to bind, uptake, and lipoprotein, By "leptin" activity is meant its binding, uptake and degradation by LSR, as well as its transport across a blood brain barrier, and potentially these occurrences where LSR is not necessarily the mediating factor or the only mediating factor. Similarly, by "lipoprotein" activity is meant its binding, uptake and degradation by LSR, as well as these occurrences where LSR is not necessarily the mediating factor or the only mediating factor.

Isolated, purified or recombinant gOBG3 polypeptide fragments may be used in the invention. OBG3 polypeptide fragments described herein are useful for reducing or increasing (using antagonists of OBG3 polypeptides) body weight either as a cosmetic treatment or for treatment or prevention of obesity-related diseases and disorders. OBG3 polypeptide fragments are also useful *inter alia* in screening assays for agonists or antagonists of OBG3 fragment activity, for raising OBG3 fragment-specific antibodies, and in diagnostic assays.

The full-length OBG3 polypeptide is comprised of at least four distinct regions including:
1. an N-terminal putative signal sequence from amino acids 1-17 of SEQ ID NO:6, SEQ ID NO:2, or SEQ ID NO:4;
2. a unique region from amino acids 18-41 of SBQ ID NO:6 or 18:-44 of SEQ ID NO:2, or SEQ ID NO:4;
3. a collagen-like region from amino acids 42-107 of SEQ ID NO:6 or 45-110 of SEQ ID NO:2 or SBQ ID NO:4; and
4. a globular region from amino acids 108-244 of SEQ ID NO:6 or 111-247 ofSEQ ID NO:2 or SEQ ID NO:4.

The term "collagen residues" is used in the manner standard in the art to mean the amino acid triplet glycine, X, Y, where X and Y can be any amino acid.

The gOBG3 polypetide fragments described herein preferably provided in an isolated form, and may be partially or substantially purified. A recombinantly produced version of an OBG3 polypeptide fragment can be substantially purified by the one-step method described by Smith et al. ((1988) Gene 67(1):31-40) or by the methods described herein or known in the art (see, e.g., Examples 1-3). Fragments also can be purified from natural or recombinant sources using antibodies directed against the polypeptide fragments described herein by methods known in the art of protein purification.

Preparations of gOBG3 polypeptide fragments involving a partial purification of or selection for the gOBG3 polypeptide fragments are also specifically comtemplated. These crude preparations are envisioned to be the result of the concentration of cells expressing OBG3 polypeptide fragments with perhaps a few additional purification steps, but prior to complete purification of the fragment. The cells expressing OBG3 polypeptide fragments are present in a pellet, they are lysed, or the crude polypeptide is lyophilized, for example.

The gOBG3 polypeptide fragments for use in that the invention are selected from amino acids 101 to 244, 108 to 244, or 132 to 244 of SEQ ID NO:6 and amino acids 104 to 247, 111 to 247, or 135 to 247 ofSBQ ID NO:2 or SBQ ID NO:4.
The gOBG3 fragment is human or mouse, but most preferably human.
The gOBG3 fragments described herein include variants of the gOBG3 fragments described above.

### Variants

It will be recognized by one of ordinary skill in the art that some amino acids of the gOBG3 fragment sequences described herein can be varied without significant effect on the structure or function of the protein; there will be critical amino acids in the fragment sequence that determine activity. Thus, of gOBG3 polypeptide fragments that have obesity-related activity as described above may be used in the invention. Such variants include gOBG3 fragment sequences with one or more permissive amino acid substitutions either from natural mutations or human manipulation selected according to general rules known in the art so as to have little effect on activity. Guidance concerning how to make phenotypically silent amino acid substitutions is provided below.

There are two main approaches for studying the tolerance of an amino acid sequence to change (*see*, Bowie, et al. (1990) Science, 247, 1306-10). The first method relies on the process of evolution, in which mutations are either accepted or rejected by natural selection. The second approach uses genetic engineering to introduce amino acid changes at specific positions of a cloned gene and selections or screens to identify sequences that maintain functionality.

These studies have revealed that proteins are surprisingly tolerant of amino acid substitutions and indicate which amino acid changes are likely to be permissive at a certain position of the protein. For example, most buried amino acid residues require nanvolar side chains, whereas few features of surface side chains are generally conserved. Other such phenorotypically silent substitutions are described by Bowie et al. (supra) and the references cited therein.

Typically seen as conservative substitutions are the replacements, one for another, among the aliphatic amino acids Ala, Val, Leu and Phe; interchange of the hydroxyl residues Ser and Thr; exchange of the acidic residues Asp and Glu; substitution between the amide residues Asn and Gln; exchange of the basic residues Lys and Arg; and replacements among the aromatic residues Phe, Tyr. In addition, the following groups of amino acids generally represent equivalent changes: (1) Ala, Pro, Gly, Gln, Asp, Gln, Asn, Ser, Thr, (2) Cys, Ser, Tyr, Thr; (3) Val, Ile, Leu, Met, Ala, Phe; (4) Lys, Arg, His; (5) Phe, Tyr, Trp, His.

Similarly, amino acids in the gOBG3 polypeptide fragment sequences described herein that are essential for function can also be identified by methods known in the art, such as site-directed mutagenesis or alanine-scanning mutagenesis (*see, e.g.,* Cunningham, et al. (1989) Science 244(4908):1081-5). The latter procedure introduces single alanine mutations at every residue in the molecule. The resulting mutant molecules are then tested for obesity-related activity using assays as described above. Of special interest are substitutions of charged amino acids with other charged or neutral amino acids that may produce proteins with highly desirable improved characteristics, such as less aggregation. Aggregation may not only reduce activity but also be problematic when preparing pharmaceutical or physiologically acceptable formulations, because aggregates can be immunogenic (*see, e.g.,* Pinckard, et al., (1967) Clin. Exp. Immunol 2:331-340; Robbins, et al., (1987) Diabetes Jul;36(7):838-41; and Cleland, et al., (1993) Crit Rev Ther Drug Carrier Syst. 10(4):307-77).
The variant of the gOBG3 fragment described herein may be, for example: one in which one or more of the amino acid residues are substituted with a conserved or non-conserved amino acid residue (preferably a conserved amino acid residue) and such substituted amino acid residue may or may not be one encoded by the genetic code (i.e. may be a non-naturally occurring amino acid). A gOBG3 fragment described herein may also be fused with another compound to increase the half-life of the fragment (polyethylene glycol); or one in which the additional amino acids of an IgG Fc fusion region peptide are fused to the gOBG3 fragment. Such variants and fusions are deemed to be within the scope of those skilled in the art from the teachings herein.
Preferably the variant has at least one conservative amino acid substitution but not more than 10, 9, 8, 7, 6, 5, 4, 3, 2 or 1 conservative amino acid substitutions.

Another specific embodiment of a modified gOBG3 fragment for use in described herein is a polypeptide that is resistant to proteolysis, for example a gOBG3 fragment in which a-CONH-peptide bond is modified and replaced by one or more of the following: a(CH2NH) reduced bond; a (NHCO) retro inverso boud; a (CH2-O) methylene-oxy bond; a (CH2-S) thiomethylene bond; a (CH2CH2) carba bond; a (CO-CH2) cetomethylene bond; a (CHOH-CH2) hydroxyethylene bond; a (N-N) bond; a E-alcene bond; or a -CH=CH- bond. Thus, the invention also encompasses use of gOBG3 fragment or a variant thereof as described herein in which at least one peptide bond has been modified as described above.

In addition, amino acids have chirality within the body of either L or D. In some embodiments it may be preferable to alter the chirality of the amino acids in the gOBG3 polypeptide fragments described herein in order to extend half-life within the body. Thus, in some embodiments, one or more of the amino acids are preferably in the L configuration. In other embodiments, one or more of the amino acids are preferably in the D configuration.

### Percent Identity

A polypeptide may include polypeptides having an amino acid sequence at least 50% identical, at least 60% identical, or 70%, 80%, 85%, 90%, 91%, 92%, 93%, 94%, 95%, 96%, 97%, 98% or 99% identical to an OBG3 or gOBG3 fragment. By a polypeptide having an amino acid sequence at least, for example, 95% "identical" to an OBG3 or gOBG3 fragment amino acid sequence is meant that the amino acid sequence is identical to the OBG3 or gOBG3 polypeptide fragment sequence except that it may include up to five amino acid alterations per each 100 amino acids of the OBG3 or gOBG3 polypeptide fragment amino acid sequence. The reference sequence is the OBG3 or gOBG3 polypeptide fragment with a sequence corresponding to the sequence of the sequence listing. Thus, to obtain a polypeptide having an amino acid sequence at least 95% identical to an OBG3 or gOBG3 fragment amino acid sequence, up to 5% (5 of 100) of the amino acid residues in the sequence may be inserted, deleted, or substituted with another amino acid compared with the OBG3 or gOBG3 polypeptide fragment sequence. These alterations may occur at the amino or carboxy termini or anywhere between those terminal positions, interspersed either individually among residues in the sequence or in one or more contiguous groups within the sequence.

As a practical matter, whether any particular polypeptide is a percentage identical to an OBG3 or gOBG3 fragment can be determined conventionally using known computer programs. Such algorithms and programs include, but are by no means limited to, TBLASTN, BLASTP, FASTA, TFASTA, and CLUSTALW (Pearson and Lipman, (1988) Proc Natl Acad Sci USA Apr;85(8):2444-8; Altschul et al., (1990) J. Mol. Biol. 215(3):403-410; Thompson et al., (1994) Nucleic Acids Res. 22(2):4673-4680; Higgins et al., (1996) Meth. Enzymol. 266:383-402; Altschul et al., (1997) Nuc. Acids Res. 25:3389-3402; Altschul et al., (1993) Nature Genetics 3:266-272). In a particularly preferred embodiment, protein and nucleic acid sequence homologies are evaluated using the Basic Local Alignment Search Tool ("BLAST"), which is well known in the art *(See, e.g.,* Karlin and Altschul (1990) Proc Natl Acad Sci USA Mar,87(6):2264-8; Altschul et al., 1990, 1993, 1997, all supra). In particular, five specific BLAST programs are used to perform the following tasks:
(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which is preferably obtained from a protein or nucleic acid sequence database. High-scoring segment pairs are preferably identified (*i.e.,* aligned) by means of a scoring matrix, many of which are known in the art. Preferably, the scoring matrix used is the BLOSUM62 matrix (see, Gonnet et al., (1992) Science Jun 5;256(5062):1443-5; Henikoff and Henikoff (1993) Proteins Sep;17(1):49-61). Less preferably, the PAM or PAM250 matrices may also be used (*See, e.g.,* Schwartz and Dayhoff, eds, (1978) Matrices for Detecting Distance Relationships: Atlas of Protein Sequence and Structure, Washington: National Biomedical Research Foundation). The BLAST programs evaluate the statistical significance of all high-scoring segment pairs identified, and preferably selects those segments which satisfy a user-specified threshold of significance, such as a user-specified percent homology. Preferably, the statistical significance of a high-scoring segment pair is evaluated using the statistical significance formula of Karlin (*See, e.g.,* Karlin and Altschul, (1990) Proc Natl Acad Sci USA Mar;87(6):2264-8). The BLAST programs may be used with the default parameters or with modified parameters provided by the user. Preferably, the parameters are default parameters.

A preferred method for determining the best overall match between a query sequence (a sequence of the present invention) and a subject sequence, also referred to as a global sequence alignment, can be determined using the FASTDB computer program based on the algorithm of Brutlag et al. (1990) Comp. App. Biosci. 6:237-245. In a sequence alignment the query and subject sequences are both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group=25 Length=0, Cutoff Score=1, Window Size=sequence length, Gap Penalty=5, Gap Size Penalty=0.05, Window Size=247 or the length of the subject amino acid sequence, whichever is shorter.

If the subject sequence is shorter than the query sequence due to N-or C-terminal deletions, not because of internal deletions, the results, in percent identity, must be manually corrected because the PASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N-and C-terminal of the subject sequence, that are not matehed/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameter, to arrive at a final percent identity score. This final percent identity score is what is used for the purposes of the present disclosure. Only residues to the N- and C-termini of the subject sequence, which are not matched/aligned with the query sequence, are considered for the purposes of manually adjusting the percent identity score. That is, only query amino acid residues outside the farthest N- and C-terminal residues of the subject sequence.

For example, a 90 amino acid residue subject sequence is aligned with a 100-residue query sequence to determine percent identity. The deletion occurs at the N-terminus of the subject sequence and therefore, the FASTDB alignment does notmatce/align with the first residues at the N-terminus. The 10 unpaired residues represent 10% of the sequence (number of residues at the N-and C-termini not matched/total number of residues in the query sequence) so 10% is subtracted from the percent identity score calculated by the FASTDB program. If the remaining 90 residues were perfectly matched the final percent identity would be 90%.

In another example, a 90-residue subject sequence is compared with 8 100-residue query sequence. This time the deletions are internal so there are no residues at the N-termind of the subject sequence, which are not matched/aligned with the query. In this case, the percent identity calculated by FASTDB is not manually corrected. Once again, only residue positions outside the N-and C-terminal ends of the subject sequence, as displayed in the FASTDB alignment, which are not matched/aligued with the query sequence are manually corrected. No other manual corrections are made for the purposes of the present disclosure.

### Production

Note, throughout the disclosure, wherever OBG3 polypeptide fragments are discussed, gOBG3fragments are specifically intended to be included as a preferred subset of OBG3 polypeptide fragments.

OBG3 polypeptide fragments are preferably isolated from human or mammalian tissue samples or expressed from human or mammalian genes in human or mammalian cells. The gOBG3 polypeptide fragments described herein can be made using routine expression methods known in the art. The polynucleotide encoding the desired polypeptide fragments is ligated into an expression vector suitable for any convenient host. Both eukaryotic and prokaryotic host systems are used in forming recombinant polypeptide fragments. The polypeptide fragment is then isolated from lysed cells or from the culture medium and purified to the extent needed for its intended use. Purification is by any technique known in the art, for example, differential extraction, salt fractionation, chromatography, centrifugation, and the like. See, for example, Methods in Enzymology for a variety of methods for purifying proteins. Also, see Examples 1-3 for methods previously used for OBG3 polypeptide fragments.

The polypeptides described herein may be isolated from milk. The polypeptides can be purified as full length OBG3 polypeptides, which can then be cleaved, if appropriate, in vitro to generate an OBG3 fragment, or, alternatively, OBG3 fragments themselves can be purified from the milk. Any of a large number of methods can be used to purify the present polypeptides from milk, including those taught in Protein Purification Applications, A Practical Approach (New Edition), Edited by Simon Roe, ABA Technology Products and Systems, Biosciences, Harwell; Clark (1998) J Mammary Gland Biol Neophsta 3:337-50; Wilkins and Velander (1992) 49:333-8; U.S. Patent Nos. 6,140,552; 6,025,540; Hennighausen, Protein Expression and Purification, vol. 1, pp. 3-8 (1990); Harris et al. (1997) Bioseparation 7:31-7; Degener et al. (1998) J. Chiomatog. 799:125-37; Wilkins (1993) J. Cell. Biochem. Soppl. 0 (17 part A):39. In a typical embodiment, milk is centrifuged, e.g. at a relatively low speed, to separate the lipid fraction, and the aqueous supernatant is then centrifuged at a higher speed to separate the casein in the milk from the remaining, "whey" fraction. Often, biomedical proteins are found in this whey fraction, and can be isolated from this fraction using standard chromatographic or other procedures commonly used for protein purification, e.g. as described elsewhere in the present application. OBG3 polypeptides may be purified using antibodies specific to OBG3 polypeptides, e.g. using affinity chromatography. In addition, methods can be used to isolate particular OBG3 fragments, e.g. electrophoretic or other methods for isolating proteins of a particular size. The OBG3 polypeptides isolated using these methods can be naturally occurring, as OBG3 polypeptides have been discovered to be naturally present in the milk of mammals (see, e.g. Example 17), or can be the result of the recombinant production of the protein in the mammary glands of a non-human mammal, as described infra. The OBG3 fragment may be produced as a fusion protein with a heterologous, antigenic polypeptide sequence, which antigenic sequence can be used to purify the protein, e.g., using standard immuno-affinity methodology.

In addition, shorter protein fragments may be produced by chemical synthesis. Alternatively, the proteins may be extracted from cells or tissues of humans or non-human animals. Methods for purifying proteins are known in the art, and include the use of detergents or chaotropic agents to disrupt particles followed by differential extraction and separation of the polypeptides by ion exchange chromatography, affinity chromatography, sedimentation according to density, and gel electrophoresis.

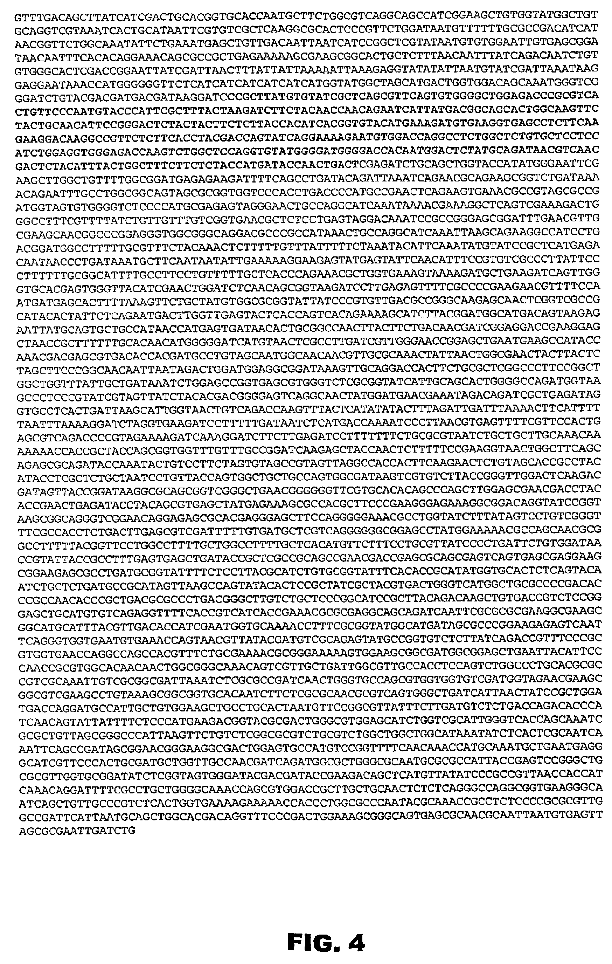

Any OBG3 fragment cDNA, including that in Fig. 4, can be used to express OBG3 polypeptide fragments. The nucleic acid encoding the OBG3 fragment to be expressed may be operably linked to a promoter in an expression vector using conventional cloning technology. The OBG3 fragment cDNA insert in the expression vector may comprise the coding sequence for: the full length OBG3 polypeptide (to be later modified); from 6 amino acids to 6 amino acids less than the full-length OBG3 polypeptide; a gOBG3 fragment: or variants and % similar polypeptides.

The expression vector may be any of the mammalian, yeast, insect or bacterial expression systems known in the art, some of which are described herein, and examples of which are given in the Examples (Examples 1-3). Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon context and codon pairing of the sequence can be optimized for the particular expression organism into which the expression vector is introduced, as explained by Hatfield, et al., U.S. Patent No. 5,082,767.

If the nucleic acid encoding OBG3 polypeptide fragments lacks a methionine to serve as the initiation site, an initiating methionine can be introduced next to the first codon of the nucleic acid using conventional techniques. Similarly, if the insert from the OBG3 polypeptide fragment cDNA lacks a poly A signal, this sequence can be added to the construct by, for example, splicing out the Poly A signal from pSG5 (Stratagene) using BglI and Sall restriction endonuclease enzymes and incorporating it into the mammalian expression vector pXT1 (Stratagene). pXT1 contains the LTRs and a portion of the gag gene from Moloney Murine Leukemia Virus. The position of the LTRs in the construct allow efficient stable transfection. The vector includes the Herpes Simplex Thymidine Kinase promoter and the selectable neomycin gene.

The nucleic acid encoding an OBG3 fragment can be obtained by PCR from a vector containing the OBG3 nucleotide sequence using oligonucleotide primers complementary to the desired OBG3 cDNA and containing restriction endonuclease sequences for Pst I incorporated into the 5' primer and BglII at the 5' end of the corresponding cDNA 3' primer, taking care to ensure that the sequence encoding the OBG3 fragment is positioned properly with respect to the poly A signal. The purified fragment obtained from the resulting PCR reaction is digested with PstI, blunt ended with an exonuclease, digested with Bgl II, purified and ligated to pXT1, now containing a poly A signal and digested with BglII. Alternative methods are presented in Examples 1-3.

Transfection of an OBG3 fragment-expressing vector into mouse NIH 3T3 cells is one method of introducing polynucleotides into host cells. Introduction of a polynucleotide encoding a polypeptide into a host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al. ((1986) Methods in Molecular Biology, Elsevier Science Publishing Co., Inc., Amsterdam). It is specifically contemplated that the polypeptides described herein may in fact be expressed by a host cell lacking a recombinant vector. Methods of expressing OBG3 fragments in cells are described in Examples 1-3.

A polypeptide (i.e. a gOBG3 fragment) can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography Most preferably, high performance liquid chromatography ("HPLC") is employed for purification. Polypeptides described herein, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells.

Depending upon the host employed in a recombinant production procedure, the polypeptides described herein may be glycosylated or may be non-glycosylated. Preferably the polypeptides described herein are non-glycosylated. In addition, polypeptides described herein may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In addition to containing the vector constructs discussed herein, host cells may encompass primary, secondary, and immortalized host cells of vertebrate origin, particularly mammalian origin, that have been engineered to delete or replace endogenous genetic material (*e.g.,* coding sequence), and/or to include genetic material (*e.g.,* heterologous polynucleotide sequences) that is operably associated with the polynucleotides described herein and which activates, alters, and/or amplifies endogenous polynucleotides. For example, techniques known in the art may be used to operably associate heterologous control regions (*e.g.,* promoter and/or enhancer) and endogenous polynucleotide sequences via homologous recombination, see, *e.g.,* U.S. Patent No. 5,641,670, issued June 24, 1997; International Publication No. WO 96/29411, published September 26,1996; International Publication No. WO 94/12650, published August 4, 1994; Koller et al., (1989) Proc Natl Acad Sd USA Nov;86(22):8932-5;Koller et al., (1989) Proc Natl Acad Sci USA Nov;86(22):8927-31; and Zijlstra et al. (1989) Nature Nov 23;342(6248):435-8;

### Modifications

In addition, polypeptides described herein can be chemically synthesized using techniques known in the art *(See, e.g.,* Creighton, 1983 Proteins. New York, New Yodc W.H. Freeman and Company; and Hunkapiller et al., (1984) Nature Jul 12-19-310(5973):105-11). For example, a relative short fragment described herein can be synthesized by use of a peptide synthesizer. Furthermore, if desired, nonclassical amino acids or chemical amino acid analogs can be introduced as a substitution into the fragment sequence Non-classical amino acids include, but are not limited to, to the D-isomers of the common amino acids, 2,4-diaminobutyric add, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, b-alanine, fluoroamino acids, designer amino acids such as b-methyl amino acids, Ca-methyl amino acids, Na-methyl amino acids, and amino acid analogs in general. Furthermore, the amino acid can be D (dextrorotary) or L (levorotary).

Polypeptide fragments may be differentially modified during or after translation, *e.g.,* by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, NaBH4; acetylation, formylation, oxidation, reduction; metabotic synthesis in the presence of tunicamycin; etc.

Additional post-translational modifications include, for example, N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptide fragments may also be modified with a detectable label, such as an enzymatic, fluorescent isotopic or affinity label to allow for detection and isolation of the polypeptide.

Also provided by the invention are uses of chemically modified derivatives of the polypeptides described herein that may provide additional advantages such as increased solubility, stability and circulating time of the polypeptide, or decreased immunogenicity. See U.S. Patent No: 4,179,337. The chemical moiety for derivitization is polyethylene glycol. The polypeptides may be modified at random positions within the molecule, or at predetermined positions within the molecule and may include one, two, three or more attached chemical moieties.

The polymer may be of any Molecular weight, and may be blanched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog).

The polyethylene glycol molecules should be attached to the polypeptide with consideration of effects on functional or antigenic domains of the polypeptide. There are a number of attachment methods available to those skilled in the art, e.g., EP 0 401 384, (coupling PBG to G-CSF), see also Malik et al. (1992) Exp Hematol. Sep;20(8):1028-35, reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues, glutamic acid residues and the C-tenninal amino acid residue. Sulthydryl groups may also be used as a reactive group for attaching the polyethylene glycol molecules. Preferred for therapeutic purposes is attachment at an amino group, such as attachment at the N-terminus or lysine group.

One may specifically desire proteins chemically modified at the N-terminus. Using polyethylene glycol as an illustration of the present composition, one may select from a variety of polyethylene glycol molecules (by molecular weight, branching, etc.), the proportion of polyethylene glycol molecules to protein (polypeptide) molecules in the reaction mix, the type of pegylation reaction to be performed, and the method of obtaining the selected N-teminally pegylated protein. The method of obtaining the N-teaninally pegylated preparation (i.e., separating this moiety from other monopegylated moieties if necessary) may be by purification of the N-terminally pegylated material from a population of pegylated protein molecules. Selective proteins chemically modified at the N-terminus may be accomplished by reductive alkylation, which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

### Multimers

The polypeptide fragments described herein may be in monomers or multimers (i.e., dimers, trimers, tetramers and higher multimers). Accordingly, the present disclosure relates to monomers and multimers of polypeptide fragments described herein their preparation, and compositions (preferably, pharmaceutical or physiologically acceptable compositions) containing them. In specific embodiments, the polypeptides described herein are monomers, dimers, timers or tetramers; In additional embodiments, the multimers are at least dimers, at least trimers, or at least tetramers.

Multimers for use in the invention may be homomers or heteromers. As used herein, the term homomer, refers to a multimer containing only polypeptides corresponding to the gOBG3 polypeptide fragments described herein (including polypeptide fragments, variants, splice variants, and fusion proteins corresponding to these polypeptide fragments as described herein). These homomers may contain polypeptide fragments having identical or different amino acid sequences. In a specific embodiment a homomer is a multimer containing only polypeptide fragments having an identical amino acid sequence. In another specific embodiment, a homomer is a multimer containing polypeptide fragments having different amino acid sequences. In specific embodiments, the multimer is a homodimer (*e.g.*, containing polypeptide fragments having identical or different amino acid sequences) or a homotrimer (*e.g.*, containing polypeptide fragments having identical and/or different amino acid sequences). In additional embodiments, the homomeric multimer is at least a homodimer, at least a homotrimer, or at least a homotetramer.

As used herein, the term heteromer refers to a multimer containing one or more heterologous polypeptides (*i.e*., corresponding to different proteins or polypeptide fragments thereof) in addition to the polypeptides for use or described herein. In a specific embodiment, the multimer is a heterodimer, a heterotrimer, or a heterotetramer. In additional embodiments, the heteromeric multimer is at least a heterodimer, at least a heterotrimer, or at least a heterotetramer.

Multimers described herein be the result of hydrophobic, hydrophilic, ionic and/or covalent associations and/or may be indirectly linked, by for example, liposome formation. Thus, in one embodiment, multimers, such as, for example, homodimers or homotrimers, are formed when polypeptides described herein contact one another in solution. In another embodiment, heteromultimer, such for example, heterotrimers or heterotetramers, are formed when polypeptides described herein contact antibodies to the polypeptides described herein including antibodies to the heterologous polypeptide sequence in a fusion protein described herein in solution. In other embodiments, multimers are formed by covalent associations with and/or between the polypeptides described herein. Such covalent associations may involve one or more amino acid residues contained in the polypeptide sequence (e.g., that recited in the sequence listing, or contained in the polypeptide encoded by a deposited clone). In one instance, the covalent associations are cross-linking between cysteine residues located within the polypeptide sequences, which interact in the native (*i.e.,* naturally occurring) polypeptide. In another instance, the covalent associations are the consequence of chemical or recombinant manipulation. Alternatively, such covalent associations may involve one or more amino acid residues contained in the heterologous polypeptide sequence in a fusion protein described herein.

In one example, covalent associations are between the heterologous sequence contained in a fusion protein (see. *e.g.,* US Patent Number 5,478,925). In a specific example, the covalent associations are between the heterologous sequence contained in an Fc fusion protein (as described herein). In another specific example, covalent associations of fusion proteins are between heterologous polypeptide sequence from another protein that is capable of forming covalently associated multimers, such as for example, oseteoprotegerin (see, e.g., International Publication NO: WO 98/49305 ). In another embodiment, two or more polypeptides described herein may be joined through peptide linkers. Examples include those peptide linkers described in U.S. Pat. No. 5,073,627. Proteins comprising multiple polypeptides described herein separated by peptide linkers may be produced using conventional recombinant DNA technology.

Another method for preparing multimer polypeptides described herein involves use of polypeptides described herein fused to a leucine zipper or isoleucine zipper polypeptide sequence. Leucine zipper and isoleucine zipper domains are polypeptides that promote multimerization of the proteins in which they are found. Leucine zippers were originally identified in several DNA-binding proteins, and have since been found in a variety of different proteins (Landschulz et al., (1988) Genes Dev. Jul;2(7):786-800). Among the known leucine zippers are naturally occurring peptides and derivatives thereof that dimerize or trimerize. Examples of leucine zipper domains suitable for producing soluble multimeric proteins are those described in PCT application WO 94/10308 Recombinant fusion proteins comprising a polypeptide described herein fused to a polypeptide sequence that dimerizes or trimerizes in solution may be expressed in suitable host cells, and the resulting soluble multimeric fusion protein recovered from the culture supernatant using techniques known in the art.

Trimeric polypeptides may offer the advantage of enhanced biological activity. Preferred leucine zipper moieties and isoleucine moieties are those that preferentially form trimers. One example is a leucine zipper derived from lung surfactant protein D (SPD), as described in Hoppe et al. FEBS Letters (1994) May 16;344(2-3):191-5. and in U.S. patent application Ser. No. 08/446,922 Other peptides derived from naturally occurring trimeric proteins may be employed in preparing trimeric polypeptides. In another example, proteins described herein may be associated by interactions between Flag® & polypeptide sequence contained in fusion proteins containing Flag® polypeptide sequence. In a further embodiment, proteins formula in described herein may be associated by interactions between heterologous polypeptide sequence contained in Flag® fusion proteins and anti Flag® antibody.

The multimers described herein may be generated using chemical techniques known in the art. For example, polypeptides desired to be contained in the multimers described herein may be chemically cross-linked using linker molecules and linker molecule length optimization techniques known in the art (see, e.g., US Patent Number 5,478,925). Additionally, multimers may be generated using techniques known in the art to form one or more inter-molecule cross-links between the cysteine residues located within the sequence of the polypeptides desired to be contained in the multimer (see, e.g., US Patent Number 5,478,925). Further, polypeptides described herein may be routinely modified by the addition of cysteine or biotin to the C-terminus or N-terminus of the polypeptide and techniques known in the art may be applied to generate multimers containing one or more of these modified polypeptides (*see*, *e.g.*, US Patent Number 5,478,925, ). Additionally, at least 30 techniques known in the art may be applied to generate liposomes containing the polypeptide components desired to be contained in the multimer (see, e.g., US Patent Number 5,478,925,).

Alternatively, multimers described herein may be generated using genetic engineering techniques known in the art. In one embodiment, polypeptides contained in multimers may be produced recombinantly using fusion protein technology described herein or otherwise known in the art (see, e.g., US Patent Number 5,478,925,). In a specific embodiment, polynucleotides coding for a homodimer may be generated by ligating a polynucleotide sequence encoding a polypeptide described herein to a sequence encoding a linker polypeptide and then further to a synthetic polynucleotide encoding the translated product of the polypeptide in the reverse orientation from the original C-terminus to the N-terminus (lacking the leader sequence) (see, e.g., US Patent Number 5,478,925,). In another embodiment, recombinant techniques described herein or otherwise known in the art may be applied to generate recombinant polypeptides described herein which contain a transmembrane domain (or hyrophobic or signal peptide) and which can be incorporated by membrane reconstitution techniques into liposomes (*See, e.g.,* US Patent Number 5,478,925,).

### II. OBG3 Polynucleotides

Preferred polynucleotides are those that encode gOBG3 polypeptide fragments described herein. The recombinant polynucleotides encoding gOBG3 polypeptide fragments can be used in a variety of ways, including, but not limited to, expressing the polypeptide in recombinant cells for use in screening assays for antagonists and agonists of its activity as well as to facilitate its purification for use in a variety of ways including, but not limited to screening assays for agonists and antagonists of its activity, diagnostic screens, and raising antibodies, as well as treatment and/or prevention of obesity-related diseases and disorders and/or to reduce body mass.

Polynucleotides encoding OBG3 and gOBG3 polypeptide fragments and variant polypeptide fragments thereof are may be purified, isolated, and/or recombinant. In all cases, the desired gOBG3 polynucleotides are those that encode gOBG3 polypeptide fragments of the invention having obesity-related activity as described and discussed herein.

### Fusions

Polynucleotides encoding the polypeptides described herein may be fused in frame to the coding sequences for additional heterologous amino acid sequences. Also, nucleic acids may encode polypeptides described herein together with additional, non-coding sequences, including for example, but not limited to non-coding 5' and 3' sequences, vector sequence, sequences used for purification, probing, or priming. For example, heterologous sequences include transcribed, non-translated sequences that may play a role in transcription, and mRNA processing for example, ribosome binding and stability of mRNA. The heterologous sequences may alternatively comprise additional coding sequences that provide additional functionalities. Thus, a nucleotide sequence encoding a polypeptide may be fused to a tag sequence, such as a sequence encoding a peptide that facilitates purification of the fused polypeptide. The tag amino acid sequence may be such as the tag provided in a pQE vector (QIAGEN. Inc., 9259 Eton Avenue, Chatsworth, CA, 91311), among others, many of which am commercially available. For instance, hexa-histidine provides for convenient purification of the fusion protein (See, Gentz et al; (1989) Proc Natl Acad Sci USA Feb;86(3):821-4). The "HA" tag is another peptide useful for purification which corresponds to an epitope derived from the influenza hemagglutinin protein (See, Wilson et al., (1984) Cell 37(3):767-78). As discussed above, fusion proteins for use as described in the first aspect include gOBG3 fragments fused to IgG Fc at the N- or C-terminus.

### III. Recombinant Vectors

The term "vector' is used herein to designate either a circular or a linear DNA or RNA molecule, that is either double-stranded or single-stranded, and that comprises at least one polynucleotide of interest that is sought to be transferred in a cell host or in a unicellular or multicellular host organism.
A recombinant vector may comprise a promoter operably linked to a coding region capable of expressing (a) a globular OBG3 fragment or variant for use in the first aspect; or (b) a fusion of said globular OBG3 fragment wherein the fragment is in the form of an IgG Fc fusion peptide.
Expression vectors may be employed to express such a globular OBG3 fragment, variant or fusion peptide which can then be purified and, for example, be used as a treatment for obesity-related diseases, or simply to reduce body mass of individuals.

Expression requires that appropriate signals are provided in the vectors, said signals including various regulatory elements, such as enhancers/promoters from both viral and mammalian sources, that drive expression of the genes of interest in host cells. Dominant drug selection markers for establishing permanent, stable, cell clones expressing the products may be included in expression vectors as they are elements that link expression of the drug selection markers to expression of the polypeptide.

Expression vectors may include nucleic acids encoding a gOBG3 fragment described herein, or a modified gOBG3 fragment as described herein, or variants or fragments thereof, under the control of a regulatory sequence selected among OBG3 polypeptide fragments, or alternatively under the control of an exogenous regulatory sequence.

Consequently, such expression vectors may consist of: (a) an OBG3 fragment regulatory sequence and driving the expression of a coding polynucleotide operably linked thereto; and (b) fragment coding for a gOBG3 sequences of the described herein operably linked to regulatory sequences allowing its expression in a suitable cell host and/or host organism.

Some of the elements which can be found in the vectors are described in further detail in the following sections.

### 1) General features of expression vectors

A recombinant vector may comprise, but is not limited to, a YAC (Yeast Artificial Chromosome), a BAC (Bacterial Artificial Chromosome), a phage, a phagemid, a cosmid, a plasmid, or even a linear DNA molecule which may consist of a chromosomal, non-chromosomal, semi-synthetic or synthetic DNA. Such a recombinant vector can comprise a transcriptional unit comprising an assembly of:
(1) a genetic element or elements having a regulatory role in gene expression, for example promoters or enhancers. Enhancers are cis-acting elements of DNA, usually from about 10 to 300 bp in length that act an the promoter to increase the transcription;
(2) a structural or coding sequence which is transcribed into mRNA and eventually translated into a polypeptide, said structural or coding sequence being operably linked to the regulatory elements described in (1); and
(3) appropriate transcription initiation and termination sequences. Structural units intended for use in yeast or eukaryotic expression systems preferably include a leader sequence enabling extracellular secretion of translated protein by a host cell Alternatively, when a recombinant protein is expressed without a leader or transport sequence, it may include a N-terminal residue. This residue may or may not be subsequently cleaved from the expressed recombinant protein to provide a final product.

Generally, recombinant expression vectors will include origins of replication, selectable markers permitting transformation of the host cell, and a promoter derived from a highly expressed gene to direct transcription of downstream structural sequence. The heterologous structural sequence is assembled in appropriate phase with translation initiation and termination sequences, and preferably a leader sequence capable of directing secretion of the translated protein into the periplasmic space or the extracellular medium. In a specific embodiment wherein the vector is adapted for transfecting and expressing desired sequences in mammalian host cells, preferred vectors will comprise an origin of replication in the desired host, a suitable promoter and enhancer, and also any necessary ribosome binding sites, polyadenylation sites, splice donor and acceptor sites, transcriptional termination sequences, and 5'-flanking non-transcribed sequences. DNA sequences derived from the SV40 viral genome, for example SV40 origin, early promoter, enhancer, splice and polyadenylation sites may be used to provide the required non-transcribed genetic elements.

### 2) Resulatory elements

### Promoters

The suitable promoter regions used in expression vectors are chosen taking into account the cell host in which the heterologous gene is expressed. The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell, such as, for example, a human or a viral promoter.

A suitable promoter may be heterologous with respect to the nucleic acid for which it controls the expression or alternatively can be endogenous to the native polynucleotide containing the coding sequence to be expressed. Additionally, the promoter is generally heterologous with respect to the recombinant vector sequences within which the construct promoter/coding sequence has been inserted.

Promoter regions can be selected from any desired gene using, for example, CAT (chloramphenicol transferase) vectors and more preferably pKK232-8 and pCM7 vectors. Preferred bacterial promoters are the LacI, LacZ, the T3 or T7 bacteriophage RNA polymerase promoters, the gpt, lambda PR, PL and trp promoters (EP 0036776), the polyhedrin promoter, or the p10 protein promoter from baculovirus (Kit Novagen) (Smith et al., (1983) Mol Cell Biol Dec;3(12):2156-65; O'Reilly et al., 1992), the lambda PR promoter or also the trc promoter.

Eukaryotic promoters include CMV immediate early, HSV thymidine kinase, early and late SV40, LTRs from retrovirus, and mouse metallothionein-L. In addition, promoters specific for a particular cell type may be chosen, such as those facilitating expression in adipose tissue, muscle tissue, or liver. Selection of a convenient vector and promoter is well within the level of ordinary skill in the art.

The choice of a promoter is well within the ability of a person skilled in the field of genetic engineering. For example, one may refer to Sambrook et al. (1989) Molecular Cloning: A Laboratory Manual, Cold Spring Harbor Laboratory Press, NY, Vol. 1,2,3 (1989), or also to the procedures described by Fuller et al. (1996) Immunology in Current Protocols in Molecular Biology.

### Other regulatory elements

Where a cDNA insert is employed, one will typically desire to include a polyadenylation signal to effect proper polyadenylation of the gene transcript. The nature of the polyadenylation signal is not believed to be crucial to the successful practice of the invention, and any such sequence may be employed such as human growth hormone and SV40 polyadenylation signals. Also contemplated as an element of the expression cassette is a terminator. These elements can serve to enhance message levels and to minimize read through from the cassette into other sequences.

Vectors containing the appropriate DNA sequence as described above can be utilized to transform an appropriate host to allow the expression of the desired polypeptide or polynucleotide.

### 3) Selectable markers

Such markers would confer an identifiable change to the cell permitting easy identification of cells containing the expression construct. The selectable marker genes for selection of transformed host cells are preferably dihydrofolate reductase or neomycin resistance for eukaryotic cell culture, TRP1 for S. cerevisiae or tetracycline, rifampicin or ampicillin resistance in *E. coli,* or levan saccharase for mycobacteria, this latter marker being a negative selection marker.

### 4) Preferred vectors

### Bacterial vectors

As a representative but non-limiting example, useful expression vectors for bacterial use can comprise a selectable marker and a bacterial origin of replication derived from commercially available plasmids comprising genetic elements of pBR322 (ATCC 37017). Such commercial vectors include, for example, pKK223-3 (Pharmacia, Uppsala, Sweden), and pGEM1 (Promega Biotec, Madison, WI, USA).

Large numbers of other suitable vectors are known to those of skill in the art, and are commercially available, such as the following bacterial vectors: pQE70, pQE60, pQE-9 (Qiagen), pbs, pD10, phagescript, psiX174, pbluescript SK, pbsks, pNH8A, pNH16A, pNH18A, pNH46A (Stratagene); ptrc99a, pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIAexpress).

### Baculovirus vectors

A suitable vector for the expression of polypeptides described herein is a baculovirus vector that can be propagated in insect cells and in insect cell lines. A specific suitable host vector system is the pVL1392/1393 baculovirus transfer vector (Pharmingen) that is used to transfect the SF9 cell line (ATCC N°CRL 1711) which is derived from Spodoptera frugiperda.

Other suitable vectors for the expression of an Apml globular head polypeptide in a baculovirus expression system include those described by Chai et al. (1993; Biotechnol Appl Biochem. Dec;18 (Pt 3):259-73); Vlasak et al. (1983; Eur J Biochem Sep 1;135(1):123-6); and Lenhard et al. (1996; Gene Mar 9;169(2):187-90).

### Viral vectors

The vector of the present disclosure may be derived from an adenovirus. Preferred adenovirus vectors are those described by Feldman and Steg (1996; Semin Interv Cardiol Sep;1(3):203-8) or Ohno et al. (1994; Science Aug 5;265(5173):781-4). Another preferred recombinant adenovirus is the human adenovirus type 2 or 5 (Ad 2 or Ad 5) or an adenovirus of animal origin (Freach patent application No.FR-93.05954).

Retrovirus vectors and adeno-associated virus vectors are generally understood to be the recombinant gene delivery systems of choice for the transfer of exogenous polynucleotides *in vivo,* particularly to mammals, including humans. These vectors provide efficient delivery of genes into cells, and the transferred nucleic acids are stably integrated into the chromosomal DNA of the host.

Particularly preferred retroviruses for the preparation or construction of retroviral *in vitro* or *in vivo* gene delivery vehicles include retroviruses selected from the group consisting of Mink-Cell Focus Inducing Virus, Murine Sarcoma Virus, Reticuloendotheliosis virus and Rous Sarcoma vims. Particularly preferred Murine Leukemia include the 4070A and the 1504A viruses, Abelson (ATCC No VR-999). Friend (ATCC No VR-245), Gross (ATCC No VR-590), Rauscher (ATCC No VR-998) and Moloney Murine Leukemia Virus (ATCC No VR-190; PCT Application No WO 94/24298). Particularly preferred Rous Sarcoma Viruses include Bryan high titer (ATCC Nos VR-334, VR-657, VR-726, VR-659 and VR-728). Other preferred retroviral vectors are those described in Roth et al. (1996), PCT Application No WO 93/25234, PCT Application No WO 94/ 06920, Roux et al., ((1989) Proc Natl Acad Sci U S A Doc;86(23):9079-83), Julan et al., (1992) J. Gen. Virol. 3:3251-3255 and Neda et al., ((1991) J Biol Chem Aug 5;266(22):14143-6).

Yet another viral vector system that is contemplated consists of the adeno-associated virus (AAV). The adeno-associated virus is a naturally occurring defective virus that requires another virus, such as an adenovirus or a herpes virus, as a helper virus for efficient replication and a productive life cycle (Muzyczka et al., (1992) Curr Top Microbiol Immunol;158:97-129). It is also one of the few viruses that may integrate its DNA into non-dividing cells, and exhibits a high frequency of stable integration (Flotte et al., (1992) Am J Respir Cell Mol Biol Sep;7(3):349-56; Samulski et al., (1989) J Virol Sep;63(9):3822-8; McLanghlin et al., (1989) Am. J. Hum. Genet. 59:561-569). One advantageous Mature of AAV derives from its reduced efficacy for transducing primary cells relative to transformed cells.

### 5) Delivery of the recombinant vectors

In order to effect expression of the polynucleotides described herein, these constructs must be delivered into a cell. This delivery may be accomplished *in vitro,* as in laboratory procedures for transforming cell lines, or *in vivo* or *ex vivo,* as in the treatment of certain disease states.

One mechanism is viral infection where the expression construct is encapsulated in an infectious viral particle.

Several non-viral methods for the transfer of polynucleotides into cultured mammalian cells are also contemplated and include, without being limited to, calcium phosphate precipitation (Graham et al., (1973) Virology Aug;54(2):536-9; Chen et al., (1987) Mol Cell Biol Aug;7(8):2745-52), DEAE-dextran (Gopal, (1985) Mol Cell Biol May;5(5):1188-90), electroporation (Tur-Kaspa et al., (1986) Mol Cell Biol Feb;6(2):716-8; Potter et al., (1984) Proc Natl Acad Sci USA Nov;81(22):7161-5.), direct microinjection (Harland et al., (1985) J Cell Biol Sep;101(3):1094-9), DNA-loaded liposomes (Nicolau et al., (1982) Biochim Biophys Acta Oct 11;721(2):185-90; Fraley et al., (1979) Proc Natl Acad Sci USA Jul;76(7):3348-52), and receptor-mediated transfection (Wu and Wu, (1987) J Biol Chem Apr 5;262(10):4429-32; Wu and Wu (1988) Biochemistry Feb 9;27(3):887-92). Some of these techniques may be successfully adapted for *in vivo* or *ex vivo* use.

Once the expression polynucleotide has been delivered into the cell, it may be stably integrated into the genome of the recipient cell. This integration may be in the cognate location and orientation via homologous recombination (gene replacement) or it may be integrated in a random, non specific location (gene augmentation). In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle.

One specific embodiment for a method for delivering a protein or peptide to the interior of a cell of a vertebrate *in vivo* comprises the step of introducing a preparation comprising a physiologically acceptable carrier and a naked polynucleotide operatively coding for the polypeptide of interest into the interstitial space of a tissue comprising the cell, whereby the naked polynucleotide is taken up into the interior of the cell and has a physiological effect. This is particularly applicable for transfer *in vitro* but it may be applied to *in vivo* as well.

Compositions for use *in vitro* and *in vivo* comprising a "naked" polynucleotide are described in PCT application No. WO 90/11092 (Vical Inc.) and also in PCT application No. WO 95/11307 (Institut Pasteur, INSERM, Université d'Ottawa) as well as in the articles of Tascon et al. (1996) Nature Medicine. 2(8):888-892 and of Huygen et al. ((1996) Nat Med Aug;2(8):893-8).

In still another embodiment, the transfer of a naked polynucleotide, including a polynucleotide construct, into cells may be proceeded with a particle bombardment (biolistic), said particles being DNA-coated microprojectiles accelerated to a high velocity allowing them to pierce cell membranes and enter cells without killing them, such as described by Klein et al. ((1990) Curr Genet Feb;17(2):97-103).

In a further embodiment, the polynucleotide may be entrapped in a liposome (Ghosh and Bacchawat, (1991) Targeted Diagn Ther;4:87-103; Wong et al., (1980) Gene 10:87-94; Nicolau et al., (1987) Methods Enzymol.;149:157-76). These liposomes may further be targeted to cells expressing LSR by incorporating leptin, triglycerides, ACRP30, or other known LSR ligands into the liposome membrane.

In a specific embodiment, a composition may be provided for the *in vivo* production of an Apml globular head polypeptide described herein. It comprises a naked polynucleotide operatively coding for this polypeptide, in solution in a physiologically acceptable carrier, and suitable for introduction into a tissue to cause cells of the tissue to express the said polypeptide.

The amount of vector to be injected to the desired host organism varies according to the site of injection. As an indicative dose, it will be injected between 0.1 and 100 µg of the vector in an animal body, preferably a mammal body, for example a mouse body.

In another embodiment of the vector, it may be introduced *in vitro* in a host cell, preferably in a host cell previously harvested from the animal to be treated and more preferably a somatic cell such as a muscle cell. In a subsequent step, the cell that has been transformed with the vector coding for the desired Apml globular head polypeptide or the desired fragment thereof is reintroduced into the animal body in order to deliver the recombinant protein within the body either locally or systemically.

### IV. Recombinant cells

Host cells may comprise a recombinant vector as described above These may include host cells that are transformed (prokaryotic cells) or that are transfected (eukaryotic cells) with a recombinant vector such as any one of those described in "Recombinant Vectors".

Generally, a recombinant host cell may comprise at least one of the recombinant vectors that are described herein.

Preferred host cells used as recipients for the recombinant vectors are the following:
a) Prokaryotic host cells: *Escherichia coli* strains (I.E. DH5-α strain), *Bacillus subtilis, Salmonella typhimurium,* and strains from species like *Pseudomonas*, *Streptomyces* and *Staphylococcus,* and
b) Bukaryotic host cells : HeLa cells (ATCC N°CCL2; N°CCL2.1; N°CCL2.2), Cv 1 cells (ATCCN°CCL70), COS cells (ATCC N°CRL1650; N°CRL1651), Sf-9 cells (ATCC N°CRL1711), C127 cells (ATCC N° CRL-1804), 3T3 (ATCC N° CRL-6361), CHO (ATCCN° CCL-61), human kidney 293 (ATCC N° 45504; N° CRL-1573), BHK (ECACC N° 84100501; N° 84111301), PLC cells, HepG2, and Hep3B.

The constructs in the host cells can be used in a conventional manner to produce the gene product encoded by the recombinant sequence.

Following transformation of a suitable host and growth of the host to an appropriate cell density, the selected promoter is induced by appropriate means, such as temperature shift or chemical induction, and cells are cultivated for an additional period.

Cells are typical harvested by centrifugation, disrupted by physical or chemical means, and the resulting crude extract retained for further purification.

Microbial cells employed in the expression of proteins can be disrupted by any convenient method, including freeze-thaw cycling, sonication, mechanical disruption, or use of cell lysing agents. Such methods are well known by the skilled artisan.

Further, these recombinant cells can be created *in vitro* or *in vivo* in an animal, preferably a mammal, most preferably selected from the group consisting of mice, rats, dogs, pigs, sheep, cattle, and primates, not to include humans. Recombinant cells created *in vitro* can also be later surgically implanted in an animal, for example. Methods to create recombinant cells *in vivo* in animals are well-known in the art.

Primary, secondary, and immortalized homologously recombinant host cells of vertebrate origin, preferably mammalian origin and particularly human origin, may be engineered to: a) insert exogenous (heterologous) polynucleotides into the endogenous chromosomal DNA of a targeted gene, b) delete endogenous chromosomal DNA, and/or c) replace endogenous chromosomal DNA with exogenous polynucleotides. Insertions, deletions, and/or replacements of polynucleotide sequences may be to the coding sequences of the targeted gene and/or to regulatory regions, such as promoter and enhancer sequences, operably associated with the targeted gene.

A homologously recombinant: host cell may be made *in vitro* or *in vivo,* wherein the expression of a targeted gene not normally expressed in the cells in altered. Preferably the alteration causes expression of the targeted gene under normal growth conditions or under conditions suitable for producing the polypeptide encoded by the targeted gene. The method of making such a cell may comprise the steps of (a) transfecting the cell *in vitro* or *in vivo* with a polynucleotide construct, the polynucleotide construct comprising; (i) a targeting sequence; (ii) a regulatory sequence and/or a coding sequence; and (iii) an unpaired splice donor site, if necessary, thereby producing a transfected cell; and (b) maintaining the transfected cell *in vitro* or *in vivo* under conditions appropriate for homologous recombination.

A method of altering the expression of a targeted gene in a cell *in vitro* or *in vivo* wherein the gene is not normally expressed in the cell, may comprise the steps of: (a) transfecting the cell in *vitro* or *in vivo* with a polynucleotide construct, the polynucleotide construct comprising: (i) a targeting sequence; (ii) a regulatory sequence and/or a coding sequence; and (iii) an unpaired splice donor site, if necessary, thereby producing a transacted cell; and (b) maintaining the transfected cell in *vitro* or *in vivo* under conditions appropriate for homologous recombination, thereby producing a homologously recombinant cell; and (c) maintaining the homologously recombinant cell *in vitro* or *in vivo* under conditions appropriate for expression of the gene.

A method of making a gOBG3 polypeptide fragment described herein may comprise altering the expression of a targeted endogenous gene in a cell *in vitro* or *in vivo* the wherein the gene is not normally expressed in the cell, comprising the steps of: a) transfecting the cell *in vitro* with a polynucleotide construct, the polynucleotide construct comprising: (i) a targeting sequence; (ii) a regulatory sequence and/or a coding sequence; and (iii) an unpaired splice donor site, if necessary, thereby producing a transfected cell; (b) maintaining the transfected cell in *vitro* or *in* vivo under conditions appropriate for homologous recombination, thereby producing a homologously recombinant cell; and c) maintaining the homologously recombinant cell *in vitro* or in *vivo* under conditions appropriate for expression of the gene thereby making the polypeptide.

A polynucleotide construct may alters the expression of a targeted gene in a cell type in which the gene is not normally expressed. This occurs when a polynucleotide construct is inserted into the chromosomal DNA of the target cell, wherein the polynucleotide construct comprises: a) a targeting sequence; b) a regulatory sequence and/or coding sequence; and c) an unpaired splice-donor site, if necessary. Further, polynucleotide constructs, as described above, may further comprise a polynucleotide which encodes a polypeptide and is in-frame with the targeted endogenous gene after homologous recombination with chromosomal DNA.

The compositions may be produced, and methods performed, by techniques known in the art, such as those described in U.S. Patent Nos: 6,054,288; 6,048,729; 6,048,724; 6,048,524; 5,994,127; 5,968,502; 5,965,125; 5,869,239; 5,817,789; 5,783,385; 5,733,761; 5,641,670; 5,580,734 ; International Publication Nos:WO96/29411, WO 94/12650; and scientific articles described by Koller et al., (1994) Annu. Rev. Immunol. 10:705-730.

The OBG3 gene expression in mammalian, and typically human, cells may be rendered defective, or alternatively it may be enhanced, with the insertion of an OBG3 genomic or cDNA sequence with the replacement of the OBG3 gene counterpart in the genome of an animal cell by an OBG3 polynucleotide. These genetic alterations may be generated by homologous recombination events using specific DNA constructs that have been previously described.

One kind of host cell that may be used are mammalian zygotes, such as murine zygotes. For example, murine zygotes may undergo microinjection with a purified DNA molecule of interest, for example a purified DNA molecule that has previously been adjusted to a concentration range from 1 ng/ml -for BAC inserts- 3 ng/µl -for P1 bacteriophage inserts- in 10 mM Tris-HCl, pH 7.4, 250 µM EDTA containing 100 mM NaCl, 30 µM spermine, and 70 µM spermidine. When the DNA to be microinjected has a large size, polyamines and high salt concentrations can be used in order to avoid mechanical breakage of this DNA, as described by Schedl et al ((1993) Nature Mar 18;362(6417):258-61).

Any one of the polynucleotides described herein, including the DNA constructs described herein, may be introduced in an embryonic stem (ES) cell line, preferably a mouse ES cell line. ES cell lines are derived from pluripotent, uncommitted cells of the inner cell mass of pre-implantation blastocysts. Preferred ES cell lines are the following: ES-E14TG2a (ATCC No.CRL-1821), ES-D3 (ATCC No.CRL1934 and No. CRL-11632), YS001 (ATCC No. CRL-11776), 36.5 (ATCC No. CRL-11116). To maintain ES cells in an uncommitted state, they are cultured in the presence of growth inhibited feeder cells which provide the appropriate signals to preserve this embryonic phenotype and serve as a matrix for ES cell adherence. Preferred feeder cells are primary embryonic fibroblasts that are established from tissue of day 13- day 14 embryos of virtually any mouse strain, that are maintained in culture, such as described by Abbondanzo et al. (1993; Methods Enzymol;225:803-23) and are inhibited in growth by irradiation, such as described by Robertson ((1987) Embryo-derived stem cell lines. In: EJ. Robertson Ed. Tesatocarcinomas and embrionic stem cells: a practical approach. IRL Press, Oxford), or by the presence of an inhibitory concentration of LIF, such as described by Pease and Williams (1990; Exp Cell Res. Oct;190(2):209-11).

The constructs in the host cells can be used in a conventional manner to produce the gene product encoded by the recombinant sequence.

Following transformation of a suitable host and growth of the host to an appropriate cell density, the selected promoter is induced by appropriate means, such as temperature shift or chemical induction, and cells are cultivated for an additional period. Cells are typically harvested by centrifugation, disrupted by physical or chemical means, and the resulting crude extract retained for further purification. Microbial cells employed in the expression of proteins can be disrupted by any convenient method, including freeze-thaw cycling, sonication, mechanical disruption, or use of cell lysing agents. Such methods are well known by the skilled artisan.

### IV. Transgenic animals

Methods and compositions for the generation of non-human animals and plants that express recombinant OBG3 polypeptides, i.e. recombinant OBG3 fragments or fill-lengh OBG3 polypeptide are described herein. The animals or plants can be transgenic, i.e. each of their cells contains a gene encoding the OBG3 polypeptide, or, alternatively, a polynucleotide encoding the polypeptide can be introduced into somatic cells of the animal or plant, e.g. into mammary secretory epithelial cells of a mammal. The non-human animal may be a mammal such as a cow, sheep, goat, pig, or rabbit.

Methods of making transgenic animals such as mammals are well known to those of skill in the art, and any such method can be used. Briefly, transgenic mammals can be produced, e.g., by transfecting a pluripotential stem cell such as an ES cell with a polynucleotide encoding a polypeptide of interest. Successfully transformed ES cells can then be introduced into an early stage embryo which is then implanted into the uterus of a mammal of the same species. In certain cases, the transformed ("transgenic") cells will comprise part of the germ line of the resulting animal, and adult animals comprising the transgenic cells in the germ line can then be mated to other animals, thereby eventually producing a population of transgenic animals that have the transgene in each of their, cells, and which can stably transmit the transgene to each of their offspring. Other methods of introducing the polynucleotide can be used, for example introducing the polynucleotide encoding the polypeptide of interest into a fertilized egg or early stage embryo via microinjection. Alternatively, the transgene may be introduced into animal by infection of zygotes with a retrovirus containing the transgene (Jaenisch, R. (1976) Proc. Natl. Acad. Sci. USA 73, 1260-1264). Methods of making transgenic mammals are described, e.g., in Wall et (1922) J Cell Biochem 1992 Jun;49(2):113-20, Hogan, et al. (1986) in Manipulating the mouse embryo. A Laboratory Manual. Cold Spring Harbor Laboratory Press, Cold Spring Harbor, N.Y.; in WO 91/08216, or in U.S. Patent No. 4,736,866.

In a preferred method, the polynucleotides are microinjected into the fertilized oocyte. Typically, fertilized oocytes are microinjected using standard techniques, and then cultured in vitro until a "pre-implantation embryo" is obtained. Such pre-implantation embryos preferably contain approximately 16 to 150 cells. Methods for culturing fertilized oocytes to the pre-implantation stage are described, e.g., by Gordon et al. ((1984) Methods in Enzymology, 101, 414); Hogan et al. ((1986) in Manipulating the mouse embryo. A Laboratory Manual. Cold Spring Harbor Laboratory Press, Cold Spring Harbor, N.Y) (for the mouse embryo); Hammer et al. ((1985) Nature, 315,680) (for rabbit and porcine embryos); Gandolfi et al. ((1987) J. Reprod. Fert. 81,23-28); Rexroad at al. ((1988) J. Anim. Sci. 66,947-953) (for ovine embryos); and Eyestone et al. ((1989) J. Reprod. Fert. 85,713-720); Camous et al. ((1984) J. Reprod. Fert. 72,779-785); and Heyman et al. ((1987) Theriogenology 27, 5968) (for bovine embryos). Pre-implantation embryos are then transferred to an appropriate female by standard methods to permit the birth of a transgenic or chimeric animal, depending upon the stage of development when the transgene is introduced.

As the frequency of trangene incorporation is often low, the detection of transgene integration in pre-implantation embryos is often desirable using any of the hetem-descnbed methods. Any of a member of methods can be used to detect the presence of a transgene in a pre-implantation embryo. For example, one or more cells may be removed from the pre-implantation embryo, and the presence or absence of the transgene in the removed cell or cells can be detected using any standard method e.g. PCR. Alternatively, the presence of a transgene can be detected in utero or post partum using standard methods.

Transgenic mammals may be generated that secrete recombinant OBG3 polypeptides in their milk As the mammary gland is a highly efficient protein-producing organ, such methods can be used to produce protein concentrations in the gram per liter range, and often significantly more. Preferably, expression in the mammary gland is accomplished by operably linking The polynucleotide encoding the OBG3 polypeptide to a mammary gland specific promoter and, optionally, other regulatory elements. Suitable promoters and other elements include, but are not limited to, those derived from mammalian short and long WAP, alpha, beta, and kappa, casein, alpha and beta lactoglobulin, beta-CN 5' genes, as well as the the mouse mammary tumor virus (MMTV) promoter. Such promoters aud other elements may be derived from any mammal, including, but not limited to, cows, goats, sheep, pigs, mice, rabbits, and guinea pigs. Promoter and other regulatory sequences, vectors, and other relevant teachings are provided, e.g., by Clark (1998) J Mammary Gland Biol Neoplasia 3:337-50; Jost et al. (1999) Nat Biotechnol 17:160-4; U.S. Patent Nos. 5,994.616; 6,140,552; 6,013,957; Sohn et al. (1999) DNA Cell Biol. 18:845-52; Kim et al. (1999) J. Biochem. (Japan) 126:320-5; Soulier et al. (1999) Euro. J. Biochem. 260:533-9; Zhang et al. (1997) Chin. J. Biotech. 13:271-6; Rijnkels et al. (1998) Transgen. Res. 7:5-14; Korhonen et al. (1997) Euro. J. Biochem. 245:482-9; Uusi-Oukari et al. (1997) Transgen. Res. 6:75-84; Hitchin et al. (1996) Prot. Expr. Purif. 7:247-52; Platenburg et al. (1994) Transgen. Res. 3:99-108; Heng-Cherl et al. (1993) Animal Biotech. 4:89-107, and Christa et al. (2000) Buro. J. Biochem. 267:1665-71.;

The polypeptides described herein can be produced in milk by introducing polynucleotides encoding the polypeptides into somatic cells of the mammary gland in vivo, e.g. mammary secreting epithelial cells. For example, plasmid DNA can be infused through the nipple canal, e.g. in association wit DEAE-dextran (see, e.g., Hens et al. (2000) Biochim. Biophys. Acta 1523:161-171), in association with a ligand that can lead to receptor-mediated endocytosis of the construct (see, e.g., Sobolev et al, (1998) 273:7928-33), or in a vital vector such as a retroviral vector, e.g. the Gibbon ape leukemia virus (see, e.g., Archer et aL.(1994) PNAS 91:6840-6844). In any of these embodiments, the polynucleotide may be operably linked to a mammary gland specific promoter, as described above, or, alternatively, any strongly expressing promoter such as CMV or MoMLV LTR.

The suitability of any vector, promoter, regulatory element, etc. for use as described herein can be assessed beforehand by transfecting cells such as mammary epithelial cells, e.g. MacT cells (bovine mammary epithelial cells) or GMB cells (goat mammary epithelial cells), in vitro and assessing the efficiency of transfection and expression of the transgene in the cells.

For in vivo administration, the polynucleotides can be administered in any suitable formulation, at any of a range of concentrations (e.g. 1-500 µg/ml, preferably 50-100 µg/ml), at any volume (e.g. 1-100 ml, preferably 1 to 20 ml), and can be administered any number of times (e.g. 1, 2, 3, 5, or 10 times), at any frequency (e.g. every 1. 2, 3, 5, 10, or any number of days). Suitable concentrations, frequencies, modes of administration, etc. will depend upon the particular polynucleotide, vector, animal, etc., and can readily be determined by one of skill in the art.

A retroviral vector such as as Gibbon ape leukemia viral vector may be used, as described in Archer et al. ((1994) PNAS 91:6840-6844). As retroviral infection typically requires cell division, cell division in the mammary glands can be stimulated in conjunction with the administration of the vector, e.g. using a factor such as estrodiol benzoate, progesterone, reserpine, or dexamethasone. Further, retroviral and other methods of infection can be facilitated using accessory compounds such as polybrene.

In any of the herein-described methods for obtaining OBG3 polypeptides from milk, the quantity of milk obtained, and thus the quantity of OBG3 polypeptides produced, can be enhanced using any standard method of lacation induction, e.g. using hexestrol, estrogen, and/or progesterone.

The polynucleotides used can either encode a full-length OBG3 polypeptide or an OBG3 fragment. Typically, the encoded polypeptide will include a signal sequence to ensure the secretion of the protein into the milk. Where a full length OBG3 sequence is used, the full length protein can, e.g., be isolated from milk and cleaved in vitro using a suitable protease. Alternatively, a second, protease-eacoding polynucleotide can be introduced into the animal or into the mammary gland cells, whereby expression of the protease results in the cleavage of the OBG3 polypeptide in vivo, thereby allowing the direct isolation of OBG3 fragments from milk.

### V. Pharmaceutical or Physiologically AcceptablE Compositions

The gOBG3 polypeptide fragments described herein can be administered to non-human animals and/or humans, alone or in pharmaceutical or physiologically acceptable compositions where they are mixed with suitable carriers or excipient(s). The pharmaceutical or physiologically acceptable composition may then be provided at a therapeutically effective dose. A therapeutically effective dose refers to that amount of gOBG3 fragment sufficient to result in prevention or amelioration of symptoms or physiological status of obesity-related discases or disorders as determined by the methods described herein. A therapeutically effective dose can also refer to the amount of OBG3 or gOBG3 fragment necessary for a reduction in weight or a prevention of an increase in weight or prevention of an increase in the rate of weight gain in persons desiring this affect for cosmetic reasons. A therapeutically effective dosage of gGBG3 fragment described herein is that dosage that is adequate to promote weight loss or weight gain with continued periodic use or administration. Techniques for formulation and administration of OBG3 polypeptide fragments may be found in "Remington's Pharmaceutical Scieaces," Mack Publishing Co., Easton, PA, latest edition.

Other diseases or disorders that gOBG3 polypeptide fragments could be used to treat or prevent include, but are not limited to, obesity and obesity-related diseases and disorders such as obesity, insulin resistance, atherosclerosis, atheromatous disease, heart disease, heart disease, hypertension, stroke, Syndrome X, non-insulin-dependent diabetes and Type II diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders to be treated by compounds described herein include hyperlipidemia and hyperuricemia. Yet other obesity-related diseases or disorders of the invention include cachexia, wasting, AIDS-related weight loss, anorexia, and bulimia. The gOBG3 polypeptide fragments may also be used to enhance physical performance during work or exercise or enhance a feeling of general well-being. Physical performance activities include walking, running, jumping, lifting and/or climbing.

The OBG3 or gOBG3 polypeptide fragments or antagonists thereof may also be used to treat dyslexia, attention-deficit disorder (ADD), attention-deficit/hyperactivity disorder (ADHD), and psychiatric disorders such as schizophrenia by modulating fatty acid metabolism, more specifically, the production of certain long-chain polyunsaturated fatty acids.

It is expressly considered that the gOBG3 polypeptide fragments described herein may be provided alone or in combination with other pharmaceutically or physiologically acceptable compounds. Other compounds useful for the treatment of obesity and other diseases and disorders are currently well-known in the art.

In a preferred embodiment, the gOBG3 polypeptide fragments are useful for, and used in, the treatment of insulin resistance and diabetes using methods described herein and known in the art. More particularly, a process for the therapeutic modification and regulation of glucose metabolism in an animal or human subject may comprises administering to a subject in need of treatment (alternatively on a timed daily basis) gOBG3 polypeptide fragment (or polynucleotide encoding said polypeptide) in dosage amount and for a period sufficient to reduce plasma glucose levels in said animal or human subject.

Further methods for the prophylaxis or treatment of diabetes may comprise administering to a subject in need of treatment (alternatively on a timed daily basis) OBG3 polypeptide fragment (or polynucleotide encoding said polypeptide) in dosage amount and for a period sufficient to reduce plasma glucose levels in said animal or human subject.

### Routes of Administration

Suitable routes of administration include oral, nasal, rectal, transmucosal, or intestinal administration, parenteral delivery, including intramuscular, subcutaneous, intramedullary injections, as well as intrathecal, direct intraventricular, intravenous, intraperitoneal, intranasal, intrapulmonary (inhaled) or intraocular injections using methods known in the art. A particularly useful method of administering compounds for promoting weight loss involves surgical implantation for example into the abdominal cavity of the recipient, of a device for delivering OBG3 or gOBG3 polypeptide fragments over an extended period of time. Other particularly preferred routes of administration are aerosol and depot formulation. Sustained release formulations, particularly depot, of the medicaments described herein are expressly contemplated.

### Composition/Formulation

Pharmaceutical or physiologically acceptable compositions and medicaments may be formulated in a conventional manner using one or more physiologically acceptable carriers comprising excipients and auxiliaries. Proper formulation is dependent upon the route of administration chosen.

Certain of the medicaments described herein will include a pharmaceutically or physiologically acceptable acceptable carrier and at least one polypeptide that is a gOBG3 polypeptide fragment as described herein. For injection the agents described herein may be formulated in aqueous polypeptide solutions, preferably in physiologically compatible buffers such as Hanks's solution, Ringer's solution, or physiological saline buffer such as a phosphate or bicarbonate buffer. For transmucosal administration, penetrants appropriate to the barrier to be permeated are used in the formulation. Such penetrants are generally known in the art.

Pharmaceutical or physiologically acceptable preparations that can be taken orally include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as tale or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable gaseous propellant, e.g., carbon dioxide. In the case of a pressurized aerosol the dosage unit may be determined by providing a valve to deliver a metered amount. Capsules and cartridges of, e.g., gelatin, for use in an inhaler or insufflator, may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in aqueous vehicles, and may contain formulatory agents such as suspending, stabilizing and/or dispersing agents.

Pharmaceutical or physiologically acceptable formulations for parenteral administration include aqueous solutions of the active compounds in water-soluble form. Aqueous suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Alternatively, the active ingredient may be in powder or lyophilized form for constitution with a suitable vehicle, such as sterile pyrogen-free water, before use.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Additionally, the compounds may be delivered using a sustained-release system, such as semipermeable matrices of solid hydrophobic polymers containing the therapeutic agent. Various sustained release materials have been established and are well known by those skilled in the art. Sustained-release capsules may, depending on their chemical nature, release the compounds for a few weeks up to over 100 days.

Depending on the chemical nature and the biological stability of the therapeutic reagent, additional strategies for protein stabilization may be employed.

The pharmaceutical or physiologically acceptable compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

### Effective Dosage.

Pharmaceutical or physiologically acceptable compositions may include compositions wherein the active ingredients are contained in an effective amount to achieve their intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used as described herein, the therapeutically effective dose can be estimated initially from cell culture assays. For example, a dose can be formulated in animal models to achieve a circulating concentration range that includes or encompasses a concentration point or range shown to increase leptin or lipoprotein uptake or binding in an *in vitro* system. Such information can be used to more accurately determine useful doses in humans.

A therapeutically effective dose refers to that amount of the compound that results in amelioration of symptoms in a patient. Toxicity and therapeutic efficacy of such compounds can be determined by standard pharmaceutical procedures in cell cultures or experimental animals, *e.g.,* for for determining the LD50, (the dose lethal to 50% of the test population) and the ED50 (the dose therapeutically effective in 50% of the population). The dose ratio between toxic and therapeutic effects is the therapeutic index and it can be expressed as the ratio between LD50 and ED50. Compounds that exhibit high therapeutic indices are preferred.

The data obtained from these cell culture assays and animal studies can be used in formulating a range of dosage for use in humans. The dosage of such compounds lies preferably within a range of circulating concentrations that include the ED50, with little or no toxicity. The dosage may vary within this range depending upon the dosage form employed and the route of administration utilized. The exact formulation, route of administration and dosage can be chosen by the individual physician in view of the patient's condition. (*See, e.g.,* Fingl et al, 1975, in "The Pharmacological Basis of Therapeutics", Ch. 1).

Dosage amount and interval may be adjusted individually to provide plasma levels of the active compound which are sufficient to maintain or prevent weight loss or gain, depending on the particular situation. Dosages necessary to achieve these effects will depend on individual characteristics and route of administration.

Dosage intervals can also be determined using the value for the minimum effective concentration. Compounds should be administered using a regimen that maintains plasma levels above the minimum effective concentration for 10-90% of the time, preferably between 30-90%, and most preferably between 50-90%. In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

A preferred dosage range for the amount of a gOBG3 polypeptide fragment described herein which can be administered on a daily or regular basis to achieve desired results, including a reduction in levels of circulating plasma triglyceride-rich lipoproteins, range from 0.01 - 0.5 mg/kg body mass. A more preferred dosage range is from 0.05-0.1 mg/kg. Of course, these daily dosages can be delivered or administered in small amounts periodically during the course of a day. It is noted that these dosage ranges are only preferred ranges and are not meant to be limiting to the invention.

### VL Methods of Treatment

Treatment of mice with gOBG3 polypeptide fragments results in decreased triglyceride levels, decreased free fatty acid levels, decreased glucose levels, and decreased body weight as well as increased muscle oxidation.

Methods of preventing or treating obesity-related diseases and discorders may comprise providing an individual in need of such treatment with a gOBG3 polypeptide fragment as described herein. Preferably, the OBG3 polypeptide fragment has obesity-related activity either *in vitro* or *in vivo.* Preferably the OBG3 polypeptide fragment is provided to the individual in a pharmaceutical composition that is preferably taken orally. Preferably the individual is a mammal, and most preferably a human. In preferred embodiments, the obesity-related disease or disorder is selected from the group consisting of atherosclerosis, cardiovascular disease, insulin resistance, hypertension, stroke, Syndrome X, Type II diabetes and lipoatrophic diabetes. Type II diabetes-related complications to be treated by the methods of the invention include microangiopathic lesions, ocular lesions, and renal lesions. Heart disease includes, but is not limited to, cardiac insufficiency, coronary insufficiency, and high blood pressure. Other obesity-related disorders to be treated by compounds described herein include hyperlipidemia, hypertriglyceridemia, and hyperuricemia. Yet other obesity-related diseases or disorders of the invention include cachexia, wasting, AIDS-related weight loss, neoplasia-related weight loss, anorexia, and bulimia. In highly preferred embodiments gOBG3 polypeptide fragments in pharmaceutical compositions are used to modulate body weight in healthy individuals for cosmetic reasons.

Methods of preventing or treating obesity-related diseases and disorders may comprise providing an individual in need of such treatment with a compound identified by assays described in Section VI of the Preferred Embodiments and in the Examples. Preferably these compounds antagonize or agonize effects OBG3 or gOBG3 polypeptide fragments in cells *in vitro,* muscles *ex vivo,* or in animal models. Alternatively, these compounds agonize or antagonize the effects of OBG3 or gOBG3 polypeptide fragments on leptin and/or lipoprotein uptake and/or binding. Optionally, these compounds prevent the interaction, binding, or uptake of OBG3 or gOBG3 polypeptide fragments with LSR *in* vitro or *in vivo.* Preferably, the compound is provided to the individual in a pharmaceutical composition that is preferably taken orally. Preferably the individual is a mammal, and most preferably a human. In preferred embodiments, the obesity-related disease or disorder is selected from the group consisting of obesity and obesity-related diseases and disorders such as atherosclerosis, heat disease, insulin resistance, hypertension, stroke, Syndrome X, Type II diabetes, and lipoatrophic diabetes. Type II diabetes-related complications to be treated by the methods described herein may include microangiopathic lesions, ocular lesions, and renal lesions. Heart disease includes, but is not limited to, cardiac insufficiency, coronary insufficiency, and high blood pressure. Other obesity-related disorders to be treated by compounds described herein may include hyperlipidemia, hypertriglyceridemia, and hyperuricemia. Yet other obesity-related diseases or disorders of the invention include cachexia, wasting, AIDS-related weight loss, neoplasis-related weight loss, anorexia, and bulimia. The pharmaceutical compositions may be used to modulate body weight for cosmetic reasons.

Treatment may be with gOBG3 polypeptide fragments where an individual is shown to have a particular genotype for an Apml marker (Apml designates the human homolog of the full-length OBG3 polypeptide), or where they have been shown to have a reduced amount of plasma Apml, either full-length or preferably a more biologically active fragment of Apm1, as compared to control values, e.g. values representative of non-diseased individuals, or as compared to that individual prior to the onset of a disease or condition. In either case, treatment would comprise providing pharmaceutically acceptable gOBG3 polypeptide fragments to the individual. The exact amount of gOBG3 fragment provided would be determined through clinical trials under the guidance of qualified physicians, but would be expected to be in the range of 5-7 mg per individual per day. In general, a preferred range would be from 0.5 to 14 mg per individual per day, with a highly preferred range being between 1 and 10 mg per individual per day. Individuals who could benefit from treatment with gOBG3 polypeptide fragments could be identified through at least two methods: plasma serum level determinations and genotyping.

### OBG3/APM1 levels

Preliminary studies have shown that obese people have lower levels of full-length OBG3/Apm than non-obese people. Treatment of individuals (preferably obese) that have low levels of full-length OBG3/Apm1 with gOBG3 polypeptide fragments described herein is contemplated. In addition, of individuals with low levels of the biologically active fragment of OBG3/Apm1 with gOBG3 polypeptide is contemplated gOBG3 polypeptide fragments described herein may be administered to individuals, preferably obese individuals, that have levels of full-length OBG3 (or alternatively a mature OBG3 polypeptide fragment) at least 10% 20%, 30%, 40%, 50%, 60%, 70%, 80%, 90%, about 100% or 100% lower than non-obese individuals, preferably healthy individuals as determined by a physician using normal standards in the art. Methods to determine and compare the levels of full-length OBG3 in individuals are well-known in the art and include, but are not limited to using an antibody specific for Apml in a format such as a Radio Immune Assay, ELISA, Western blot, dotblot, or as part of an array, for example. Methods of generating antibodies to Apml and fragments thereof as well as to proteins with SNPs are discussed in PCT/IB99/01858, US application No. 09/434,848, and WO 99/07736. Further, antibodies specific for gOBG3 polypeptide fragments described herein, their generation, and their use are described herein.

Other characteristics and advantages of the invention are described in the Brief Description of the Figures and the Examples. These are meant to be exemplary only, and not to limit the invention in any way.

### EXAMPLES

The following Examples are provided for illustrative purposes and not as a means of limitation. One of ordinary skill in the art would be able to design equivalent assays and methods based on the disclosure herein all of which form part of the instant invention.

It should be noted that the term full-length OBG3 polypeptide used throughout the specification is intended to encompass the protein homologs ACRP30 (Scherer et al (1995) J Biol. Chem. 270:26746-9), AdipoQ (Hu et al (1996) J Biol Chem 271:10697-10703) and the human homolog Apm-1 (Maeda et al (1996) Biochem Biophys Res Commun 221:289-9) or GBP28 (Nakano et al (1996) J Biochem (Tokyo) 120:803-812). OBG3 is also intended to encompass other homologs.

### EXAMPLE 1: Production of Recombinant OBG3

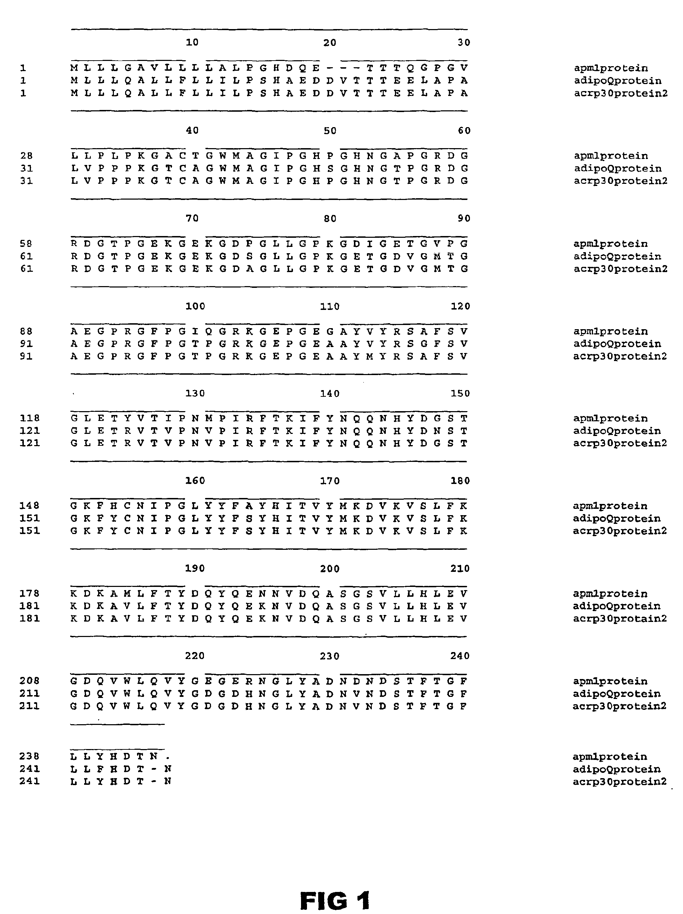

An exemplary method for generating recombinant OBG3 is given below. Although the method describes the production of the mouse analog, a person with skill in tho art would be able to use the information provided to produce other OBG3 analogs, including but not limited to the human analog. An alignment of the amino acid sequences of the human (apml) and mouse (AdipoQ and acrp30) OBG3 is shown in Fig. 1.

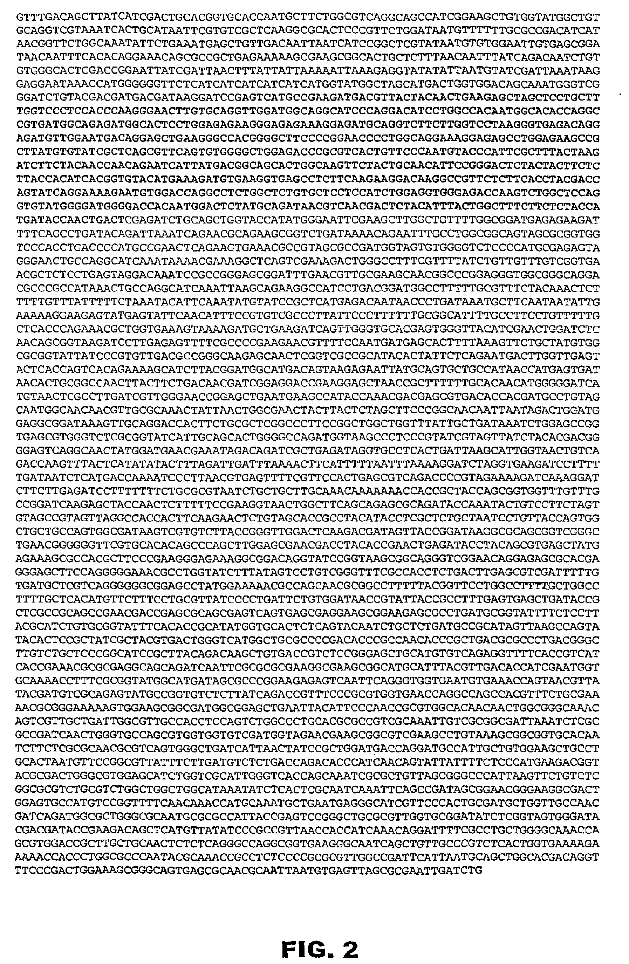

The recombinant OBG3 analog is cloned in pTRC His B (Invitrogen) between BamH1 and Xhol (Fig. 2) and maintained in E. *coli* DH5-alpha. The sequence of the OBG3 insert corresponds to ACRP 30 genbank U37222 bases 88 to 791 except in position 382 where in #3 G replaces A found in ACRP 30 (V instead of M). The corresponding nucleotide in AdipoQ U49915 is G as in clone #3. The amino acid V is also conserved in the human sequence APM-1 D45371.

### Culture:

Plate out bacteria in LB agar media containing 100 µg/mL ampicillin. Inoculate 1 colony into 5 mL media (no agar) at 37 °C overnight. Add 2 mL of this initial culture into 500 mL Erlenmeyer flasks containing 200 mL LB media and 100 µg/mL ampicillin. Incubate at 37 °C in an orbital shaker until the OD₆₀₀ = 0.2. Add IPTG to a final concentration of 1 mM (stock solution = 1 M). Incubate at 37 °C overnight.

### Lysis:

Pellet the bacteria by centrifugation (Sorvall, 3500 rpm, 15 min, 4°C) in a pre-weighed tube.
At 4°C resuspend the pellet in 3 mL/g of lysis buffer
Add 40 µL/g PMSF 10 mM
Add 80 µL/g of lysozyme 10 mg/mL
Incubate 20 min on ice, shaking intermittently
Add 30 µL/g 10% sodium deoxycholate
Incubate at 37°C until the lysate is viscous
Freeze in liquid Nitrogen and thaw at 37°C three times
Sonicate 2X, 30 sec, 25% cycle, 2.5 power level
Centrifuge 30 min, 15000 rpm, 4°C
Recover the supernatant
Note: The lysate can be stored frozen before or after the sonication step.

### Batch Purification:

1. Pack 1 mL of Probond resin (Invitrogen; 1 mlL= 2 mL suspended gel) into a 5 mL column. Wash with 5 mL PBS.
2. Apply 5 mL bacterial supernatant to the 1 mL of gel. (If volume is very high, use several small columns.)
3. Wash with 24 mL phosphate buffer, pH 7.8, followed by a wash with 24 mL phosphate buffer, pH 6.
4. Elute with imidazole buffer and collect fractions of 1 mL.
5. Analyze fractions by OD at 280 nm or by SDS-PAGE (12.5%; dilution ½ in 2X sample buffer) under reducing conditions (100°C, 5 min)
6. Pool the fractions containing protein (usually fraction numbers 2-4 for concentrations of 0.8 - 1 mg/mL and fractions 1, 5 and 6 for concentrations of 0.2 - 0.4 mg/mL).
7. Dialyze thoroughly against 1 X PBS, 24 mM ammonium bicarbonate or 50 mM Tris, pH 7.4 containing 250 nM NaCl. Concentrate by Speed-Vac if needed.
8. Analyze protein by the Lowry method.
9. Aliquot and store at -20°C.

### Purification On Liquid Chromatography System

1. Pack 5 mL of Probond resin into a 5 mL column.
2. Wash with 4 bed volumes of phosphate buffer pH 7.8, 1 mL/min.
3. Inject 25 mL lysate (filtered on 0.45 µ or centrifuged at 3000 rpm, 30 min, 4°C, Beckman Allegra 6R) at 0.5 mL/min.
4. Wash with 4 bed volumes of phosphate buffer, pH 7.8 at 1 mL/min.
5. Wash with 12 bed volumes of phosphate buffer pH 5.5 at 1 mL/min.
6. Elute bound fraction with phosphate buffer, pH 5.5, containing 1 M imidazole at 1 mL/min.
7. Collect fractions, dialyze and analyze protein as described for batch purification, steps 7-9.

### EXAMPLE 2: Generation of Globular OBG3 by Enzymatic Cleavage

Incubate purified OBG3 (obtained as described above or through equivalent method) with acetylated Trypsin-Type V-S from Bovine Pancreas (Sigma E.C. = 3.4.21.4 ) at 400 u/mg protein at 25°C for 10 min.

Stop reaction by running the sample over a Poly-Prep Column (Biorad 731-1550) at + 4°C containing immobilized Trypsin inhibitor.

Collect 1.0 mL fractions. Determine protein concentration.

Pool the protein containing fractions and dialyze extensively against PBS using dialysis tubing with M.W. cutoff= 10,000 da.

Concentrate on Amicon YM-10 Centricon Filter (Millipore, M.W. cutoff = 10,000 da). Sterile filter.

Determine final protein concentration using Markwell's modified Lowry procedure (1981) or BCA protein assay (Pierce Chemical Co, Rockford, IL) and BSA as standard.

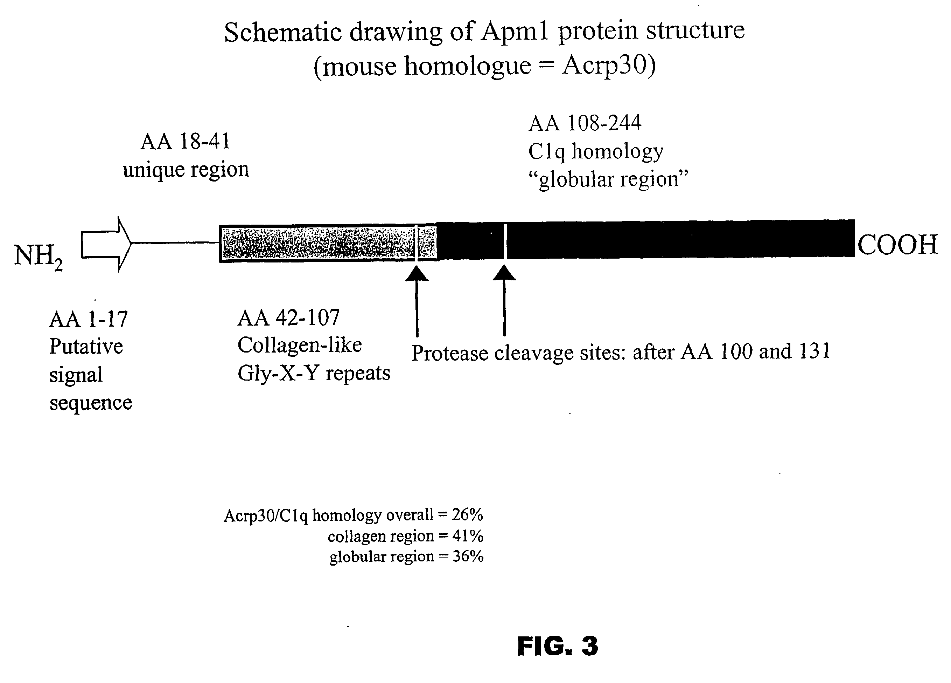

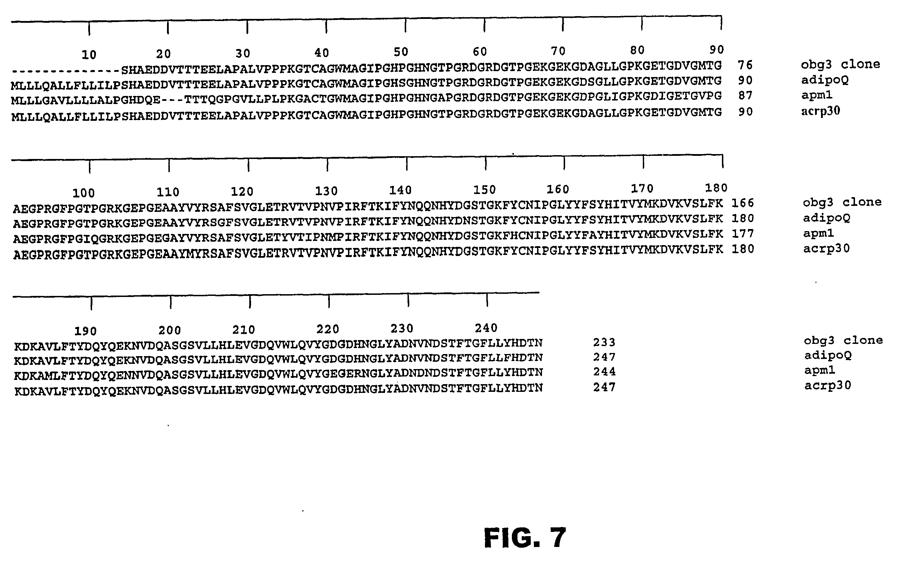

Check purity and efficiency of cleavage by SDS - PAGE analysis using a 4-20% gradient gel. The intact OBG3 migrates as a single band at approximately 37 kda apparently due to co-transcribed vector sequences attached to the histidine tag at the N-terminus of AdipoQ, and forms a dimer at 74 kDa. The cleaved OBG3 forms a band at approx. 18 kda (gOBG3). Additional degradation products, all smaller than 10 kda are also generated from the N-terminal region. These are separated from the desired 18 kda band by dialysis with semipermeable membranes with a MW cutoff of 10,000. The two potential cleavage sites for gOBG3 are shown in Fig. 3. The actual cleavage site has been identified as the one after amino acid 103 (amino acid 100 for human gOBG3 or APM1) (Fig. 7). That is, the N-terminus of the gOBG3 cleavage product is Lys 104 (Lys 101 for human gOBG3 or APM1).

Other enzymatic/proteolytic methods can also be used that yield similar products, e.g. clostripain. Other preferred enzymes would preferably cleave OBG3 at a site close to the junction between the collagen-like tail and the globular head (about amino acid 108 for human gOBG3 and about amino acid 111 for murine gOBG3), preferably permit the reaction to be easily stopped, preferably be easily removed using an immobilized inhibitor, or similar method, and preferably cuts the N-terminal fragment into small pieces (less than 10,000 MW). The cleavage preferably results in the presence of no more than 6 collagen repeats, more preferably 3 collagen repeats, and most preferably no collagen repeats. A collagen repeat consists of GLY-X-Y. A determination of whether an active gOBG3 has been generated can be checked using *the in vitro* and *in vivo* assays described herein (Examples 4-6, 8-10).

### EXAMPLE 3: Generation of gOBG3 by Recombinant Methodology

### Restriction Site Cloning

A first approach is to look for unique restriction sites near the beginning of the globular head region (nucleic acid sequences of mouse and human OBG3 polypeptides are provided in the sequence listing). If present, it can be used to cleave the 5' collagen-like region from the globular head region. If a unique site is not present, it is also possible, although more difficult, to do this using restriction enzymes that cut in more than one location by doing partial digestions. The 3' end of the globular head can be cut from its vector backbone using an appropriate enzyme. The globular head can then be cloned into an expression vector and constructs containing the correct fragments can be identified. For AdipoQ, Tau I seems to be a unique enzyme that would separate the collagen tail from the globular head.

### PCR Cloning

Another approach is to PCR the region of interest from the intact sequence (if cDNA is available) using primers with restriction sites on the end so that PCR products can be directly cloned into vectors of interest. Alternatively, gOBG3 can also be generated using RT-PCR to isolate it from adipose tissue RNA.

### E. coli Vector

For example, the AdipoQ globular region can be cloned into pTrcHisB, by putting a Bam HI site on the sense oligo and a Xho I site on the antisense oligo. This allows isolation of the PCR product, digestion of that product, and ligation into the pTrcHisB vector that has also been digested with Bam HI and Xho I (Fig. 4). The vector, pTrcHisB, has an N-terminal 6-Histidine tag, that allows purification of the over expressed protein from the lysate using a Nickel resin column. The pTrcHisB vector is used for over-expression of proteins in *E. coli.*

Exemplary oligos for cloning into the *E. coli* vector include:
A) obg3 sense CTTAGTGGATCCCGCTTATGTGTATCGCTCAG 6 base pairs from the left there is a 6 bp BamHI site. Thus the region that is homologous to the gene begins at nucleotide 13.
B) obg3 antisense GCTGTTCTCGAGTCAGTTGGTATCATGG 6 base pairs from the left there is a 6 bp. XhoI site. Thus the region that is homologous to the gene begins at nucleotide 13.

The following are exemplary PCR conditions.

Final concentrations in the reaction are:
1X PE Biosystems buffer A
1.5mM MgCl₂
200µM of each dNTP (dATP, dCTP, dGTP, dTTP)
2.5 Units of Amplitaq Gold from PE Biosystems
0.4µM of each primer (sense and antisense)
10 ng of plasmid template

Cycling parameters:
95°C 10min --- 1 cycle
95°C 30sec
56°C 30sec
72°C 30sec
repeat above 3 steps for 30 cycles
72°C 7min --- 1 cycle.

### BAC Vector

The globular head can also be over expressed in a Baculovirus system using the 6xHis Baculovirus kit (Pharmingen), for example. The AdipoQ globular region is cloned into the appropriate vector using enzymes available in the multiple cloning site. This allows over-expression of the protein in a eukaryotic system which has some advantages over the *E. coli* system, including:
Multiple gene expression, Signal peptide cleavage, Intron splicing, Nuclear transport, Functional protein, Phosphorylation, Glycosylation, and Acylation.
Exemplary oligos for cloning into the Baculovirus vector are the following:
   A). obg3 sense CTTAGTGAATTCGCTTATGTGTATCGCTCAGA 6 base pairs from the left there is a 6 bp. EcoRI site. Thus the region that is homologous to the gene begins at nucleotide 13.
   B). obg3 antisense GCTGTTCTGCAGTCAGTTGGTATCATGG 6 base pairs from the left there is a 6 bp. PstI site. Thus the region that is homologous to the gene begins at nucleotide 13.

The following are exemplary PCR conditions.

Final concentrations in the reaction are:
1X PE Biosystems buffer A
1,5mM MgCl₂
200µM of each dNTP (dATP, dCTP, dGTP, dTTP)
2.5 Units of Amplitaq Gold from PE Biosystems
0.4µM of each primer (sense and antisense)
10 ng of plasmid template

Cycling parameters:
95°C 10min --- 1 cycle
95°C 30sec
60°C 30sec
72°C 30sec
repeat above 3 steps for 30 cycles
72°C 7min --- 1 cycle.

### Mammalian Vector

Globular OBG3 can also be cloned into a mammalian expression vector and expressed in and purified from mammalian cells, for example 3T3-L1 cells (undifferentiated adipocyte precursors). The globular head is then generated in an environment very close to its endogenous environment. However, this is not necessarily the most efficient way to make protein.

### EXAWLE 4: In Vitro Tests of Obesity-related Activity

The activity of various preparations and various sequence variants of gOBG3 polypeptide fragments are assessed using various *in vitro* assays including those provided below. These assays are also exemplary of those that can be used to develop gOBG3 polypeptide fragment antagonists and agonists. To do that, the effect of gOBG3 polypeptide fragments in the above assays; *e.g.* on leptin and/or LSR activity, in the presence of the candidate molecules would be compared with the effect of gOBG3 polypeptide fragments in the assays in the absence of the candidate molecules. Since gOBG3 polypeptide fragments have been shown to reduce body weight in mice on a high-cafeteria diet (Example 5), these assays also serve to identify candidate treatments for reducing (or increasing) body weight.

### Liver Cell Line:

Tests of efficacy of gOBG3 polypeptide fragments on LSR can be performed using liver cell lines, including for example, PLC, HepG2, Hep3B (human), Hepa 1-6, BPRCL (mouse), or MCA-RH777, MCA-RH8994 (rat). For human cell lines, APM1 and globular APM1 would be used preferentially; for rodents, full-length and globular AdipoQ/ACRP30 would be used preferentially.

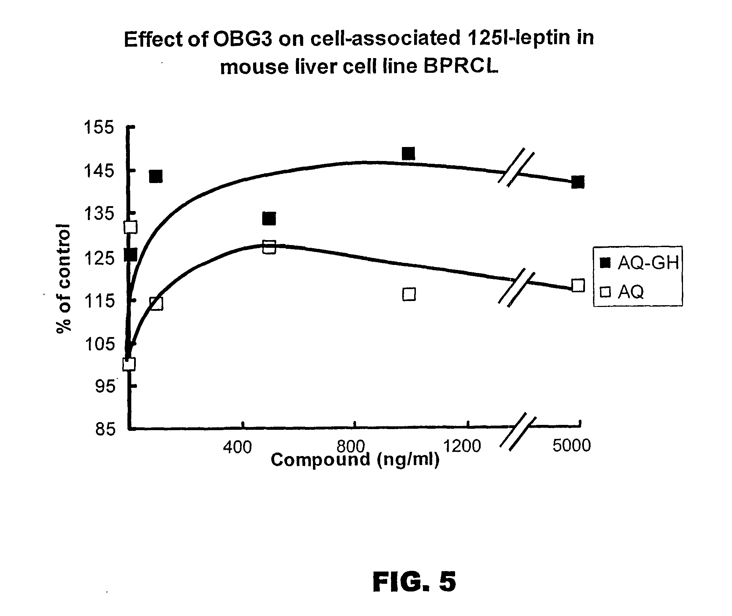

BPRCL mouse liver cells (ATCC Repository) were plated at a density of 300,000 cells/well in 6-well plates (day 0) in DMEM (high glucose) containing glutamine and penicillin-streptomycin (Bihain & Yen, 1992). Media was changed on day 2. On day 3, the confluent monolayers were washed once with phosphate-buffered saline (PBS, pH 7.4) (2 mL/well). Cells were incubated at 37°C for 30 min with increasing concentrations of recombinant AdipoQ (AQ) or globular AdipoQ (AQ-GH) in DMEM containing 0.2% (w/v) BSA, 5 mM Hepes, 2 mM CaCl₂, 3.7 g/L, sodium bicarbonate, pH 7.5. Incubations were continued for 3 h at 37°C after addition of 10 ng/mL ¹²⁵I-mouse leptin (specific activity, 22100 cpm/ng). Monolayers were washed 2 times consecutively with PBS containing 0.2% BSA, followed by 1 wash with PBS/BSA, and then 2 times consecutively with PBS. Cells were lysed with 0.1 N NaOH containing 0.24 mM EDTA Lysates were collected into tubes, and counted in a gamma-counter.
Results of an exemplary experiment are shown as the mean of triplicate determinations in Fig. 5.

The results indicate that gOBG3 polypeptide fragments are at least 30% more efficient than OBG3 in increasing leptin uptake in a liver cell line (Fig. 5). This assay could be used to determine the efficiency of gOBG3 polypeptide fragments and related compounds (or agonists or antagonists) to increase or decrease leptin uptake into the liver, as well as the mechanism by which the gOBG3 polypeptide fragment/compound exerts this effect.

### Blood Brain Barrier Model:

The effect ofgOBG3 polypeptide fragments on leptin transport in the brain can be determined using brain-derived cells. One method that is envisioned is to use the blood/brain barrier model described by Dehouck, et al (J Neurochem 54:1798-801, 1990 that uses a co-culture of brain capillary endothelial cells and astrocytes to test the effects of gOBG3 polypeptide fragments on leptin (or other molecules) transport via LSR or other receptors.

This assay would be an indicator of the potential effect of gOBG3 polypeptide fragments on leptin transport to the brain and could be used to screen gOBG3 polypeptide fragment variants for their ability to modulate leptin transport through LSR or other receptors in the brain. In addition, putative agonists and antagonists of the effect of gOBG3 polypeptide fragments on leptin transport through LSR or other receptors could also be screened using this assay. Increased transport of leptin across the blood/brain barrier would presumably increase its action as a satiety factor.

### FACs Analysis of LSR Expression

The effect of gOBG3 polypeptide fragments on LSR can also be determined by measuring the level of LSR expression at the cell surface by flow surface cytometry, using anti-LSR antibodies and fluorescent secondary antibodies. Flow cytometry is a laser-based technology that is used to measure characteristics of biological particles. The underlying principle of flow cytometry is that light is scattered and fluorescence is emitted as light from the excitation source strikes the moving particles.

This is a high through-put assay that could be easily adapted to screen OBG3 and gOBG3 polypeptide fragments and variants as well as putative agonists or antagonists of gOBG3 polypeptide fragments. Two assays are provided below. The antibody, cell-line and gOBG3 polypeptide fragment analog would vary depending on the experiment, but a human cell-line, human anti-LSR antibody and globular APM1 could be used to screen for variants, agonists, and antagonists to be used to treat humans.

### Assay 1:

Cells are pretreated with either intact OBG3 or gOBG3 polypeptide fragments (or untreated) before harvesting and analysis by FACS. Cells are harvested using non-enzymatic dissociation solution (Sigma), and then are incubated for 1 h at 4°C with a 1:200 dilution of anti-LSR 81B or an irrelevant anti-serum in PBS containing 1% (w/v) BSA. After washing twice with the same buffer, goat anti-rabbit FITC-conjugated antibody (Rockland, Gilbertsville, PA) is added to the cells, followed by a further incubation for 30 min at 4 °C. After washing, the cells are fixed in 2% formalin. Flow cytometry analysis is done on a FACSCalibur cytometer (Becton-Dickinson, Franklin Lakes, NJ).

The *in vitro* Liver Cell Line assay (described above) has shown that LSR activity (leptin binding) increases with increasing concentrations of gOBG3 polypeptide fragments. Whle not wishing to be bound by any particular theory, this could either be the result of an increased number of LSR binding sites on the cell surface, or a change in affinity for leptin. The FACS assay would presumably be detecting changes in the number of LSR binding sites, although changes in conformation reflecting changes in affinity might also be detected. Preferably the antibody would be to the C-terminus of LSR.

### Assay 2:

Cells are cultured in T175 flasks according to manufacturer's instructions for 48 hours prior to analysis.

Cells are washed once with FACs buffer (1x PBS/2% FBS, filter sterilized), and manually scraped from the flask in 10 mLs of FACs buffer. The cell suspension is transferred to a 15 mL conical tube and centrifuged at 1200 rpm, 4°C for 5 minutes. Supernatant is discarded and cells are resuspended in 10 mL FACs buffer chilled to 4°C. A cell count is performed and the cell density adjusted with FACs buffer to a concentration of 1 x 10⁶ cells/ mL. One milliliter of cell suspension was added to each well of a 48 well plate for analysis. Cells are centrifuged at 1200 rpm for 5 minutes at 4°C. Plates are checked to ensure that cells are pelleted, the supernatant is removed and cells resuspended by running plate over a vortex mixer. One milliliter of FACs buffer is added to each well, followed by centrifugation at 1200 rpm for 5 minutes at 4°C. This described cell washing was performed a total of 3 times.

Primary antibody, titered in screening experiments to determine proper working dilutions (for example 1:25, 1:50, 1:100, 1:200, 1:400, 1:500, 1:800, 1:1000, 1:2000, 1:4000, 1:5000, or 1:10000), is added to cells in a total volume of 50 µL FACs buffer. Plates are incubated for 1h at 4°C protected from light. Following incubation, cells are washed 3 times as directed above. Appropriate secondary antibody, titered in screening experiments to determine proper working dilutions (for example 1:25, 1:50, 1:100, 1:200, 1:400, 1:500, 1:800, 1:1000, 1:2000, 1:4000, 1:5000, or 1:10000), is added to cells in a total volume of 50 µL FACs buffer. Plates are incubated for 1h at 4°C protected from light. Following incubation, cells are washed 3 times as directed above. Upon final wash, cells are resuspended in 500 µL FACs buffer and transfered to a FACs acquisition tube. Samples are placed on ice protected from light and analyzde within 1 hour.

### Cellular Binding and Uptake of gOBG-3 as Detected by Fluorescence Microscopy

Fluorecein isothiocyanate (FITC) conjugation of gOBG3: Purified gOBG3 at 1 mg/mL concentration was labeled with FITC using Sigma's FluoroTag FITC conjugation kit (Stock No. FITC-1). Protocol outlined in the Sigma Handbook for small scale conjugation was followed for gOBG3 labeling.

Cell Culture: C2C12 mouse skeletal muscle cells (ATCC, Manassas, VA CRL-1772) and Hepa-1-6 mouse hepatocytes (ATCC, Manassas, VA CRL-1830) were seeded into 6 well plates at a cell density of 2x10⁵ cells per well. C2C12 and Hepa-1-6 cells were cultured according to repository's instructions for 24-48 hours prior to analysis. Assay was performed when cells were 80% confluent.

FITC labeled gOBG3 cellular binding and uptake using microscopy: C2C12 and Hepa 1-6 cells were incubated in the presence/absence of antibody directed against human LSR (81B: N-terminal sequence of human LSR; does not cross react with mouse LSR and 93A: c-terminal sequence, cross reacts with mouse LSR) or an antiserum directed against gC1qr (953) for 1 hour at 37°C, 5% CO2. LSR antibodies were added to the media at a concentration of 2 µg/mL. The anti-gC1qr antiserum was added to the media at a volume of 2.5 µL undiluted serum (high concentration) or 1:100 dilution (low concentration). Following incubation with specified antibody, FITC-gOBG3 (50 nM/mL) was added to each cell culture well. Cells were again incubated for 1 hour at 37°C, 5% CO2. Cells were washed 2x with PBS, cells were scraped from well into 1 mL of PBS. Cell suspension was transferred to an eppendorftube and centrifuged at 1000 rpm for 2 minutes. Supernatant was removed and cells resuspended in 200 µL of PBS. Binding and uptake of FITC-gOBG3 was analyzed by fluorescence microscopy under 40X magnification.

Analysis of C2C12 and Hepa 1-6 cells reveals identical phenotypes with respect to FITC-gOBG3 binding and uptake profiles both in the presence or absence of LSR antibodies. FITC-gOBG3 appears to be localized within vesicles in the cytoplasm of both mouse hepatocytes and mouse myoblasts, suggesting that binding and uptake of FITC-gOBG3 is occurring. FITC-gOBG3 uptake appears to be blocked when cells were pre-treated with the anti-LSR antibody that recognizes mouse LSR. However, binding of FITC- gOBG3 to the cell surface does occur in a small portion of the cells (C2C12 and Hepa 1-6). At low concentration of the gC1qr antiserum, FITC-gOBG3 appears to be localized within vesicles in the cytoplasm of both cell types, similarly to the phenotype of cells that have not received antibody pre-treatment prior to addition of FITC- gOBG3. FITC-gOBG3 uptake and binding phenotype is not affected by pre-treatment with an LSR antibody that does not recognize mouse LSR. Together, these data suggest that uptake of FTTC-gOBG3 can be blocked by a human LSR antibody which cross-reacts with mouse LSR. However, this phenotype is not reproduced with other non cross-reactive LSR antibodies. Thus, this assay may be useful for identifying agents that facilitate or prevent the uptake and/or binding of OBG3 or gOBG3 polypeptide fragments to cells.

### Effect on LSR as a Lipoprotein Receptor

The effect of gOBG3 on the lipoprotein binding, internalizing and degrading activity of LSR can also be tested. Measurement of LSR as lipoprotein receptor is described in Bihain & Yen, ((1992) Biochemistry May 19;31(19):4628-36 The effect of gOBG3 on the lipoprotein binding, internalizing and degrading activity of LSR (or other receptors) can be compared with that of intact OBG3, with untreated cells as an additional control. This assay can also be used to screen for active and inhibitory variants of gOBG3, as well as agonists and antagonists of obesity-related activity.

Human liver PLC cells (ATCC Repository) were plated at a density of 300,000 cells/well in 6-well plates (day 0) in DMEM (high glucose) containing glutamine and penicillin-streptomycin (Bihain & Yen, 1992). Media was changed on day 2. On day 3, the confluent monolayers were washed once with phosphate-buffered saline (PBS, pH 7.4) (2 mL/well). Cells were incubated at 37°C for 30 min with 10 ng/mL human recombinant leptin in DMEM containing 0.2% (w/v) BSA, 5 mM Hepes, 2 mM CaCl₂, 3.7 g/L sodium bicarbonate, pH 7.5, followed by another 30 min incubation at 37°C with increasing concentrations of gOBG3. Incubations were continued for 2 h at 37°C after addition of 0.8 mM oleate and 20 µg/mL ¹²⁵I-LDL. Monolayers were washed 2 times consecutively with PBS containing 0.2% BSA, followed by 1 wash with PBS/BSA, and then 2 times consecutively with PBS. The amounts of oleate-induced binding, uptake and degradation of ¹²⁵I-LDL were measured as previously described (Bihain & Yen, 1992, supra). Results are shown as the mean of triplicate determinations.

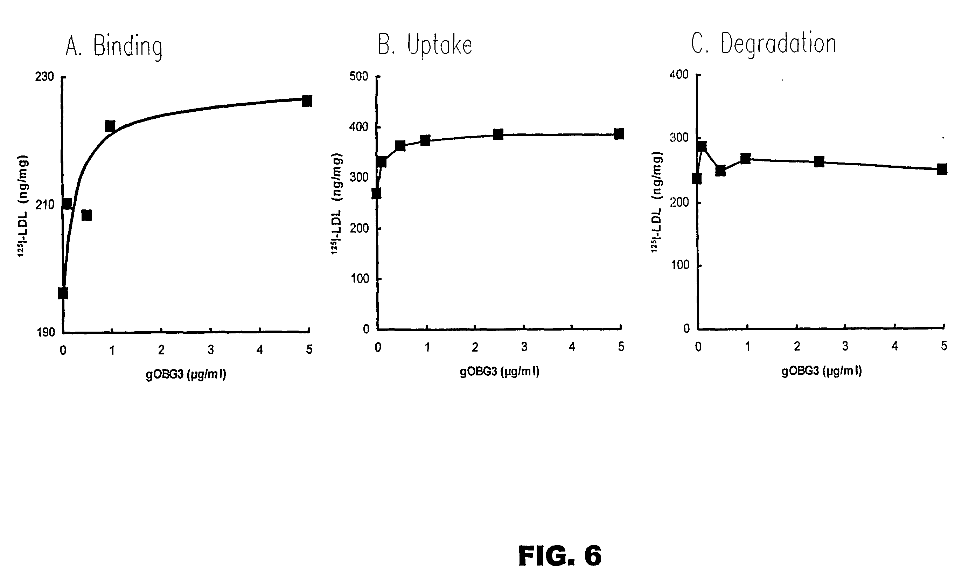

As shown in Figure 6, the addition of gOBG3 leads to an increased activity of LSR as a lipoprotein receptor. The oleate-induced binding and uptake of LDL appears more affected by gOBG3 as compared to the degradation. This increased LSR activity would potentially result in an enhanced clearance of triglyceride-rich lipoproteins during the postprandial state. Thus, more dietary fat would be removed through the liver, rather than being deposited in the adipose tissue.

This assay could be used to determine the efficiency of a compound (or agonists or antagonists) to increase or decrease LSR activity (or lipoprotein uptake, binding and degradation through other receptors), and thus affect the rate of clearance of triglyceride-rich lipoproteins.

### Effect on Muscle Differentiation

C2C12 cells (murine skeletal muscle cell line; ATCC CRL 1772, Rockville, MD) are seeded sparsely (about 15-20%) in complete DMEM (w/glutamine, pen/strep, etc) + 10% FCS. Two days later they become 80-90% confluent. At this time, the media is changed to DMEM+2% horse serum to allow differentiation. The media is changed daily. Abundant myotube formation occurs after 3-4 days of being in 2% horse serum, although the exact time course of C2C12 differentiation depends on how long they have been passaged and how they have been maintained, among other things.

To test the effect of the presence of gACRP30 on muscle differentiation, gACRP30 (1 to 2.5 µg/mL) was added the day after seeding when the cells were still in DMEM w/ 10% FCS. Two days after plating the cells (one day after gACRP30 was first added), at about 80-90% confluency, the media was changed to DMEM+2% horse serum plus gACRP30.

The results show that the addition of gACRP30 causes the cells to begin organizing within one day after its addition. In contrast to the random orientation of the cells not treated with gACRP30, those treated with gACRP30 aligned themselves in relation to each other. In addition, differentiation occurred after only 2 days of gACRP30 treatment, in contrast to the 3 to 4 days needed in its absence.

### Effect on Muscle Cell Fatty Acid Oxidation

C2C12 cells were differentiated in the presence or absence of 2 µg/mL gACRP30 for 4 days. On day 4, oleate oxidation rates were determined by measuring conversion of 1-¹⁴C-oleate (0.2 mM) to ¹⁴CO₂ for 90 min. C2C12 cells differentiated in the presence of gACRP30 undergo 40% more oleate oxidation than controls differentiated in the absence of gACRP30. This experiment can be used to screen for active fragments and peptides as well as agonists and antagonists or activators and inhibitors of OBG3 and gOBG3 polypeptides.

The effect of gACRP30 on the rate of oleate oxidation was compared in differentiated C2C12 cells (murine skeletal muscle cells; ATCC, Manassas, VA CRL-1772) and in a hepatocyte cell line (Hepal-6; ATCC, Manassas, VA CRL-1830). Cultured cells were maintained according to manufacturer's instructions. The oleate oxidation assay was performed as previously described (Muoio et al (1999) Biochem J 338;783-791). Briefly, nearly confluent myocytes were kept in low serum differentiation media (DMEM, 2.5% Horse serum) for 4 days, at which time formation of myotubes became maximal. Hepatocytes were kept in the same DMEM medium supplemented with 10% FCS for 2 days. One hour prior to the experiment the media was removed and 1 mL of preincubation media (MEM, 2.5% Horse serum, 3 mM glucose, 4 mM Glutamine, 25 mM Hepes, 1% FFA free BSA, 0.25 mM Oleate, 5 µg/mL gentamycin) was added. At the start of the oxidation experiment ¹⁴C-Oleic acid (1µCi/mL, American Radiolabeled Chemical Inc., St. Louis, MO) was added and cells were incubated for 90 min at 37°C in the absence/presence of 2.5 µg/mL gACRP30. After the incubation period 0.75 mL of the media was removed and assayed for ¹⁴C-oxidation products as described below for the muscle FFA oxidation experiment.

Oleate oxidation in C2C12 cells determined over 90 min increased significantly (39%; p = 0.036, two-tailed t-Test) in cells treated with gACRP30. In contrast, no detectable increase in the rate of FFA oxidation was seen in hepatocytes incubated with gACRP30.

### Triglyceride and Protein Analysis following Oleate Oxidaiton in cultured cells

Following transfer of media for oleate oxidation assay, cells were placed on ice. To determine triglyceride and protein content, cells were washed with 1 mL of 1x PBS to remove residual media. To each well 300 µL of cell dissociation solution (Sigma) was added and incubated at 37°C for 10 min. Plates were tapped to loosen cells, and 0.5 mL of 1x PBS was added. The cell suspension was transferred to an eppendorf tube, each well was rinsed with an additional 0.5 mL of 1x PBS, and was transferred to appropriate eppendorf tube. Samples were centrifuged at 1000 rpm for 10 minutes at room temperature. Supernatant was discarded and 750 µL of 1x PBS/2% chaps was added to cell pellet. Cell suspension was vortexed and place on ice for 1 hour. Samples were then centrifuged at 13000 rpm for 20 min at 4°C. Supernatants were transferred to new tube and frozen at -20°C until analyzed. Quantitative measure of triglyceride level in each sample was determined using Sigma Diagnostics GPO-TRINDER enzymatic kit. The procedure outlined in the manual was adhered to, with the following exceptions: assay was performed in 48 well plate, 350 µL of sample volume was assayed, control blank consisted of 350 µL PBS/2% chaps, and standard contained 10 µL standard provide in kit plus 690 µL PBS/2% chaps. Analysis of samples was carried out on a Packard Spectra Count at a wavelength of 550 nm. Protein analysis was carried out on 25 µL of each supernatant sample using the BCA protein assay (Pierce) following manufacturer's instructions. Analysis of samples was carried out on a Packard Spectra Count at a wavelength of 550 nm.

Triglyceride production in both C2C12 and Hepa 1-6 cells did not change significantly in the absence/presence of ACRP30 and gACRP30. The protein content of all cells analyzed was equivalent in the absence/presence of ACRP30 and gACRP30.

### EXAMPLE 5: Effect of gOBG3 on Mice Fed a High-Fat Diet

Experiments are performed using approximately 6 week old C57B1/6 mice (8 per group). All mice are housed individually. The mice are maintained on a high fat diet throughout each experiment. The high fat diet (cafeteria diet; D12331 from Research Diets, Inc.) has the following composition: protein kcal% 16, sucrose kcal% 26, and fat kcal% 58. The fat was primarily composed of coconut oil, hydrogenated.

After the mice are fed a high fat diet for 6 days, micro-osmotic pumps are inserted using isoflurane anesthesia, and are used to provide gOBG3, OBG3, saline, and an irrelevant peptide to the mice subcutaneously (s.c.) for 18 days. gOBG3 is provided at doses of 50, 25, and 2.5 µg/day; OBG3 is provided at 100, 50, and 5 µg/day; and the irrelevant peptide is provided at 10 µg/day. Body weight is measured on the first, third and fifth day of the high fat diet, and then daily after the start of treatment. Final blood samples are taken by cardiac puncture and are used to determine triglyceride (TG), total cholesterol (TC), glucose, leptin, and insulin levels. The amount of food consumed per day is also determined for each group.

In a preliminary experiment, mice treated with 2.5 µg/day gOBG3 had significantly lowered body weight.

### EXAMPLE 6: Tests of Obesity-related Activity in Humans

Tests of the efficacy of gOBG3 in humans are performed in accordance with a physician's recommendations and with established guidelines. The parameters tested in mice are also tested in humans (e.g. food intake, weight, TG, TC, glucose, insulin, leptin, FFA). It is expected that the physiological factors would show changes over the short term. Changes in weight gain might require a longer period of time. In addition, the diet would need to be carefully monitored. Globular OBG3 would be given in daily doses of about 6 mg protein per 70 kg person or about 10 mg per day. Other doses would also be tested, for instance 1 mg or 5 mg per day up to 20 mg, 50 mg, or 100 mg per day.

### EXAMPLE 7: Tests of Obesity-related Activity in a Murine Lipoatrophic Diabetes Model

Previously, leptin was reported to reverse insulin resistance and diabetes mellitus in mice with congenital lipodystrophy (Shimomura et al. Nature 401: 73-76 (1999) ). Leptin was found to be less effective in a different lipodystrophic mouse model of lipoatrophic diabetes (Gavrilova et al Nature 403: 850 (2000) OBG3 or gOBG3 polypeptide fragments may be used for reducing the insulin resistance and hyperglycaemia in this model either alone or in combination with leptin, the leptin peptide (US provisional application No 60/155,506), or other compounds. Assays include that described previously in Gavrilova et al. ((2000) Diabetes Nov;49(11):1910-6; (2000) Nature Feb 24;403(6772):850) using A-ZIP/F-1 mice, except that gOBG3 would be administered using the methods previously described in Example 5 (or Examples 8-10). The glucose and insulin levels of the mice would be tested, and the food intake and liver weight monitored, as well as other factors, such as leptin, FFA, and TG levels, typically measured in our experiments (see Example 5, above, or Examples 8-10).

### EXAMPLE 8: Effect of gOBG-3 on plasma Free Fatty Acid in C57 BL/6 Mice

The effect of the globular head of acrp-30 on postprandial lipemia (PPL) in normal C57BL6/J mice was tested. ACRP-30 is another name for adipo Q and is the mouse protein homologue to the human apm-1 protein. OBG3 is a generic way to refer to all of these forms. The globular head form is indicated by placing a 'g' in front, *e.g.* g-acrp30 or gOBG3. The gOBG3 used was prepared by proteolytic digestion of recombinant OBG3 as described previously in Example 2. Acetylated trypsin was used as protease.

The mice used in this experiment were fasted for 2 hours prior to the experiment after which a baseline blood sample was taken. All blood samples were taken from the tail using EDTA coated capillary tubes (50 µL each time point). At time 0 (8:30 AM), a standard high fat meal (6g butter, 6 g sunflower oil, 10 g nonfat dry milk, 10 g sucrose, 12 mL distilled water prepared fresh following Nb#6, JF, pg.1) was given by gavage (vol.=1% of body weight) to all animals.

Immediately following the high fat meal, 25µg gOBG3 was injected i.p. in 100 µL saline. The same dose (25µg/mL in 100µL) was again injected at 45 min and at I hr 45 min (treated group, n=8). Control animals (n=8) were injected with saline (3x100µL). Untreated and treated animals were handled in an alternating mode.

Blood samples were taken in hourly intervals, and were immediately put on ice. Plasma was prepared by centrifugation following each time point. Plasma was kept at -20°C and free fatty acids (FFA), triglycerides (TG) and glucose were determined within 24 hours using standard test kits (Sigma and Wako). Due to the limited amount of plasma available, glucose was determined in duplicate using pooled samples. For each time point, equal volumes of plasma from all 8 animals per treatment group were pooled. Error bars shown for glucose therefore represent the SD of the duplicate determination and not the variation between animals as for TG and FFA.

### Results

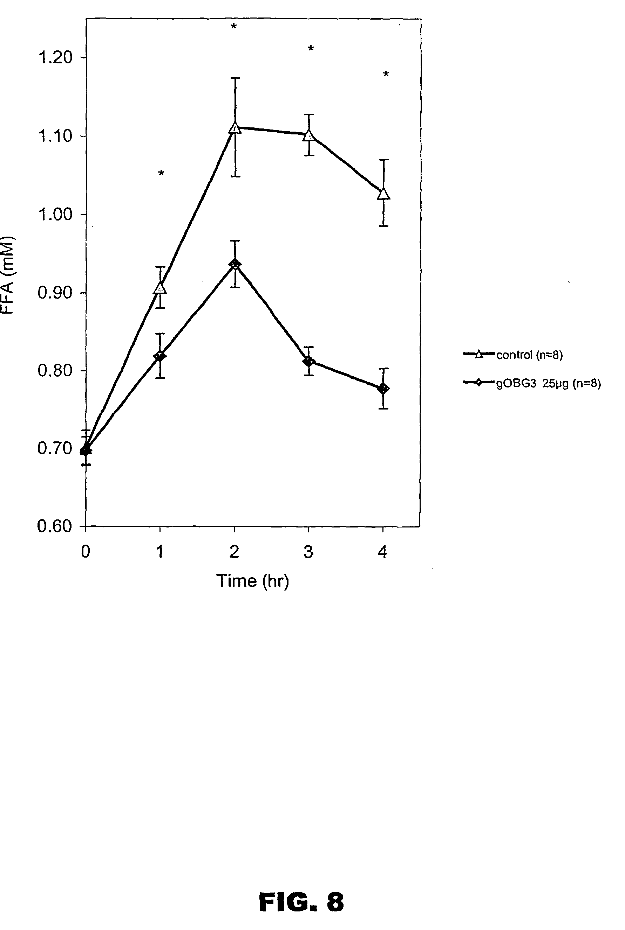

The increase in plasma FFA due to the high fat meal was significantly lower in mice treated with gOBG3 at all time points between 1 and 4 hr. This can be interpreted as increase in FFA oxidation (Fig.8).

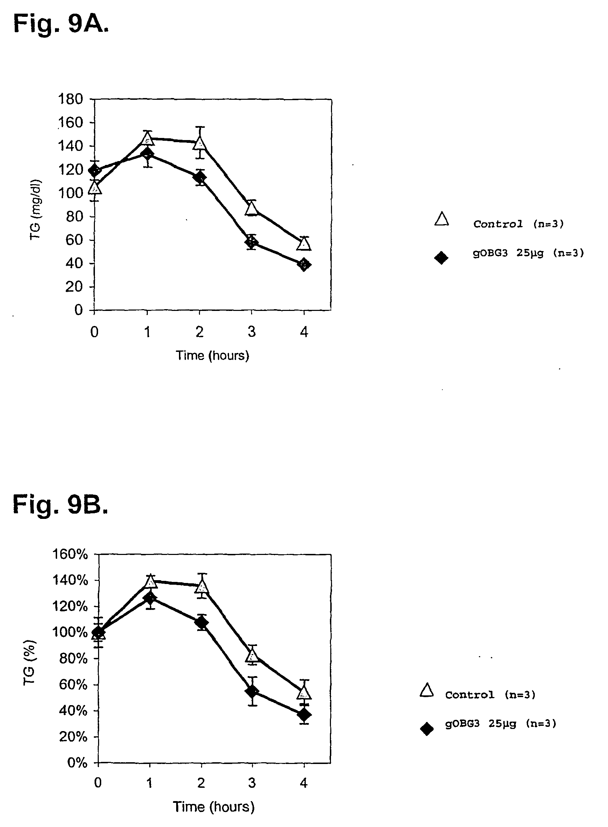

Treatment with gOBG3 also led to a significantly smaller increase in plasma TG compared to untreated mice. However, this effect was less pronounced than the effect on FFA (Fig.9).

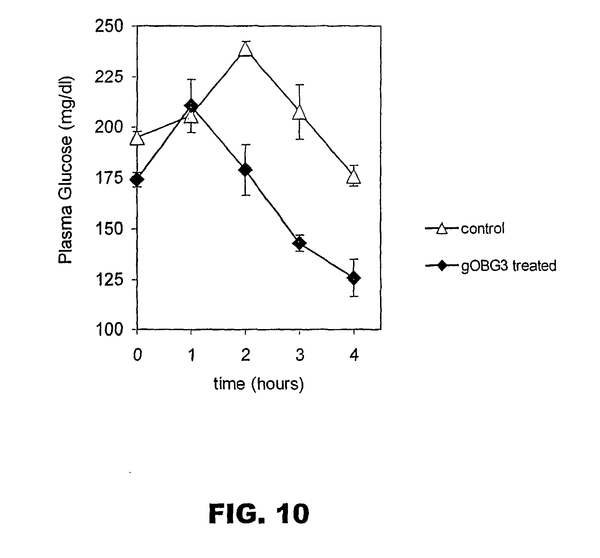

Glucose turnover was significantly improved following treatment with gOBG3; this effect can be interpreted as improved insulin sensitivity possibly due to the decrease in FFA (Fig.10).

Similar results were seen previously in a prior experiment involving only 2 treatments (at 0 and at 45 minutes; data not shown). A strong FFA lowering effect of gOBG3 coupled with a less dominant TG lowering effect was observed.

### EXAMPLE 9: Effect of gOBG-3 on Plasma Leptin and Insulin in C57 BL/6 Mice

The effect of the globular head of acrp-30 on plasma leptin and insulin levels during postprandial lipemia (PPL) in normal C57BL6/J mice was tested. The experimental procedure was the same as that described in Example 8, except that blood was drawn only at 0, 2 and 4 hours to allow for greater blood samples needed for the determination of leptin and insulin by RIA.

Briefly, 16 mice were fasted for 2 hours prior to the experiment after which a baseline blood sample was taken. All blood samples were taken from the tail using EDTA coated capillary tubes (100 µL each time point). At time 0 (9:00AM), a standard high fat meal (see Example 8) was given by gavage (vol.=1% of body weight) to all animals. Immediately following the high fat meal, 25 µg gOBG3 was injected i.p. in 100 µL saline. The same dose (25µg in 100µL) was again injected at 45 min and at 1 hr 45 min (treated group, n=8). Control animals (n=8) were injected with saline (3x100µL). Untreated and treated animals were handled in an alternating mode.

Blood samples were immediately put on ice and plasma was prepared by centrifugation following each time point. Plasma was kept at -20°C and free fatty acids (FFA) were determined within 24 hours using a standard test kit (Wako). Leptin and Insulin were determined by RIA (ML-82K and SRI-13K, LINCO Research, Inc., St. Charles, MO) following the manufacturer's protocol. However, only 20 µL plasma was used. Each determination was done in duplicate. Due to the limited amount of plasma available, leptin and insulin were determined in 4 pools of 2 animals each in both treatment groups.

### Results

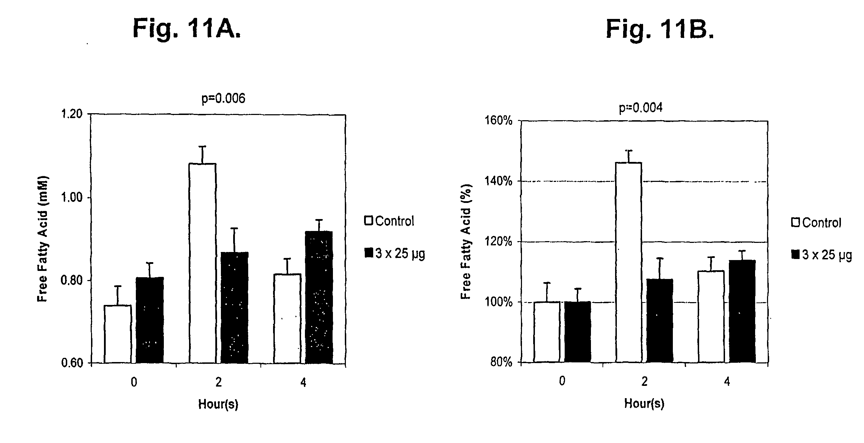

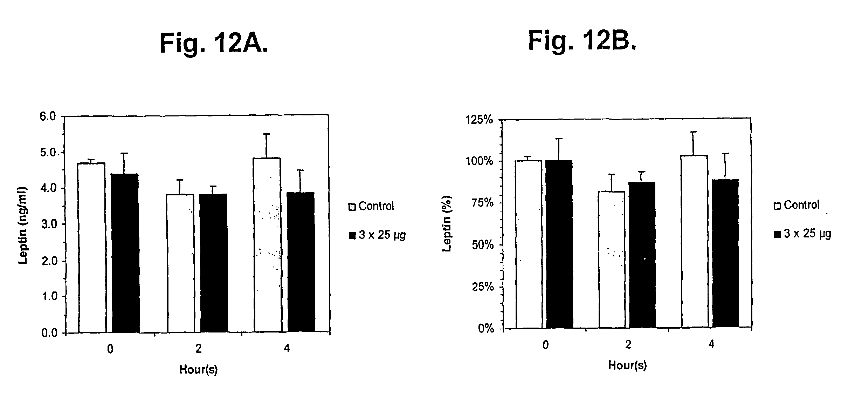

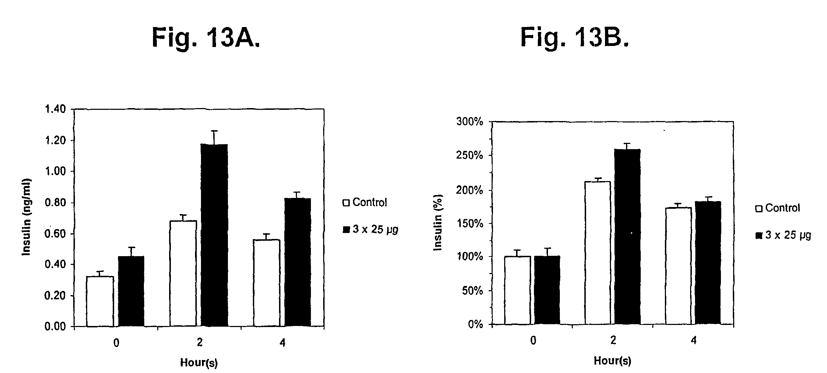

As shown previously (Example 8), treatment with gOBG3 significantly reduced the postprandial increase in plasma FFA caused by the high fat meal at 2 hours (Fig. 11). There was no significant change in plasma leptin levels at any time point; treatment with gOBG3 did not affect leptin levels (Fig. 12). Insulin levels (Fig. 13) indicate a marginal increase in insulin at 2 hours. However, when analyzed as percentage change from t₀, this increase (212% vs. 260%, control vs. treated) was statistically not significant (p = 0.09).

These data reconfirm the previously shown acceleration of FFA metabolism by treatment with gOBG3. They also show that gOBG3 does not affect leptin and insulin plasma levels and that gOBG3 reduces hyperglycemia during postprandial lipemia and also induces weight loss during treatment over several days. Without being limited by any particular theory, the data suggests: a) that the reduction in weight is caused by a leptin independent increase in metabolism; and b) that gOBG3 leads to increased insulin sensitivity.

### EXAMPLE 10: Effect of OBG-3 on Plasma FFA, TG and Glucose in C57 BL/6 Mice

The effect of the globular head of acrp30 on plasma FFA, TG, glucose, leptin and insulin levels during postprandial lipemia (PPL) in normal C57BL6/J mice has been described. Weight loss resulting from gOBG3 (2.5µg/day) given to normal C57BL6/J mice on a high fat diet has also been shown (Example 5). In comparison, a much higher dose of the complete form of acrp30 (200µg/day) was needed to induce a relatively smaller effect in mice. This example shows the effect of the acrp30-complete form on plasma FFA, TG and glucose levels.

The experimental procedure was similar to that described in Example 8. Briefly, 14 mice were fasted for 2 hours prior to the experiment after which a baseline blood sample was taken. All blood samples were taken from the tail using EDTA coated capillary tubes (50 µL each time point). At time 0 (9:00AM), a standard high fat meal (see Example 8) was given by gavage (vol.=1% of body weight) to all animals. Immediately following the high fat meal, 4 mice were injected 25 µg OBG3 i.p. in 100µL saline. The same dose (25µg in 100µL) was again injected at 45 min and at 1 hr 45 min. A second treatment group (n=4) received 3 times 50 µg OBG3 at the same intervals. Control animals (n=6) were injected with saline (3x100µL). Untreated and treated animals were handled in an alternating mode.

Blood samples were immediately put on ice. Plasma was prepared by centrifugation following each time point. Plasma was kept at -20 °C and free fatty acids (FFA), triglycerides (TG) and glucose were determined within 24 hours using standard test kits (Sigma and Wako).

### Results

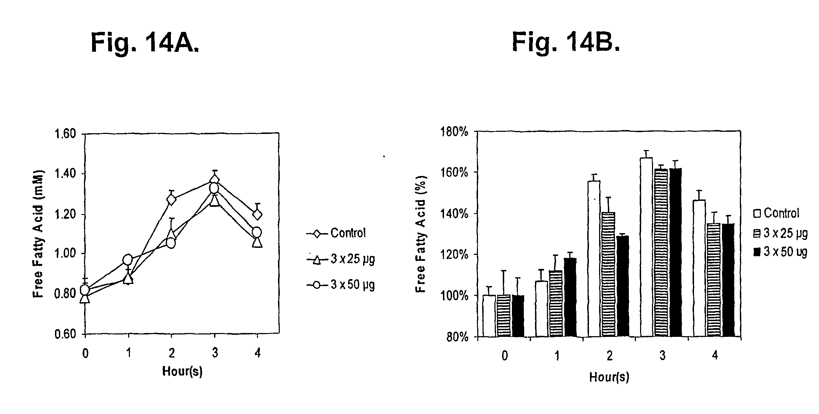

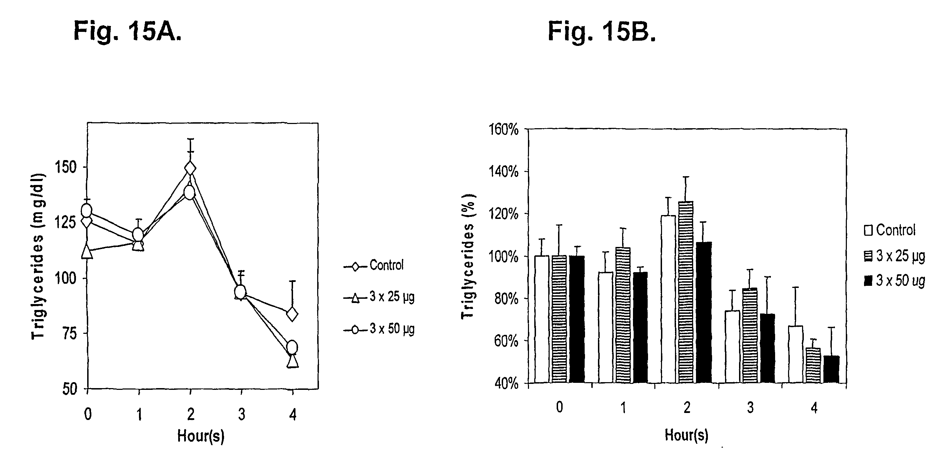

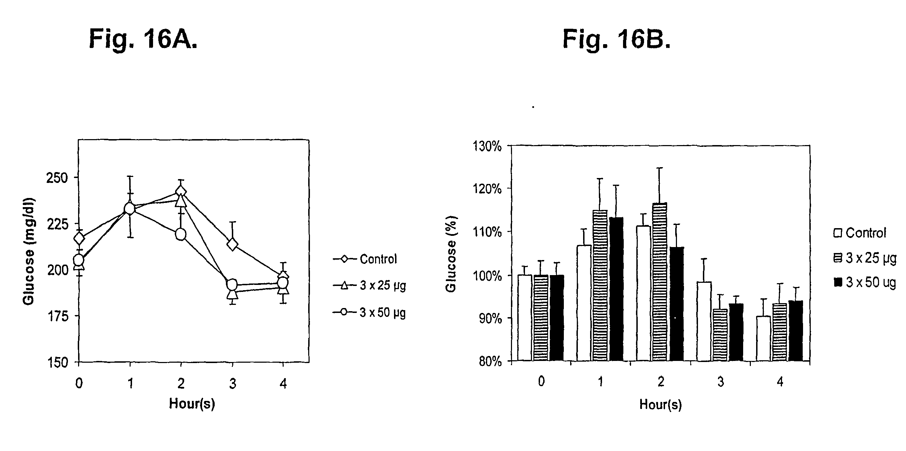

Treatment with full length OBG3 had no effect on plasma FFA levels (Fig.14) except for t = 2 hours when a statistically significant reduction was shown (p<0.05). No significant change in postprandial TG (Fig. 15) and glucose levels (Fig.16) was seen in treated animals.

The data presented show that the complete form of OBG3 did not reduce FFA, TG and glucose levels in contrast to what was observed for the globular region (Examples 5, 8, 9). Only at 2 hours post-gavage, did treatment with OBG3 reduce FFA plasma concentrations significantly (p<0.05). These results demonstrate that gOBG3 is much more active *in vivo* than the full length protein. A similar effect was seen for body weight reduction; the globular head was much more active than the full-length protein.

### EXAMPLE 11: Effect of gACRP30 on FFA following Epinephrine Injection

In mice, plasma free fatty acids increase after intragastric administration of a high fat/sucrose test meal. These free fatty acids are mostly produced by the activity of lipolytic enzymes *i.e.* lipoprotein lipase (LPL) and hepatic lipase (HL). In this species, these enzymes are found in significant amounts both bound to endothelium and freely circulating in plasma¹⁶. Another source of plasma free fatty acids is hormone sensitive lipase (HSL) that releases free fatty acids from adipose tissue after β-adrenergic stimulation. To test whether gACRP30 also regulates the metabolism of free fatty acid released by HSL, mice were injected with epinephrine.

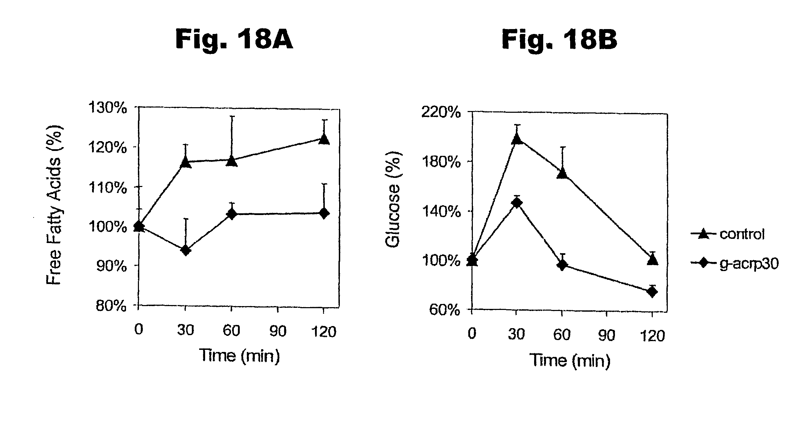

Two groups of mice (n=5 each) were given epinephrine (5µg) by intraperitoneal injection. A treated group was injected with gACRP30 (25µg) one hour before and again together with epinephrine, while control animals received saline. Plasma was isolated and free fatty acids and glucose were measured as described above (Example 10). As shown in Fig. 18, epinephrine injections (5 µg) caused an increase in plasma free fatty acids and glucose. Both effects were significantly reduced in gACRP30- treated mice.

This reduction in the increases of glucose and FFA levels was not due to blockage of the β-adrenergic effect of epinephrine, as shown by inducing the release of FFA from isolated adipose tissue *in vitro.* In these control studies, adipose tissue was removed from normal C57BL/6J mice and incubated in Krebs-Henseleit bicarbonate buffer. Epinephrine was added and the concentration of FFA in the medium following a 90 min incubation was determined. Epinephrine (10 µM) caused a 1.7-fold increase in free fatty acids in the media. Increasing concentrations of gACRP30 or ACRP30 up to 50µg/ml did not inhibit this effect of epinephrine.

The data presented thus far indicate that the globular region of ACRP30 exerts profound pharmacological effects on the metabolism of energy substrates with the most evident effect on plasma free fatty acids. Further, the reduction in plasma FFA concentration cannot be explained by inhibition of either LPL - this would cause an increase in plasma triglycerides while a decrease of plasma triglycerides is actually observed - or by inhibition of HSL. Thus, the simplest explanation is that gACRP30 causes increased removal of free fatty acids from the circulation by promoting cellular uptake.

### EXAMPLE 12: Effect of gACRP30 on Muscle FFA Oxidation

To investigate the effect of gACRP30 on muscle free fatty acid oxidation, intact hind limb muscles from C57BL/6J mice were isolated and FFA oxidation was measured using oleate as substrate (Clee et al (2000) J Lipid Res 41:521-531; Muoio et al (1999) Am J Physiol 276:E913-921). Oleate oxidation in isolated muscle was measured as previously described (Cuendet et al (1976) J Clin Invest 58:1078-1088; Le Marchand-Brustel (1978) Am J Physiol 234:E348-E358). Briefly, mice were sacrificed by cervical dislocation and soleus and EDL muscles were rapidly isolated from the hind limbs. The distal tendon of each muscle was tied to a piece of suture to facilitate transfer among different media. All incubations were carried out at 30°C in 1.5 mL of Krebs-Henseleit bicarbonate buffer (118.6 mM NaCl, 4.76 mM KCI, 1.19 mM KH₂PO₄, 1.19 mM MgSO₄, 2.54 mM CaCl₂, 25mM NaHCO₃, 10 mM Hepes, pH 7.4) supplemented with 4% FFA free bovine serum albumin (fraction V, RIA grade, Sigma) and 5 mM glucose (Sigma). The total concentration of oleate (Sigma) throughout the experiment was 0.25 mM. All media were oxygenated (95% O₂; 5% CO₂) prior to incubation. The gas mixture was hydrated throughout the experiment by bubbling through a gas washer (Kontes Inc., Vineland, NJ).

Muscles were rinsed for 30 min in incubation media with oxygenation. The muscles were then transferred to fresh media (1.5 mL) and incubated at 30°C in the presence of 1µCi/mL [1-¹⁴C] oleic acid (American Radiolabeled Chemicals). The incubation vials containing this media were sealed with a rubber septum from which a center well carrying a piece of Whatman paper (1.5 cm x 11.5 cm) was suspended.

After an initial incubation period of 10min with constant oxygenation, gas circulation was removed to close the system to the outside environment and the muscles were incubated for 90 min at 30°C. At the end of this period, 0.45 mL of Solvable (Packard Instruments, Meriden, CT) was injected onto the Whatman paper in the center well and oleate oxidation by the muscle was stopped by transferring the vial onto ice.

After 5 min, the muscle was removed from the medium, and an aliquot of 0.5 mL medium was also removed. The vials were closed again and 1 mL of 35% perchloric acid was injected with a syringe into the media by piercing through the rubber septum. The CO₂ released from the acidified media was collected by the Solvable in the center well. After a 90 min collection period at 30°C, the Whatman paper was removed from the center well and placed in scintillation vials containing 15 mL of scintillation fluid (HionicFlour, Packard Instruments, Meriden, CT). The amount of ¹⁴C radioactivity was quantitated by liquid scintillation counting. The rate of oleate oxidation was expressed as nmol oleate produced in 90min/g muscle.

To test the effect of gACRP30 or ACRP30 on oleate oxidation, these proteins were added to the media at a final concentration of 2.5 µg/mL and maintained in the media throughout the procedure.

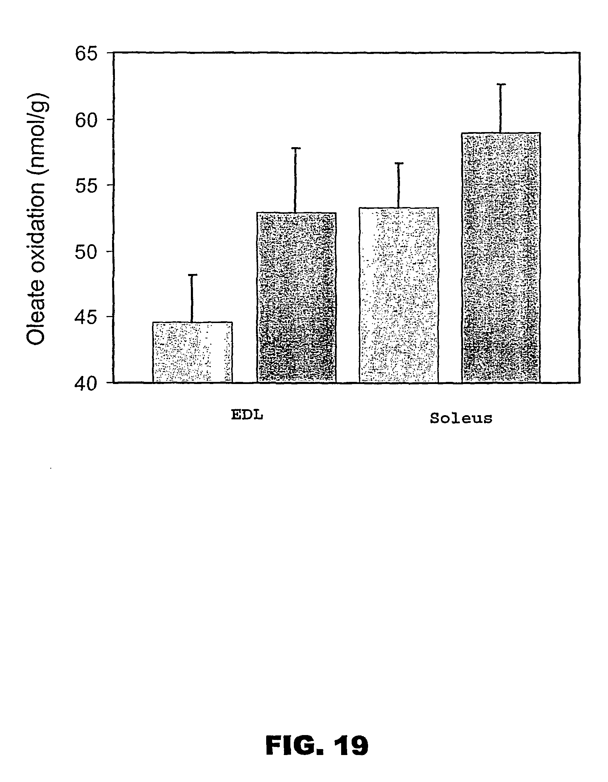

Two muscles of different oxidative capacity (soleus and extensor digitorum longus (EDL)) were tested (Fig. 19). EDL and Soleus muscles were isolated from both legs of normal C57BL/6J mice (n=18). One muscle of each pair was incubated in medium with 2.5 µg/mL gACRP30 (dark gray) and one in medium without gACRP30 (control - light gray). This experimental design allowed us to compare oleate oxidation in pairs of muscles isolated from the same animal. ¹⁴C-Oleate oxidation was determined over 90 minutes. Incubation of EDL and soleus muscles for 90 minutes in medium containing 2.5µg/ml gACRP30 leads to a statistically significant increase in oleate oxidation (p<0.05, paired, one-tailed, t-Test) or (p=0.0041, Repeated Measures Analysis of Variance, Univariate Tests of Hypotheses for Within Subject Effects) in both muscle types.

Both muscle types showed a significant response to gACRP30. The relative increase in FFA oxidation was 17% (p=0.03) and 10% (p=0.04) for EDL and soleus, respectively. In humans, muscles represent approximately 25% of body weight. Therefore, even a moderate increase in free fatty acid oxidation can have quantitatively important consequences on overall energy utilization.

### EXAMPLE 13: Effect of gArcp30 on Triglyceride in Muscle & Liver Isolated from Mice

To determine whether the increased FFA oxidation induced by gACRP30 is also accompanied by increased FFA delivery into muscle or liver, the hindlimb muscle and liver triglyceride content was measured after gACRP30 treatment of mice. Hind limb muscles as well as liver samples were removed from treated and untreated animals and the triglyceride and free fatty acid concentration was determined following a standard lipid extraction method (Shimabukuro et al (1997) Proc Natl Acad Sci USA 94:4637-4641) followed by TG and FFA analysis using standard test kits.

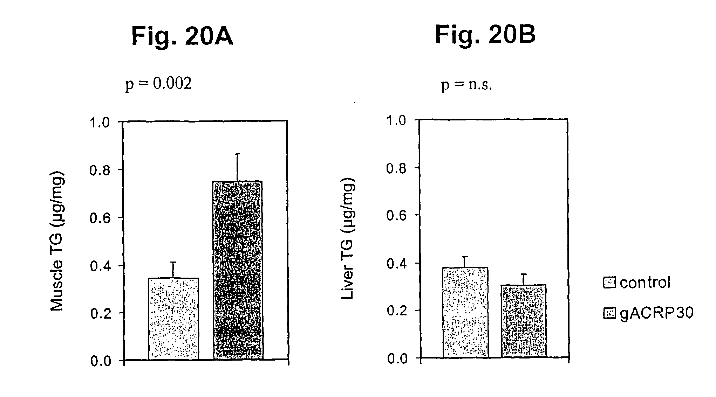

Short-term treatment of animals with gACRP30 (2 injections of 25 µg each given within 3 hours before sacrifice) did not change the triglyceride content either of hind limb muscle or liver tissue (data not shown). However, after 3 days of treatment, during which period normal C57BL/6J mice consumed a regular rodent diet, mice that had received 25 µg of gACRP30 twice daily showed significantly higher (p=0.002) muscle triglyceride content (Fig.20A) than those receiving saline (control: light gray; gACRP30: dark gray). This contrasted with a lack of increase in liver triglycerides (Fig. 20B). Furthermore, no detectable increase in muscle TG was observed after the 16-day treatment shown independently by directly measuring the muscle TG content and by oil red O staining of frozen microscope sections. In summary, the data indicate that the increase in TG content was transient.

These data are consistent with the notion that gACRP30 increases the rate of removal of free fatty acids from plasma at least partly by increasing their delivery to the muscle; much of the FFAs are immediately oxidized while some are stored as triglycerides and subsequently oxidized. Further support for this interpretation was obtained by measuring the concentration of ketone bodies in plasma of treated and untreated animals following a high fat/sucrose meal.

Ketone bodies (KB) are produced in the liver as a result of free fatty acid oxidation, but KB formation does not occur significantly in muscle. In mice receiving the high fat test meal and saline injection, the level of plasma KB increased significantly over the next 3 hours (183 ± 12%, n=6). Animals treated with gACRP30, on the other hand, showed no increase in plasma KB concentrations. Thus, gACRP30 inhibits either directly KB formation or can decrease KB production by inhibiting liver FFA oxidation.

### EXAMPLE 14: Effect of gACRP30 on Weight Gain & Weight Loss of Mice

Two independent studies showed that gACRP30 also affects overall energy homeostasis. In the first, 10-week-old male C57BL/6J mice were put on a very high fat/sucrose purified diet for 19 days to promote weight gain (see Example 5); the average body weight at this time was 30g. The mice were then surgically implanted with an osmotic pump (Alzet, Newark, DE) delivering either 2.5 µg/day of gACRP30, 5µg/day of ACRP30, or physiological saline. The mice were continued on the high fat diet and their body weight was recorded over the following 10-day period.

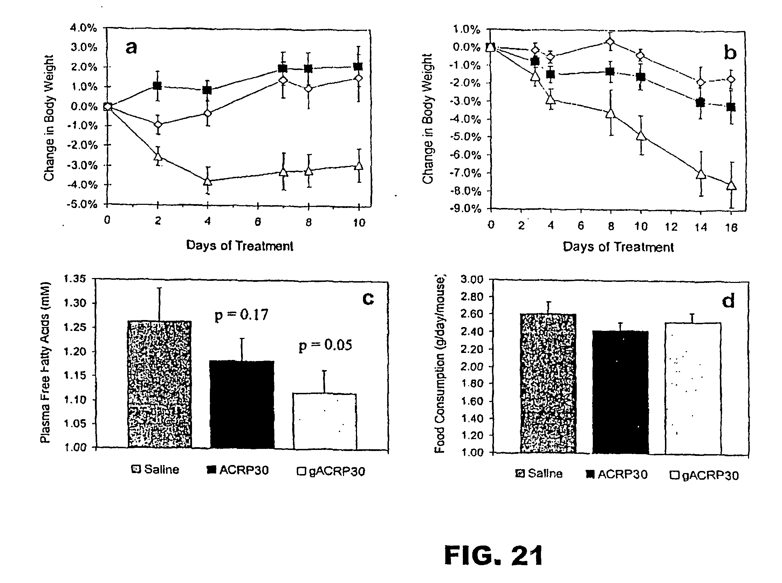

Mice treated with saline or 5 µg/day of full length ACRP30 continued to gain weight at an average daily rate of 0.16% and 0.22%, respectively. In contrast, mice treated with gACRP30 experienced a significant weight reduction (-3.7%, p = 0.002) during the first 4 days and then their weight remained constant (Fig. 21A). Thus, in this inbred strain of normal mice, a continuous infusion of a daily low dose of gACRP30 can prevent weight gain caused by high fat/sucrose feeding, in a sustainable way.

This result was confirmed and extended in a second study performed in mature 9 month old, male obese C57BL/6J mice that had been on the same high fat/sucrose diet for 6 months; the average body weight when the study began was 52.5±0.8g. Three groups of 8 mice were treated with saline, ACRP30 or gACRP30 for 16 days. Animals in the treated group received twice daily 25 µg of protein subcutaneously. Body weights were recorded at the indicated time points.

Treatment with gACRP30 led to significant (p<0.05) weight loss at day 3. This effect became even more significant as the study continued. During the 16 day study period, the obese C57BL/6J mice that received gACRP30 lost about 8% (p=0.001) of their initial body weight despite the fact that they were maintained on a high fat/sucrose diet (Fig. 21B). Saline treated animals showed only marginal fluctuations in their body weight (p = n.s.). Animals treated with the full length ACRP30, but at a 10-fold higher dose than that used in the first experiment, also lost significant weight (-3.2%, p=0.025). Interestingly, mice treated with gACRP30 continued to lose weight at a steady rate during the 16-day study period, while the rate of weight reduction in those treated with the full length ACRP30 decreased during the later phase of the study. Food consumption in gACRP30 treated animals was not significantly different from saline or ACRP30 treated animals (Fig. 21D).

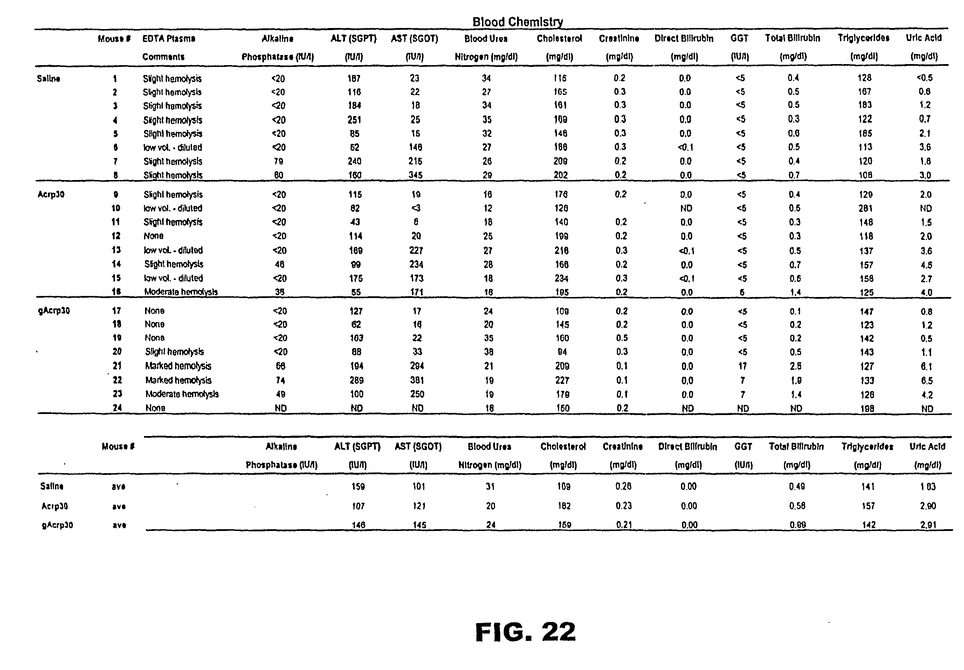

Treatment with gACRP30 caused a significant reduction in the concentration of plasma free fatty acids (Fig. 21C). This effect was significant after 3 days of treatment (p<0.05 vs. saline) and continued throughout the complete study period. Shown is the plasma FFA level at day 16 of the study. The initial FFA plasma concentration was the same in all three treatment groups. It should be noted, however, that despite this reduction the plasma free fatty acid concentration of these massively obese animals remains about 40-60% higher than that of normal mice. A blood chemistry analysis (including determination of SGPT, SGOT, urea, creatinine or bilirubin) performed on the terminal blood samples did not reveal any abnormal plasma parameters (Fig. 22).

Data are expressed throughout as mean ± SEM; a p-value < 0.05 was considered statistically significant. Statistical analysis was typically done using either the unpaired Student's t test or the paired Student's t test, as indicated in each study.

### EXAMPLE 15: Detection of APM-1 (gOBG3) Fragment in Human Plasma After Immunoprecipitation

The recombinant form of ACRP30 protein used has an apparent molecular weight of 37 kDa and forms a dimer of 74 kDa (Fig 23A, Lane II). A proteolytic fragment that contains the entire globular head region (gACRP30) and that migrates with an apparent molecular weight of 18 kDa was generated using acetylated trypsin (Fig. 23A, lane I). Both protein preparations (ACRP30 and gACRP30) were essentially endotoxin free; ActiClean Etox affinity columns (Sterogene Bioseparations Inc., Carlsbad, CA) were used to remove potential endotoxin contaminations following the manufacturer's protocol. Endotoxin levels were determined by Endosafe, Charleston, SC. As determined by N-terminal sequencing of purified gACRP30, the site of cleavage was just before amino acid 104 (just before amino acid 101 for human gOBG3 or APM1).

Immunoprecipitation of human plasma Apm1 followed by Western blotting was used to detect a cleavage product of apm-1, the human homolog of ACRP30, using a globular head specific anti-serum for the immunoprecipitation step as well as for the detection step. Preimmune serum or serum raised against the globular head domain or human non-homologous region (HDQETTTQGPGVLLPLPKGA) were cross-linked to protein A (Sigma Chemical CO, Saint Louis, MO) using dimethyl-pimelimidate-dihydrochloride (Sigma Chemical Co, Saint Louis, MO). After washing (0.2 M salt) proteins were eluted from protein A, separated by SDS-PAGE, transferred to Protran® pure nitrocellulose membrane (Schleicher and Schuell, Keene, NH) using standard procedures. Apm-1 products were visualized using globular head domain antibodies labeled with biotin; horseradish peroxidase conjugated to Streptavidin and CN/DAB substrate kit (Pierce, Rockford, IL) according to manufacturer's instructions.

The apparent molecular weight of this truncated form was 27 kDa, corresponding to about 70% of the complete form of apm-1 (Fig. 23B, Lane IV). This truncated form was not detectable when immunoprecipitation was performed using a different antibody directed against the human non-homologous region (HDQETTTQGPGVLLPLPKGA) of apm-1; this domain is located toward the NH₂ terminal end of the protein outside of the globular domain (Fig. 23, Lane V). Both anti-apm-1 antibodies directed against either the globular or the non-globular domain identified the full-length form of the protein, as well as a low abundance dimer of apparent MW 74 kDa.

### EXAMPLE 16: Effect of gACRP30 on FFA following Intralipid Injection

Two groups of mice (n=5 each) were intravenously (tail vein) injected with 30 µL bolus of Intralipid-20% (Clintec) to generate a sudden rise in plasma FFAs, thus by-passing intestinal absorption. (Intralipid is an intravenous fat emulsion used in nutritional therapy). A treated group (◆ gACRP30-treated) was injected with gACRP30 (25µg) at 30 and 60 minutes before Intralipid was given, while control animals (▲ control) received saline. Plasma was isolated and FFAs were measured as described previously.

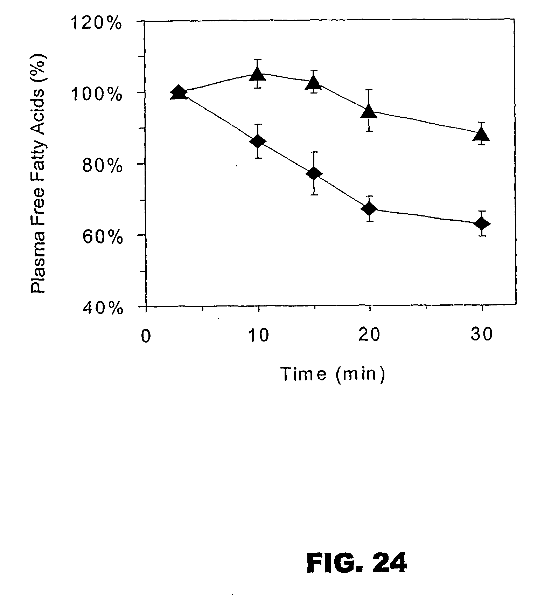

The effect of gACRP30 on the decay in plasma FFAs following the peak induced by Intralipid injection was then monitored. As shown in Fig. 24, gACRP30 accelerates the removal of FFAs from plasma after Intralipid injection. Thus, gACRP30 accelerates the clearance of FFAs without interfering with intestinal absorption. Although not wishing to be bound by any theory, because Intralipid does not elicit a significant insulin response, the results also indicate that gACRP30 regulation of FFA metabolism occurs independently of insulin.

### REFERENCES

Dehouck et al. J Neurochem 54:1798-801, 1990
1. Scherer, P. E., Williams, S., Fogliano, M., Baldini, G. & Lodish, H. F. A novel serum protein similar to Clq, produced exclusively in adipocytes. J Biol Chem 270, 26746-26749 (1995).
2. Shapiro, L. & Scherer, P. E. The crystal structure of a complement-lq family protein suggests an evolutionary link to tumor necrosis factor. Curr Biol 8, 335-338 (1998).
3. Hu, E., Liang, P. & Spiegelman, B. M. AdipoQ is a novel adipose-specific gene dysregulated in obesity. J Biol Chem 271, 10697-10703 (1996).
4. Maeda, K. et al. cDNA cloning and expression of a novel adipose specific collagen-like factor, apM1 (AdiPose Most abundant Gene transcript 1). Biochem Biophys Res Commun 221, 286-289 (1996).
5. Nakano, Y., Tobe, T., Choi-Miura, N. H., Mazda, T. & Tomita, M. Isolation and characterization of GBP28, a novel gelatin-binding protein purified from human plasma. J Biochem (Tokyo) 120, 803-812 (1996).
6. Kishore, U. & Reid, K. B. Modular organization of proteins containing Clq-like globular domain. Immunopharmacology 42, 15-21 (1999).
7. Kavety, B. & Morgan, J. I. Characterization of transcript processing of the gene encoding precerebellin-1. Brain Res Mol Brain Res 63, 98-104 (1998).
8. Satoh, F. et al. Cerebellin and cerebellin mRNA in the human brain, adrenal glands and the tumour tissues of adrenal tumour, ganglioneuroblastoma and neuroblastoma. J Endocrinol 154, 27-34 (1997).
9. Mazzocchi, G. et al. Cerebellin enhances in vitro secretory activity of human adrenal gland. J Clin Endocrinol Metab 84, 632-635 (1999).
10. Kondo, N. & Kondo, J. Identification of novel blood proteins specific for mammalian hibernation. J Biol Chem 267, 473-478 (1992).
11. Takamatsu, N., Ohba, K., Kondo, J., Kondo, N. & Shiba, T. Hibernation-associated gene regulation of plasma proteins with a collagen-like domain in mammalian hibernators. Mol Cell Biol 13, 1516-1521 (1993).
12. Spiegelman, B. M. & Hotamisligil, G. S. Through thick and thin: wasting, obesity, and TNF alpha. Cell 73, 625-627 (1993).
13. Saito, K. et al. Regulation of gelatin-binding protein 28 (GBP28) gene expression by C/EBP. Biol Pharm Bull 22, 1158-1162 (1999).
14. Takamatsu, N. et al. Expression of multiple alphal-antitrypsin-like genes in hibernating species of the squirrel family. Gene 204, 127-132 (1997).
15. Ross, S. R., Graves, R. A. & Spiegelman, B. M. Targeted expression of a toxin gene to adipose tissue: transgenic mice resistant to obesity. Genes Dev 7, 1318-1324 (1993).
16. Moitra, J. et al. Life without white fat: a transgenic mouse. Genes Dev 12, 3168-3181 (1998).
17. Clee, S. M. et al. Plasma and vessel wall lipoprotein lipase have different roles in atherosclerosis. JLipid Res 41, 521-531 (2000).
18. Muoio, D. M., Dohm, G. L., Tapscott, E. B. & Coleman, R. A. Leptin opposes insulin's effects on fatty acid partitioning in muscles isolated from obese ob/ob mice. Am J Physiol 276, E913-921 (1999).
19. Peters, S. J., Dyck, D. J., Bonen, A. & Spriet, L. L. Effects of epinephrine on lipid metabolism in resting skeletal muscle. Am JPhysiol 275, E300-309 (1998).
20. Rigotti, A., Acton, S. L. & Krieger, M. The class B scavenger receptors SR-BI and CD36 are receptors for anionic phospholipids. JBiol Chem 270, 16221-16224 (1995).
21. Hirsch, D., Stahl, A. & Lodish, H. F. A family of fatty acid transporters conserved from mycobacterium to man. Proc Natl Acad Sci USA 95, 8625-8629 (1998).
22. Hotamisligil, G. S. et al. IRS-1-mediated inhibition of insulin receptor tyrosine kinase activity in TNF-alpha- and obesity-induced insulin resistance. Science 271, 665-668 (1996).
23. Peraldi, P. & Spiegelman, B. TNF-alpha and insulin resistance: summary and future prospects. Mol Cell Biochem 182, 169-175 (1998).
24. Kirchgessner, T. G., Uysal, K. T., Wiesbrock, S. M., Marino, M. W. & Hotamisligil, G. S. Tumor necrosis factor-alpha contributes to obesity-related hyperleptinemia by regulating leptin release from adipocytes. J Clin Invest 100, 2777-2782 (1997).
25. Uysal, K. T., Wiesbrock, S. M., Marino, M. W. & Hotamisligil, G. S. Protection from obesity-induced insulin resistance in mice lacking TNF- alpha function. Nature 389, 610-614 (1997).
26. Jensen, D. R. et al. Prevention of diet-induced obesity in transgenic mice overexpressing skeletal muscle lipoprotein lipase. Am J Physiol 273, R683-689 (1997).
27. Levak-Frank, S. et al. Muscle-specific overexpression of lipoprotein lipase causes a severe myopathy characterized by proliferation of mitochondria and peroxisomes in transgenic mice. J Clin Invest 96, 976-986 (1995).
28. Scheja, L. et al. Altered insulin secretion associated with reduced lipolytic efficiency in aP2-/- mice. Diabetes 48, 1987-1994 (1999).
29. Griffin, M. E. et al. Free fatty acid-induced insulin resistance is associated with activation of protein kinase C theta and alterations in the insulin signaling cascade. Diabetes 48, 1270-1274 (1999).
30. Dresner, A. et al. Effects of free fatty acids on glucose transport and IRS-1-associated phosphatidylinositol 3-kinase activity. J Clin Invest 103, 253-259 (1999).
31. Kelley, D. E., Goodpaster, B., Wing, R. R. & Simoneau, J. A. Skeletal muscle fatty acid metabolism in association with insulin resistance, obesity, and weight loss. Am J Physiol 277, E1130-1141 (1999).
32. Roden, M. et al. Mechanism of free fatty acid-induced insulin resistance in humans. J Clin Invest 97, 2859-2865 (1996).
33. Cuendet, G. S., Loten, E. G., Jeanrenaud, B. & Renold, A. E. Decreased basal, noninsulin-stimulated glucose uptake and metabolism by skeletal soleus muscle isolated from obese-hyperglycemic (ob/ob) mice. J Clin Invest 58, 1078-1088 (1976).
34. Le Marchand-Brustel, Y., Jeanrenaud, B. & Freychet, P. Insulin binding and effects in isolated soleus muscle of lean and obese mice. Am J Physiol 234, E348-E358 (1978).
35. Shimabukuro, M. et al. Direct antidiabetic effect of leptin through triglyceride depletion of tissues. Proc Natl Acad Sci USA 94, 4637-4641 (1997).

## Claims

1. Use of a globular fragment of OBG3, wherein the globular OBG3 fragment is selected from the group consisting of
(i) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6; or
(ii) a variant of any of the globular OBG3 fragments having one or more permissive amino acid substitutions, wherein each of said permissive amino acid substitutions is a conservative amino acid substitution wherein the variant has at least one but not more than 10 conservative amino acid substitutions; or
(iii) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6 linked to polyethylene glycol or in the form of an IgG Fc fusion peptide;
the globular OBG3 fragment of (ii) or (iii) causing a change in at least one of the parameters selected from post-prandial lipemia, free fatty acid level, triglyceride level, glucose level, free fatty acid oxidation, or weight, the change caused by the globular OBG3 fragment of (ii) or (iii) being comparable to a change caused by the globular OBG3 fragment of (i), for the preparation of a medicament for the prevention or treatment of an obesity related disorder.

2. Use according to claim 1, wherein the obesity related disorder is selected from obesity, insulin-resistance, atherosclerosis, atheromatous disease, heart disease, hypertension, stroke, syndrome X, non-insulin dependent diabetes, and type II diabetes.

3. Use of a globular fragment of OBG3, wherein the globular OBG3 fragment is selected from the group consisting of
(i) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6; or
(ii) a variant of any of the globular OBG3 fragments having one or more permissive amino acid substitutions, wherein each of said permissive amino acid substitutions is a conservative amino acid substitution wherein the variant has at least one but not more than 10 conservative amino acid substitutions; or
(iii) amino acids 104 to 247 of SEQ ID NO: 2 or SEQ ID NO: 4, amino acids 111 to 247 of SEQ ID NO: 2 or SEQ ID NO: 4, amino acids 135 to 247 of SEQ ID NO: 2 or SEQ ID NO: 4, amino acids 101 to 244 of SEQ ID NO: 6, amino acids 108 to 244 of SEQ ID NO: 6, and amino acids 132 to 244 of SEQ ID NO: 6 linked to polyethylene glycol or in the form of an IgG Fc fusion peptide;
the globular OBG3 fragment of (ii) or (iii) causing a change in at least one of the parameters selected from post-prandial lipemia, free fatty acid level, triglyceride level, glucose level, free fatty acid oxidation, or weight, the change caused by the globular OBG3 fragment of (ii) or (iii) being comparable to a change caused by the globular OBG3 fragment of (i), for the preparation of a medicament for the reduction of body mass or prevention of weight gain.

4. Use according to any one of claims 1 to 3, wherein said conservative amino acid substitution is:
(a) the replacement of one amino acid with another amino acid, wherein the amino acid to be replaced is selected from the aliphatic amino acids Ala, Val, Leu and Phe, and is replaced with another of said aliphatic amino acids;
(b) interchange of the hydroxyl residues Ser and Thr;
(c) exchange of the acidic residues Asp and Glu;
(d) substitution between the amide residues Asn and Gln;
(e) exchange of the basic residues Lys and Arg; or
(f) the replacement of one aromatic residue amino acid with another aromatic residue amino acid, wherein the amino acid Phe is replaced with Tyr, or the amino acid Tyr is replaced with Phe.

5. Use according to any one of claims 1 to 4, wherein the globular OBG3 fragment has an amino acid sequence consisting of amino acids 101 to 244 of SEQ ID NO: 6.

6. Use according to any one of the previous claims, wherein the globular OBG3 fragment has an amino acid sequence consisting of amino acids 108 to 244 of SEQ ID NO: 6.

7. Use according to any one of claims 1 to 6, wherein the globular OBG3 fragment or variant is a homotrimer.

8. Use according to any one of claims 1 to 7, wherein the globular OBG3 fragment or variant has a modified peptide bond, which is resistant to proteolysis.

## Patentansprüche

1. Verwendung eines globulären Fragments von OBG3, wobei das globuläre OBG3-Fragment aus der Gruppe bestehend aus
(i) Aminosäuren 104 bis 247 der SEQ ID NO: 2 oder SEQ ID NO: 4, Aminosäuren 111 bis 247 der SEQ ID NO: 2 oder SEQ ID NO: 4, Aminosäuren 135 bis 247 der SEQ ID NO: 2 oder SEQ ID NO: 4, Aminosäuren 101 bis 244 der SEQ ID NO: 6, Aminosäuren 108 bis 244 der SEQ ID NO: 6 und Aminosäuren 132 bis 244 der SEQ ID NO: 6 oder
(ii) einer Variante irgendeines der globulären OBG3-Fragmente, die eine oder mehrere permissive Aminosäuresubstitution(en) aufweist, wobei jede der permissiven Aminosäuresubstitutionen eine konservative Aminosäuresubstitution ist, wobei die Variante mindestens eine, aber nicht mehr als 10 konservative Aminosäuresubstitutionen aufweist, oder
(iii) Aminosäuren 104 bis 247 der SEQ ID NO: 2 oder SEQ ID NO: 4, Aminosäuren 111 bis 247 der SEQ ID NO: 2 oder SEQ ID NO: 4, Aminosäuren 135 bis 247 der SEQ ID NO: 2 oder SEQ ID NO: 4, Aminosäuren 101 bis 244 der SEQ ID NO: 6, Aminosäuren 108 bis 244 der SEQ ID NO: 6 und Aminosäuren 132 bis 244 der SEQ ID NO: 6 an Polyethylenglykol gebunden oder in Form eines IgG-Fc-Fusionspeptids,
ausgewählt ist, wobei das globuläre OBG3-Fragment von (ii) oder (iii) eine Änderung bei mindestens einem der Parameter, ausgewählt aus postprandialer Lipämie, Spiegel freier Fettsäure, Triglyzeridspiegel, Glukosespiegel, Oxidation freier Fettsäure oder Gewicht, bewirkt, wobei die Änderung, die durch das globuläre OBG3-Fragment von (ii) oder (iii) bewirkt ist, mit einer Änderung vergleichbar ist, die durch das globuläre OBG3-Fragment von (i) bewirkt ist, für die Herstellung eines Medikaments zur Prävention oder Behandlung einer mit Fettleibigkeit im Zusammenhang stehenden Funktionsstörung.

2. Verwendung nach Anspruch 1, wobei die mit Fettleibigkeit im Zusammenhang stehende Funktionsstörung aus Adipositas, Insulinresistenz, Atherosklerose, Atheromatose, Herzerkrankung, Bluthochdruck, Schlag, Syndrom x, nichtinsulinabhängigem Diabetes und Diabetes Typ II ausgewählt ist.

3. Verwendung eines globulären Fragments von OBG3, wobei das globuläre OBG3-Fragment aus der Gruppe, bestehend aus
(i) Aminosäuren 104 bis 247 der SEQ ID NO: 2 oder SEQ ID NO: 4, Aminosäuren 111 bis 247 der SEQ ID NO: 2 oder SEQ ID NO: 4, Aminosäuren 135 bis 247 der SEQ ID No: 2 oder SEQ ID NO: 4, Aminosäuren 101 bis 244 der SEQ ID NO: 6, Aminosäuren 108 bis 244 der SEQ ID NO: 6 und Aminosäuren 132 bis 244 der SEQ ID NO: 6 oder
(ii) einer Variante irgendeines der globulären OBG3-Fragmente, die eine oder mehrere permissive Aminosäuresubstitution(en) aufweist, wobei jede der permissiven Aminosäuresubstitutionen eine konservative Aminosäuresubstitution ist, wobei die Variante mindestens eine, aber nicht mehr als 10 konservative Aminosäuresubstitutionen aufweist, oder
(iii) Aminosäuren 104 bis 247 der SEQ ID NO: 2 oder SEQ ID NO: 4, Aminosäuren 111 bis 247 der SEQ ID NO: 2 oder SEQ ID NO: 4, Aminosäuren 135 bis 247 der SEQ ID NO: 2 oder SEQ ID NO: 4, Aminosäuren 101 bis 244 der SEQ ID NO: 6, Aminosäuren 108 bis 244 der SEQ ID NO: 6 und Aminosäuren 132 bis 244 der SEQ ID NO: 6 an Polyethylenglykol gebunden oder in Form eines IgG-Fc-Fusionspeptids,
ausgewählt ist, wobei das globuläre OBG3-Fragment von (ii) oder (iii) eine Änderung bei mindestens einem der Parameter, ausgewählt aus postprandialer Lipämie, Spiegel freier Fettsäure, Triglyzeridspiegel, Glukosespiegel, Oxidation freier Fettsäure oder Gewicht, bewirkt, wobei die Änderung, die durch das globuläre OBG3-Fragment von (ii) oder (iii) bewirkt ist, mit einer Änderung vergleichbar ist, die durch das globuläre OBG3-Fragment von (i) bewirkt ist, für die Herstellung eines Medikaments zur Reduktion der Körpermasse oder Prävention einer Gewichtszunahme.

4. Verwendung nach einem der Ansprüche 1 bis 3, wobei die konservative Aminosäuresubstitution Folgendes ist:
(a) die Ersetzung einer Aminosäure durch eine andere Aminosäure, wobei die zu ersetzende Aminosäure aus den aliphatischen Aminosäuren Ala, Val, Leu und Phe ausgewählt ist und durch eine andere dieser aliphatischen Aminosäuren ersetzt wird,
(b) der Austausch der Hydroxylreste Ser und Thr,
(c) der Austausch der sauren Reste Asp und Glu,
(d) die Substitution zwischen den Amidresten Asn und Gin,
(e) der Austausch der basischen Reste Lys und Arg oder
(f) die Ersetzung einer aromatischen Rest-Aminosäure durch eine andere aromatische Rest-Aminosäure, wobei die Aminosäure Phe durch Tyr ersetzt wird oder die Aminosäure Tyr durch Phe ersetzt wird.

5. Verwendung nach einem der Ansprüche 1 bis 4, wobei das globuläre OBG3-Fragment eine Aminosäuresequenz aufweist, die aus den Aminosäuren 101 bis 244 der SEQ ID NO: 6 besteht.

6. Verwendung nach einem der vorhergehenden Ansprüche, wobei das globuläre OBG3-Fragment eine Aminosäuresequenz aufweist, die aus den Aminosäuren 108 bis 244 von SEQ ID NO: 6 besteht.

7. Verwendung nach einem der Ansprüche 1 bis 6, wobei das globuläre OBG3-Fragment oder die Variante davon ein Homotrimer ist.

8. Verwendung nach einem der Ansprüche 1 bis 7, wobei das globuläre OBG3-Fragment oder die Variante davon eine modifizierte Peptidbindung aufweist, die gegen Proteolyse resistent ist.

## Revendications

1. Utilisation d'un fragment globulaire d'OBG3, dans laquelle le fragment globulaire d'OBG3 est choisi dans le groupe constitué par
(i) les acides aminés 104 à 247 de SEQ ID NO : 2 ou SEQ ID NO : 4, les acides aminés 111 à 247 de SEQ ID NO : 2 ou SEQ ID NO : 4, les acides aminés 135 à 247 de SEQ ID NO : 2 ou SEQ ID NO : 4, les acides aminés 101 à 244 de SEQ ID NO : 6, les acides aminés 108 à 244 de SEQ ID NO : 6, et les acides aminés 132 à 244 de SEQ ID NO : 6 ; ou
(ii) une variante de l'un quelconque des fragments globulaires d'OBG3 ayant une ou plusieurs substitutions permissives d'acides aminés, chacune desdites substitutions permissives d'acides aminés étant une substitution conservative d'acide aminé, la variante ayant au moins une mais pas plus de 10 substitutions conservatives d'acides aminés ; ou
(iii) les acides aminés 104 à 247 de SEQ ID NO : 2 ou SEQ ID NO : 4, les acides aminés 111 à 247 de SEQ ID NO : 2 ou SEQ ID NO : 4, les acides aminés 135 à 247 de SEQ ID NO : 2 ou SEQ ID NO : 4, les acides aminés 101 à 244 de SEQ ID NO _{:} 6, les acides aminés 108 à 244 de SEQ ID NO : 6, et les acides aminés 132 à 244 de SEQ ID NO : 6 liés au polyéthylèneglycol ou sous la forme d'un peptide de fusion IgG Fc ;
le fragment globulaire d'OBG3 de (ii) ou (iii) entraînant une modification d'au moins l'un des paramètres choisis parmi la lipémie postprandiale, le niveau d'acides gras libres, le niveau de triglycérides, le niveau de glucose, l'oxydation des acides gras libres, ou le poids, la modification entraînée par le fragment globulaire d'OBG3 de (ii) ou de (iii) étant comparable à une modification entraînée par le fragment globulaire d'OBG3 de (i), pour la préparation d'un médicament destiné à la prévention ou au traitement d'un trouble lié à l'obésité.

2. Utilisation selon la revendication 1, dans laquelle le trouble lié à l'obésité est choisi parmi l'obésité, l'insulinorésistance, l'athérosclérose, une maladie athéromateuse, une cardiopathie, l'hypertension, un accident vasculaire cérébral, le syndrome X, le diabète non insulinodépendant, et le diabète de type II.

3. Utilisation d'un fragment globulaire d'OBG3, dans laquelle le fragment globulaire d'OBG3 est choisi dans le groupe constitué par
(i) les acides aminés 104 à 247 de SEQ ID NO : 2 ou SEQ ID NO : 4, les acides aminés 111 à 247 de SEQ ID NO : 2 ou SEQ ID NO : 4, les acides aminés 135 à 247 de SEQ ID NO : 2 ou SEQ ID NO : 4, les acides aminés 101 à 244 de SEQ ID NO : 6, les acides aminés 108 à 244 de SEQ ID NO : 6, et les acides aminés 132 à 244 de SEQ ID NO : 6 ; ou
(ii) une variante de l'un quelconque des fragments globulaires d'OBG3 ayant une ou plusieurs substitutions permissives d'acides aminés, chacune desdites substitutions permissives d'acides aminés étant une substitution conservative d'acide aminé, la variante ayant au moins une mais pas plus de 10 substitutions conservatives d'acides aminés ; ou
(iii) les acides aminés 104 à 247 de SEQ ID NO : 2 ou SEQ ID NO : 4, les acides aminés 111 à 247 de SEQ ID NO : 2 ou SEQ ID NO : 4, les acides aminés 135 à 247 de SEQ ID NO : 2 ou SEQ ID NO : 4, les acides aminés 101 à 244 de SEQ ID NO : 6, les acides aminés 108 à 244 de SEQ ID NO : 6, et les acides aminés 132 à 244 de SEQ ID NO : 6 liés au polyéthylèneglycol ou sous la forme d'un peptide de fusion IgG Fc ;
le fragment globulaire d'OBG3 de (ii) ou (iii) entraînant une modification d'au moins l'un des paramètres choisis parmi la lipémie postprandiale, le niveau d'acides gras libres, le niveau de triglycérides, le niveau de glucose, l'oxydation des acides gras libres, ou le poids, la modification entraînée par le fragment globulaire d'OBG3 de (ii) ou de (iii) étant comparable à une modification entraînée par le fragment globulaire d'OBG3 de (i), pour la préparation d'un médicament destiné à réduire la masse corporelle ou à prévenir la prise de poids.

4. Utilisation selon l'une quelconque des revendications 1 à 3, dans laquelle la substitution conservative d'acide aminé est :
(a) le remplacement d'un acide aminé par un autre acide aminé, l'acide aminé à remplacer étant sélectionné parmi les acides aminés aliphatiques Ala, Val, Leu et Phe, et étant remplacé par un autre desdits acides aminés aliphatiques ;
(b) l'échange des résidus hydroxyle Ser et Thr ;
(c) l'échange des résidus acides Asp et Glu ;
(d) la substitution entre les résidus amides Asn et Gln ;
(e) l'échange des résidus basiques Lys et Arg ; ou
(f) le remplacement d'un résidu d'acide aminé aromatique par un autre résidu d'acide aminé aromatique, l'acide aminé Phe étant remplacé par Tyr, ou l'acide aminé Tyr étant replacé par Phe.

5. Utilisation selon l'une quelconque des revendications 1 à 4, dans laquelle le fragment globulaire d'OBG3 a une séquence d'acides aminés constituée par les acides aminés 101 à 244 de SEQ ID NO : 6.

6. Utilisation selon l'une quelconque des revendications précédentes, dans laquelle le fragment globulaire d'OBG3 a une séquence d'acides aminés constituée par les acides aminés 108 à 244 de SEQ ID NO : 6.

7. Utilisation selon l'une quelconque des revendications 1 à 6, dans laquelle le fragment globulaire d'OBG3 ou une variante est un homotrimère.

8. Utilisation selon l'une quelconque des revendications 1 à 7, dans laquelle le fragment globulaire d'OBG3 ou une variante a une liaison peptidique modifiée, qui est résistante à la protéolyse.


### SEQUENCE LISTING

<110> GENSET
<120> OBG3 Globular Head and Uses Thereof for Decreasing Body Mass
<130> 341 627
<150> US 60/176,228
   <151> 2000-01-14
<150> US 60/198,087
   <151> 2000-04-13
<150> US 60/229,881
   <151> 2000-09-01
<160> 7
<170> Patent.pm
<210> 1
   <211> 1152
   <212> DNA
   <213> mus musculus
<400> 1
   gaattcggca cgaggg atg cta ctg ttg caa gct ctc ctg ttc ctc tta atc      52
                     Met Leu Leu Leu Gln Ala Leu Leu Phe Leu Leu Ile
                     1               5                   10
   ctg ccc agt cat gcc gaa gat gac gtt act aca act gaa gag cta gct       100
   Leu Pro Ser His Ala Glu Asp Asp Val Thr Thr Thr Glu Glu Leu Ala
           15                  20                  25
   cct gct ttg gtc cct cca ccc aag gga act tgt gca ggt tgg atg gca       148
   Pro Ala Leu Val Pro Pro Pro Lys Gly Thr Cys Ala Gly Trp Met Ala
       30                  35                  40
   ggc atc cca gga cat tct ggc cac aat ggc aca cca ggc cgt gat ggc       196
   Gly Ile Pro Gly His Ser Gly His Asn Gly Thr Pro Gly Arg Asp Gly
   45                  50                  55                  60
   aga gat ggc act cct gga gag aag gga gag aaa gga gat tca ggt ctt       244
   Arg Asp Gly Thr Pro Gly Glu Lys Gly Glu Lys Gly Asp Ser Gly Leu
                   65                  70                  75
   ctt ggt cct aag ggt gag aca gga gat gtt gga atg aca gga gct gaa       292
   Leu Gly Pro Lys Gly Glu Thr Gly Asp Val Gly Met Thr Gly Ala Glu
               80                  85                  90
   ggg cct cgg ggc ttc ccc gga acc cct ggc agg aaa gga gag cct gga       340
   Gly Pro Arg Gly Phe Pro Gly Thr Pro Gly Arg Lys Gly Glu Pro Gly
           95                  100                 105
   gaa gcc gct tat gtg tat cgc tca ggc ttc agt gtg ggg ctg gag acc       388
   Glu Ala Ala Tyr Val Tyr Arg Ser Gly Phe Ser Val Gly Leu Glu Thr
       110                 115                 120
   cgc gtc act gtt ccc aat gta ccc att cgc ttt act aag atc ttc tac       436
   Arg Val Thr Val Pro Asn Val Pro Ile Arg Phe Thr Lys Ile Phe Tyr
   125                 130                 135                 140
   aac caa cag aat cat tat gac aac agc act ggc aag ttc tac tgc aac       484
   Asn Gln Gln Asn His Tyr Asp Asn Ser Thr Gly Lys Phe Tyr Cys Asn
                   145                 150                 155
   att ccg gga ctc tac tac ttc tct tac cac atc acg gtg tac atg aaa       532
   Ile Pro Gly Leu Tyr Tyr Phe Ser Tyr His Ile Thr Val Tyr Met Lys
               160                 165                 170
   gat gtg aag gtg agc ctc ttc aag aag gac aag gcc gtt ctc ttc acc       580
   Asp Val Lys Val Ser Leu Phe Lys Lys Asp Lys Ala Val Leu Phe Thr
           175                 180                 185
   tac gac cag tat cag gaa aag aat gtg gac cag gcc tct ggc tct gtg       628
   Tyr Asp Gln Tyr Gln Glu Lys Asn Val Asp Gln Ala Ser Gly Ser Val
       190                 195                 200
   ctc ctc cat ctg gag gtg gga gac caa gtc tgg ctc cag gtg tat ggg       676
   Leu Leu His Leu Glu Val Gly Asp Gln Val Trp Leu Gln Val Tyr Gly
   205                 210                 215                 220
   gat ggg gac cac aat gga ctc tat gca gat aac gtc aac gac tct aca       724
   Asp Gly Asp His Asn Gly Leu Tyr Ala Asp Asn Val Asn Asp Ser Thr
                   225                 230                 235
   ttt act ggc ttt ctt ctc ttc cat gat acc aac tga ctgcaactac            770
   Phe Thr Gly Phe Leu Leu Phe His Asp Thr Asn  *
               240                 245
   tcatagccca tacaccagga gaatcatgga acgtcgacac actttcagct tagtttgaga     830
   gattgatttt attgcttagt ttgagagtcc tgagtattat ccacacgtgt actcacttgt     890
   tcattaaacg actttataaa aaataatttg tgttcctagt ccagaaaaaa aggcactccc     950
   tggtctccac gactcttaca tggtagcaat aacagaatga aaatcacatt tggtatgggg    1010
   gcttcacaat attcgcatga ctgtctggaa gtagaccatg ctatttttct gctcactgta    1070
   cacaaatatt gttcacataa accctataat gtaaatatga aatacagtga ttatcttctc    1130
   aaaaaaaact cgtgccgaat tc                                             1152
<210> 2
   <211> 247
   <212> PRT
   <213> mus musculus
<400> 2
   Met Leu Leu Leu Gln Ala Leu Leu Phe Leu Leu Ile Leu Pro Ser His
   1               5                   10                  15
   Ala Glu Asp Asp Val Thr Thr Thr Glu Glu Leu Ala Pro Ala Leu Val
               20                  25                  30
   Pro Pro Pro Lys Gly Thr Cys Ala Gly Trp Met Ala Gly Ile Pro Gly
           35                  40                  45
   His Ser Gly His Asn Gly Thr Pro Gly Arg Asp Gly Arg Asp Gly Thr
       50                  55                  60
   Pro Gly Glu Lys Gly Glu Lys Gly Asp Ser Gly Leu Leu Gly Pro Lys
   65                  70                  75                  80
   Gly Glu Thr Gly Asp Val Gly Met Thr Gly Ala Glu Gly Pro Arg Gly
                   85                  90                  95
   Phe Pro Gly Thr Pro Gly Arg Lys Gly Glu Pro Gly Glu Ala Ala Tyr
               100                 105                 110
   Val Tyr Arg Ser Gly Phe Ser Val Gly Leu Glu Thr Arg Val Thr Val
           115                 120                 125
   Pro Asn Val Pro Ile Arg Phe Thr Lys Ile Phe Tyr Asn Gln Gln Asn
       130                 135                 140
   His Tyr Asp Asn Ser Thr Gly Lys Phe Tyr Cys Asn Ile Pro Gly Leu
   145                 150                 155                 160
   Tyr Tyr Phe Ser Tyr His Ile Thr Val Tyr Met Lys Asp Val Lys Val
                   165                 170                 175
   Ser Leu Phe Lys Lys Asp Lys Ala Val Leu Phe Thr Tyr Asp Gln Tyr
               180                 185                 190
   Gln Glu Lys Asn Val Asp Gln Ala Ser Gly Ser Val Leu Leu His Leu
           195                 200                 205
   Glu Val Gly Asp Gln Val Trp Leu Gln Val Tyr Gly Asp Gly Asp His
       210                 215                 220
   Asn Gly Leu Tyr Ala Asp Asn Val Asn Asp Ser Thr Phe Thr Gly Phe
   225                 230                 235                 240
   Leu Leu Phe His Asp Thr Asn
                   245
<210> 3
   <211> 1276
   <212> DNA
   <213> mus musculus
<400> 3
   ctctaaagat tgtcagtgga tctgacgaca ccaaaagggc tcagg atg cta ctg ttg      57
                                                     Met Leu Leu Leu
                                                     1
   caa gct ctc ctg ttc ctc tta atc ctg ccc agt cat gcc gaa gat gac       105
   Gln Ala Leu Leu Phe Leu Leu Ile Leu Pro Ser His Ala Glu Asp Asp
   5                   10                  15                  20
   gtt act aca act gaa gag cta gct cct gct ttg gtc cct cca ccc aag       153
   Val Thr Thr Thr Glu Glu Leu Ala Pro Ala Leu Val Pro Pro Pro Lys
                   25                  30                  35
   gga act tgt gca ggt tgg atg gca ggc atc cca gga cat cct ggc cac       201
   Gly Thr Cys Ala Gly Trp Met Ala Gly Ile Pro Gly His Pro Gly His
               40                  45                  50
   aat ggc aca cca ggc cgt gat ggc aga gat ggc act cct gga gag aag       249
   Asn Gly Thr Pro Gly Arg Asp Gly Arg Asp Gly Thr Pro Gly Glu Lys
           55                  60                  65
   gga gag aaa gga gat gca ggt ctt ctt ggt cct aag ggt gag aca gga       297
   Gly Glu Lys Gly Asp Ala Gly Leu Leu Gly Pro Lys Gly Glu Thr Gly
       70                  75                  80
   gat gtt gga atg aca gga gct gaa ggg cca cgg ggc ttc ccc gga acc       345
   Asp Val Gly Met Thr Gly Ala Glu Gly Pro Arg Gly Phe Pro Gly Thr
   85                  90                  95                  100
   cct ggc agg aaa gga gag cct gga gaa gcc gct tat atg tat cgc tca       393
   Pro Gly Arg Lys Gly Glu Pro Gly Glu Ala Ala Tyr Met Tyr Arg Ser
                   105                 110                 115
   gcg ttc agt gtg ggg ctg gag acc cgc gtc act gtt ccc aat gta ccc       441
   Ala Phe Ser Val Gly Leu Glu Thr Arg Val Thr Val Pro Asn Val Pro
               120                 125                 130
   att cgc ttt act aag atc ttc tac aac caa cag aat cat tat gac ggc       489
   Ile Arg Phe Thr Lys Ile Phe Tyr Asn Gln Gln Asn His Tyr Asp Gly
           135                 140                 145
   agc act ggc aag ttc tac tgc aac att ccg gga ctc tac tac ttc tct       537
   Ser Thr Gly Lys Phe Tyr Cys Asn Ile Pro Gly Leu Tyr Tyr Phe Ser
       150                 155                 160
   tac cac atc acg gtg tac atg aaa gat gtg aag gtg agc ctc ttc aag       585
   Tyr His Ile Thr Val Tyr Met Lys Asp Val Lys Val Ser Leu Phe Lys
   165                 170                 175                 180
   aag gac aag gcc gtt ctc ttc acc tac gac cag tat cag gaa aag aat       633
   Lys Asp Lys Ala Val Leu Phe Thr Tyr Asp Gln Tyr Gln Glu Lys Asn
                   185                 190                 195
   gtg gac cag gcc tct ggc tct gtg ctc ctc cat ctg gag gtg gga gac       681
   Val Asp Gln Ala Ser Gly Ser Val Leu Leu His Leu Glu Val Gly Asp
               200                 205                 210
   caa gtc tgg ctc cag gtg tat ggg gat ggg gac cac aat gga ctc tat       729
   Gln Val Trp Leu Gln Val Tyr Gly Asp Gly Asp His Asn Gly Leu Tyr
           215                 220                 225
   gca gat aac gtc aac gac tct aca ttt act ggc ttt ctt ctc tac cat       777
   Ala Asp Asn Val Asn Asp Ser Thr Phe Thr Gly Phe Leu Leu Tyr His
       230                 235                 240
   gat acc aac tga ctgcaactac ccatagccca tacaccagga gaatcatgga          829
   Asp Thr Asn  *
   245
   acagtcgaca cactttcagc ttagtttgag agattgattt tattgcttag tttgagagtc    889
   ctgagtatta tccacacgtg tactcacttg ttcattaaac gactttataa aaaataattt    949
   gtgttcctag tccagaaaaa aaggcactcc ctggtctcca cgactcttac atggtagcaa   1009
   taacagaatg aaaatcacat ttggtatggg ggcttcacaa tattcgcatg actgtctgga   1069
   agtagaccat gctatttttc tgctcactgt acacaaatat tgttcacata aaccctataa   1129
   tgtaaatatg aaatacagtg attactcttc tcacaggctg agtgtatgaa tgtctaaaga   1189
   cccataagta ttaaagtggt agggataaat tggaaaaaaa aaaaaaaaaa aagaaaaact   1249
   ttagagcaca ctggcggccg ttactag                                       1276
<210> 4
   <211> 247
   <212> PRT
   <213> mus musculus
<400> 4
   Met Leu Leu Leu Gln Ala Leu Leu Phe Leu Leu Ile Leu Pro Ser His
   1               5                   10                  15
   Ala Glu Asp Asp Val Thr Thr Thr Glu Glu Leu Ala Pro Ala Leu Val
               20                  25                  30
   Pro Pro Pro Lys Gly Thr Cys Ala Gly Trp Met Ala Gly Ile Pro Gly
           35                  40                  45
   His Pro Gly His Asn Gly Thr Pro Gly Arg Asp Gly Arg Asp Gly Thr
       50                  55                  60
   Pro Gly Glu Lys Gly Glu Lys Gly Asp Ala Gly Leu Leu Gly Pro Lys
   65                  70                  75                  80
   Gly Glu Thr Gly Asp Val Gly Met Thr Gly Ala Glu Gly Pro Arg Gly
                   85                  90                  95
   Phe Pro Gly Thr Pro Gly Arg Lys Gly Glu Pro Gly Glu Ala Ala Tyr
               100                 105                 110
   Met Tyr Arg Ser Ala Phe Ser Val Gly Leu Glu Thr Arg Val Thr Val
           115                 120                 125
   Pro Asn Val Pro Ile Arg Phe Thr Lys Ile Phe Tyr Asn Gln Gln Asn
       130                 135                 140
   His Tyr Asp Gly Ser Thr Gly Lys Phe Tyr Cys Asn Ile Pro Gly Leu
   145                 150                 155                 160
   Tyr Tyr Phe Ser Tyr His Ile Thr Val Tyr Met Lys Asp Val Lys Val
                   165                 170                 175
   Ser Leu Phe Lys Lys Asp Lys Ala Val Leu Phe Thr Tyr Asp Gln Tyr
               180                 185                 190
   Gln Glu Lys Asn Val Asp Gln Ala Ser Gly Ser Val Leu Leu His Leu
           195                 200                 205
   Glu Val Gly Asp Gln Val Trp Leu Gln Val Tyr Gly Asp Gly Asp His
       210                 215                 220
   Asn Gly Leu Tyr Ala Asp Asn Val Asn Asp Ser Thr Phe Thr Gly Phe
   225                 230                 235                 240
   Leu Leu Tyr His Asp Thr Asn
                   245
<210> 5
   <211> 4517
   <212> DNA
   <213> Homo sapiens
<400> 5
   ctgattccat accagagggg ctcagg atg ctg ttg ctg gga gct gtt cta ctg       53
                                Met Leu Leu Leu Gly Ala Val Leu Leu
                                1               5
   cta tta gct ctg ccc ggg cat gac cag gaa acc acg act caa ggg ccc       101
   Leu Leu Ala Leu Pro Gly His Asp Gln Glu Thr Thr Thr Gln Gly Pro
   10                  15                  20                  25
   gga gtc ctg ctt ccc ctg ccc aag ggg gcc tgc aca ggt tgg atg gcg       149
   Gly Val Leu Leu Pro Leu Pro Lys Gly Ala Cys Thr Gly Trp Met Ala
                   30                  35                  40
   ggc atc cca ggg cat ccg ggc cat aat ggg gcc cca ggc cgt gat ggc       197
   Gly Ile Pro Gly His Pro Gly His Asn Gly Ala Pro Gly Arg Asp Gly
                45                  50                  55
   aga gat ggc acc cct ggt gag aag ggt gag aaa gga gat cca ggt ctt     245
   Arg Asp Gly Thr Pro Gly Glu Lys Gly Glu Lys Gly Asp Pro Gly Leu
           60                  65                  70
   att ggt cct aag gga gac atc ggt gaa acc gga gta ccc ggg gct gaa     293
   Ile Gly Pro Lys Gly Asp Ile Gly Glu Thr Gly Val Pro Gly Ala Glu
       75                  80                  85
   ggt ccc cga ggc ttt ccg gga atc caa ggc agg aaa gga gaa cct gga     341
   Gly Pro Arg Gly Phe Pro Gly Ile Gln Gly Arg Lys Gly Glu Pro Gly
   90                  95                  100                 105
   gaa ggt gcc tat gta tac cgc tca gca ttc agt gtg gga ttg gag act     389
   Glu Gly Ala Tyr Val Tyr Arg Ser Ala Phe Ser Val Gly Leu Glu Thr
                   110                 115                 120
   tac gtt act atc ccc aac atg ccc att cgc ttt acc aag atc ttc tac     437
   Tyr Val Thr Ile Pro Asn Met Pro Ile Arg Phe Thr Lys Ile Phe Tyr
               125                 130                 135
   aat cag caa aac cac tat gat ggc tcc act ggt aaa ttc cac tgc aac     485
   Asn Gln Gln Asn His Tyr Asp Gly Ser Thr Gly Lys Phe His Cys Asn
           140                 145                 150
   att cct ggg ctg tac tac ttt gcc tac cac atc aca gtc tat atg aag     533
   Ile Pro Gly Leu Tyr Tyr Phe Ala Tyr His Ile Thr Val Tyr Met Lys
       155                 160                 165
   gat gtg aag gtc agc ctc ttc aag aag gac aag gct atg ctc ttc acc     581
   Asp Val Lys Val Ser Leu Phe Lys Lys Asp Lys Ala Met Leu Phe Thr
   170                 175                 180                 185
   tat gat cag tac cag gaa aat aat gtg gac cag gcc tcc ggc tct gtg     629
   Tyr Asp Gln Tyr Gln Glu Asn Asn Val Asp Gln Ala Ser Gly Ser Val
                   190                 195                 200
   ctc ctg cat ctg gag gtg ggc gac caa gtc tgg ctc cag gtg tat ggg     677
   Leu Leu His Leu Glu Val Gly Asp Gln Val Trp Leu Gln Val Tyr Gly
               205                 210                 215
   gaa gga gag cgt aat gga ctc tat gct gat aat gac aat gac tcc acc     725
   Glu Gly Glu Arg Asn Gly Leu Tyr Ala Asp Asn Asp Asn Asp Ser Thr
           220                 225                 230
   ttc aca ggc ttt ctt ctc tac cat gac acc aac tga tcaccactaa          771
   Phe Thr Gly Phe Leu Leu Tyr His Asp Thr Asn  *
       235                 240                 245
   ctcagagcct cctccaggcc aaacagcccc aaagtcaatt aaaggctttc agtacggtta   831
   ggaagttgat tattatttag ttggaggcct ttagatatta ttcattcatt tactcattca   891
   tttattcatt cattcatcaa gtaactttaa aaaaatcata tgctatgttc ccagtcctgg   951
   ggagcttcac aaacatgacc agataactga ctagaaagaa gtagttgaca gtgctatttt  1011
   gtgcccactg tctctcctga tgctcatatc aatcctataa ggcacaggga acaagcattc  1071
   tcctgttttt acagattgta tcctgaggct gagagagtta agtgaatgtc taaggtcaca  1131
   cagtattaag tgacagtgct agaaatcaaa cccagagctg tggactttgt tcactagact  1191
   gtgccctttt atagaggtac atgttctctt tggagtgttg gtaggtgtct gtttcccacc  1251
   tcacctgaga gccattgaat ttgccttcct catgaattaa aacctccccc aagcagagct  1311
   tcctcagaga aagtggttct atgatgaagt cctgtcttgg aaggactact actcaatggc  1371
   ccctgcacta ctctacttcc tcttacctat gtcccttctc atgcctttcc ctccaacggg  1431
   gaaagccaac tccatctcta agtgctgaac tcatccctgt tcctcaaggc cacctggcca  1491
   ggagcttctc tgatgtgata tccacttttt tttttttttg agatggagtc tcactctgtc  1551
   acccaggctg gagtacagtg acacgacctc ggctcactgc agcctccttc tcctgggtcc  1611
   aagcaattat tgtgcctcag cctcccgagt agctgagact tcaggtgcat tccaccacac  1671
   atggctaatt tttgtatttt tagtagaaat ggggtttcgt catgttggcc aggctggtct  1731
   cgaactcctg gcctaggtga tccacccgcc tcgacctccc aaagtgctgg gattacaggc  1791
   atgagccacc atgcccagtc gatatctcac tttttatttt gccatggatg agagtcctgg  1851
   gtgtgaggaa cacctcccac caggctagag gcaactgccc aggaaggact gtgcttccgt  1911
   cacctctaaa tcccttgcag atccttgata aatgcctcat gaagaccaat ctcttgaatc  1971
   ccatatctac ccagaattaa ctccattcca gtctctgcat gtaatcagtt ttatccacag  2031
   aaacattttc attttaggaa atccctggtt taagtatcaa tccttgttca gctggacaat  2091
   atgaatcttt tccactgaag ttagggatga ctgtgatttt cagaacacgt ccagaatttt  2151
   tcatcaagaa ggtagcttga gcctgaaatg caaaacccat ggaggaattc tgaagccatt  2211
   gtctccttga gtaccaacag ggtcagggaa gactgggcct cctgaattta ttattgttct   2271
   ttaagaatta caggttgagg tagttgatgg tggtaaacat tctctcagga gacaataact   2331
   ccagtgatgt ttttcaaaga ttttagcaaa aacagagtaa atagcattct ctatcaatat   2391
   ataaatttaa aaaactatct ttttgcttac agttttaaat tctgaacaat ttctcttata   2451
   tgtgtattgc taatcattaa ggtattattt tttccacata taaagctttg tctttttgtt   2511
   gttgttgttg tttttaagat ggagtttccc tctgttgcca ggctagagtg cagtggcatg   2571
   atctcggctt actgcaacct ttgcctccca ggtttaagcg attcttctgc ctcagcctcc   2631
   cgagtagctg ggaccacagg tgcctaccac catgccaggc taatttttgt atttttagta   2691
   aagacagggt ttcaccatat tggccaggct ggtctcgaac tcctgacctt gtgatctgcc   2751
   cgcctccatt gtgttgttat ttgtgagaaa gatagatatg aggtttagag agggatgaag   2811
   aggtgagagt aagccttgtg ttagtcagaa ctctgtgttg tgaatgtcat tcacaacaga   2871
   aaacccaaaa tattatgcaa actactgtaa gcaagaaaaa taaaggaaaa atggaaacat   2931
   ttattccttt gcataataga aattaccaga gttgttctgt ctttagataa ggtttgaacc   2991
   aaagctcaaa acaatcaaga cccttttctg tatgtccttc tgttctgcct tccgcagtgt   3051
   aggctttacc ctcaggtgct acacagtata gttctagggt ttccctcccg atatcaaaaa   3111
   gactgtggcc tgcccagctc tcgtatcccc aagccacacc atctggctaa atggacatca   3171
   tgttttctgg tgatgcccaa agaggagaga ggaagctctc tttcccagat gccccagcaa   3231
   gtgtaacctt gcatctcatt gctctggctg agttgtgtgc ctgtttctga ccaatcactg   3291
   agtcaggagg atgaaatatt catattgact taattgcagc ttaagttagg ggtatgtaga   3351
   ggtattttcc ctaaagcaaa attgggacac tgttatcaga aataggagag tggatgatag   3411
   atgcaaaata atacctgtcc acaacaaact cttaatgctg tgtttgagct ttcatgagtt   3471
   tcccagagag acatagctgg aaaattccta ttgattttct ctaaaatttc aacaagtagc   3531
   taaagtctgg ctatgctcac agtctcacat ctggtggggg tgggctcctt acagaacacg   3591
   ctttcacagt taccctaaac tctctggggc agggttattc ctttgtggaa ccagaggcac   3651
   agagacagtc aactgaggcc caacagaggc ctgagagaaa ctgaggtcaa gatttcagga   3711
   ttaatggtcc tgtgatgctt tgaagtacaa ttgtggattt gtccaattct ctttagttct   3771
   gtcagctttt gcttcatata ttttagcgct ctattattag atatatacat gtttagtatt   3831
   atgtcttatt ggtgcattta ctctcttatc attatgtaat gtccttcttt atctgtgata   3891
   atttctgtgg ttctgaagtc tactttgtct aaaaataaca tacgcactca acttcctttt   3951
   ctttcttcct tcctttcttt cttccttcct ttctttctct ctctctcttt ccttccttcc   4011
   ttcctccttt tctctctctc tctctctctc tctcttttct tgacagactc tcgttctgtg   4071
   gccctggctg gagttcagtg gtgtgatctt ggctcactgc tacctctacc atgagcaatt   4131
   ctcctgcctc agcctcccaa gtagctggaa ctacaggctc atgccactgc gcccagctaa   4191
   tttttgtatt tttcgtagag acggggtttc accacattcg tcaggttggt ttcaaactcc   4251
   tgactttgtg atccacccgc ctcggcctcc caaagtgctg ggattacagg catgagccat   4311
   cacacctggt caactttctt ttgattagtg tttttgtggt atatcttttt ccatcatgtt   4371
   actttaaata tatctatatt attgtattta aaatgtgttt cttacagact gcatgtagtt   4431
   gggtataatt tttatccagt ctaaaaatat ctgtctttta attggtgttt agacaattta   4491
   tatttaataa aatggtggaa tttaaa                                        4517
<210> 6
   <211> 244
   <212> PRT
   <213> Homo sapiens
<400> 6
   Met Leu Leu Leu Gly Ala Val Leu Leu Leu Leu Ala Leu Pro Gly His
   1               5                   10                  15
   Asp Gln Glu Thr Thr Thr Gln Gly Pro Gly Val Leu Leu Pro Leu Pro
               20                  25                  30
   Lys Gly Ala Cys Thr Gly Trp Met Ala Gly Ile Pro Gly His Pro Gly
           35                  40                  45
   His Asn Gly Ala Pro Gly Arg Asp Gly Arg Asp Gly Thr Pro Gly Glu
       50                  55                  60
   Lys Gly Glu Lys Gly Asp Pro Gly Leu Ile Gly Pro Lys Gly Asp Ile
   65                  70                  75                  80
   Gly Glu Thr Gly Val Pro Gly Ala Glu Gly Pro Arg Gly Phe Pro Gly
                   85                  90                  95
   Ile Gln Gly Arg Lys Gly Glu Pro Gly Glu Gly Ala Tyr Val Tyr Arg
               100                 105                 110
   Ser Ala Phe Ser Val Gly Leu Glu Thr Tyr Val Thr Ile Pro Asn Met
           115                 120                 125
   Pro Ile Arg Phe Thr Lys Ile Phe Tyr Asn Gln Gln Asn His Tyr Asp
       130                 135                 140
   Gly Ser Thr Gly Lys Phe His Cys Asn Ile Pro Gly Leu Tyr Tyr Phe
   145                 150                 155                 160
   Ala Tyr His Ile Thr Val Tyr Met Lys Asp Val Lys Val Ser Leu Phe
                   165                 170                 175
   Lys Lys Asp Lys Ala Met Leu Phe Thr Tyr Asp Gln Tyr Gln Glu Asn
               180                 185                 190
   Asn Val Asp Gln Ala Ser Gly Ser Val Leu Leu His Leu Glu Val Gly
           195                 200                 205
   Asp Gln Val Trp Leu Gln Val Tyr Gly Glu Gly Glu Arg Asn Gly Leu
       210                 215                 220
   Tyr Ala Asp Asn Asp Asn Asp Ser Thr Phe Thr Gly Phe Leu Leu Tyr
   225                 230                 235                 240
   His Asp Thr Asn
<210> 7
   <211> 20966
   <212> DNA
   <213> Homo sapiens
<220>
   <221> misc_feature
   <222> 1..4811
   <223> 5' regulatory region
<220>
   <221> exon
   <222> 4812..4851
   <223> exon 1
<220>
   <221> exon
   <222> 15144..15365
   <223> exon 2
<220>
   <221> exon
   <222> 16277..20559
   <223> exon 3
<220>
   <221> misc_feature
   <222> 20560..20966
   <223> 3' regulatory region
<220>
   <221> allele
   <222> 3787
   <223> 9-27-261 : polymorphic base G or C
<220>
   <221> allele
   <222> 11118
   <223> 99-14387-129 : polymorphic base A or C
<220>
   <221> allele
   <222> 15120
   <223> 9-12-48 : polymorphic base C or T
<220>
   <221> allele
   <222> 15196
   <223> 9-12-124 : polymorphic base G or T
<220>
   <221> allele
   <222> 15427
   <223> 9-12-355 : polymorphic base G or T
<220>
   <221> allele
   <222> 15500
   <223> 9-12-428 : polymorphic base A or G
<220>
   <221> allele
   <222> 15863
   <223> 99-14405-105 : polymorphic base A or G
<220>
   <221> allele
   <222> 17170
   <223> 9-16-189 : polymorphic base deletion of A
<220>
   <221> primer_bind
   <222> 3528..3545
   <223> 9-27.pu
<220>
   <221> primer_bind
   <222> 3928..3946
   <223> 9-27.rp complement
<220>
   <221> primer_bind
   <222> 10990..11008
   <223> 99-14387.pu
<220>
   <221> primer_bind
   <222> 11423..11442
   <223> 99-14387.rp complement
<220>
   <221> primer_bind
   <222> 15073..15092
   <223> 9-12.pu
<220>
   <221> primer_bind
   <222> 15503..15520
   <223> 9-12.rp complement
<220>
   <221> primer_bind
   <222> 15759..15776
   <223> 99-14405.pu
<220>
   <221> primer_bind
   <222> 16191..16211
   <223> 99-14405.rp complement
<220>
   <221> primer_bind
   <222> 16982..17001
   <223> 9-16.pu
<220>
   <221> primer_bind
   <222> 17384..17402
   <223> 9-16.rp complement
<220>
   <221> misc_binding
   <222> 3775..3799
   <223> 9-27-261.probe
<220>
   <221> misc_binding
   <222> 11106..11130
   <223> 99-14387-129.probe
<220>
   <221> misc_binding
   <222> 15108..15132
   <223> 9-12-48.probe
<220>
   <221> misc_binding
   <222> 15184..15208
   <223> 9-12-124.probe
<220>
   <221> misc_binding
   <222> 15415..15439
   <223> 9-12-355.probe
<220>
   <221> misc_binding
   <222> 15488..15512
   <223> 9-12-428.probe
<220>
   <221> misc_binding
   <222> 15851..15875
   <223> 99-14405-105.probe
<220>
   <221> misc_binding
   <222> 17158..17182
   <223> 9-16-189.probe
<220>
   <221> primer_bind
   <222> 3768..3786
   <223> 9-27-261.mis
<220>
   <221> primer_bind
   <222> 3788..3806
   <223> 9-27-261.mis complement
<220>
   <221> primer_bind
   <222> 11099..11117
   <223> 99-14387-129.mis
<220>
   <221> primer_bind
   <222> 11119..11137
   <223> 99-14387-129.mis complement
<220>
   <221> primer_bind
   <222> 15101..15119
   <223> 9-12-48.mis
<220>
   <221> primer_bind
   <222> 15121..15139
   <223> 9-12-48.mis complement
<220>
   <221> primer_bind
   <222> 15177..15195
   <223> 9-12-124.mis
<220>
   <221> primer_bind
   <222> 15197..15215
   <223> 9-12-124.mis complement
<220>
   <221> primer_bind
   <222> 15408..15426
   <223> 9-12-355.mis
<220>
   <221> primer_bind
   <222> 15428..15446
   <223> 9-12-355.mis complement
<220>
   <221> primer_bind
   <222> 15481..15499
   <223> 9-12-428.mis
<220>
   <221> primer_bind
   <222> 15501..15519
   <223> 9-12-428.mis complement
<220>
   <221> primer_bind
   <222> 15844..15862
   <223> 99-14405-105.mis
<220>
   <221> primer_bind
   <222> 15864..15882
   <223> 99-14405-105.mis complement
<220>
   <221> primer_bind
   <222> 17151..17169
   <223> 9-16-189.mis
<220>
   <221> primer_bind
   <222> 17171..17189
   <223> 9-16-189.mis complement
<400> 7